# EUROPEAN PATENT SPECIFICATION

(11) **EP 1 809 336 B1**
(45) Date of publication and mention of the grant of the patent: **12.11.2008**
(21) Application number: 05791035.8
(22) Date of filing: 21.07.2005
(51) Int. Cl.: A61K 48/00

(54) **GROWTH HORMONE RELEASING HORMONE ENHANCES THE IMMUNE RESPONSE INDUCED BY VACCINATION**
WACHSTUMSHORMON-FREISETZENDES HORMON VERSTÄRKT DIE VAKZIN-INDUZIERTE IMMUNANTWORT
HORMONE DE LIBERATION DE L'HORMONE DE CROISSANCE PERMETTANT D'AMELIORER UNE REACTION A LA VACCINATION

(30) Priority: 23.07.2004 US 590739 P
(43) Date of publication of application: 25.07.2007
(73) Proprietor: Advisys, Inc., The Woodlands, TX 77381 (US)
(72) Inventor: DRAGHIA-AKLI, Ruxandra, Houston, TX 77035 (US); BROWN, Patrica, A., Conroe, TX 77302 (US); KHAN, Amir, S., Houston, TX 77030 (US); DAVIS, William, C., Pullman, WA 99163 (US)
(74) Representative: Fisher, Adrian John
(86) International application number: PCT/US2005/025747
(87) International publication number: WO 2006/014737

(56) References cited:
- WO-A-03/049700
- WO-A-03/099341
- KHORRAM O ET AL: "Effects of (norleucine27)growth hormone-releasing hormone (GHRH) (1-29)-NH2 administration on the immune system of aging men and women" JOURNAL OF CLINICAL ENDOCRINOLOGY AND METABOLISM, vol. 82, no. 11, November 1997 (1997-11), pages 3590-3596, XP002366966 ISSN: 0021-972X
- KHORRAM O ET AL: "The influence of aging and sex hormones on expression of growth hormone-releasing hormone in the human immune system" JOURNAL OF CLINICAL ENDOCRINOLOGY AND METABOLISM, vol. 86, no. 7, July 2001 (2001-07), pages 3157-3161, XP002366967 ISSN: 0021-972X

### SEQUENCE LISTING

<110> Advisys, Inc.
<120> Growth Hormone Releasing Hormone Enhances Vaccination Response
<130> P045944EP
<140> EP 05791035.8
   <141> 2005-07-21
<150> US 60/590739
   <151> 2004-07-23
<160> 41
<170> PatentIn version 3.1
<210> 1
   <211> 40
   <212> PRT
   <213> artificial sequence
<220>
   <223> This is a HV-growth hormone releasing hormone ("GHRH") analog.
<400> 1
   His Val Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 2
   <211> 40
   <212> PRT
   <213> Artificial sequence
<220>
   <223> This is an amino acid sequence for Pig-GHRH
<400> 2
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Gln Glu Gln Gly Ala
           35                  40
<210> 3
   <211> 40
   <212> PRT
   <213> artificial sequence
<220>
   <223> This is an amino acid sequence for Bovine-GHRH
<400> 3
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Asn Arg Gln Gln Gly
               20                  25                  30
   Glu Arg Asn Gln Glu Gln Gly Ala
           35                  40
<210> 4
   <211> 40
   <212> PRT
   <213> artificial sequence
<220>
   <223> This is an amino acid sequence for Dog-GHRH
<400> 4
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Arg Glu Gln Gly Ala
           35                  40
<210> 5
   <211> 40
   <212> PRT
   <213> Artificial Sequence
<220>
   <223> This is an amino acid sequence for Cat-GHRH
<400> 5
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Gln Glu Gln Gly Ala
           35                  40
<210> 6
   <211> 40
   <212> PRT
   <213> artificial sequence
<220>
   <223> This is a TI- growth hormone releasing hormone ("GHRH") analog.
<400> 6
   Tyr Ile Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 7
   <211> 40
   <212> PRT
   <213> artificial sequence
<220>
   <223> This is an amino acid sequence for Ovine-GHRH
<400> 7
   Tyr Ala Asp Ala Ile Phe Thr Asn Ser Tyr Arg Lys Ile Leu Gly Gln
   1               5                   10                  15
   Leu Ser Ala Arg Lys Leu Leu Gln Asp Ile Met Asn Arg Gln Gln Gly
               20                  25                  30
   Glu Arg Asn Gln Glu Gln Gly Ala
           35                  40
<210> 8
   <211> 43
   <212> PRT
   <213> artificial sequence
<220>
   <223> This is an amino acid sequence for Chicken-GHRH
<400> 8
   His Ala Asp Gly Ile Phe Ser Lys Ala Tyr Arg Lys Leu Leu Gly Gln
   1               5                   10                  15
   Leu Ser Ala Arg Asn Tyr Leu His Ser Leu Met Ala Lys Arg Val Gly
               20                  25                  30
   Ser Gly Leu Gly Asp Glu Ala Glu Pro Leu Ser
           35                  40
<210> 9
   <211> 40
   <212> PRT
   <213> artificial sequence
<220>
   <223> This is an amino acid sequence for Horse GHRH.
<220>
   <221> misc_feature
<222> (1)..(1)
   <223> "X" can be any AA sequence
<220>
   <221> misc_feature
   <222> (30)..(40)
   <223> "X" can be any AA sequence
<400> 9
   Xaa Ala Asp Ala Ile Phe Thr Asn Asn Tyr Arg Lys Val Leu Gly Gln
   1               5                   10                  15
   Leu Ser Ala Arg Lys Ile Leu Gln Asp Ile Met Ser Arg Xaa Xaa Xaa
               20                  25                  30
   Xaa Xaa Xaa Xaa Xaa Xaa Xaa Xaa
           35                  40
<210> 10
<211> 40
   <212> PRT
   <213> artificial sequence
<220>
   <223> This is an amino acid sequence for human (1-40)-GHRH
   <400> 10
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Ser Asn Gln Glu Arg Gly Ala
           35                  40
<210> 11
   <211> 40
   <212> PRT
   <213> artificial sequence
<220>
   <223> This is a TV-growth hormone releasing hormone ("GHRH") analog.
<400> 11
   Tyr Val Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 12
   <211> 40
   <212> PRT
   <213> artificial sequence
<220>
<223> This is a TA-growth hormone releasing hormone ("GHRH") analog.
<400> 12
   Tyr Ala Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 13
<211> 44
   <212> PRT
   <213> artificial sequence
<220>
   <223> This is a human (1-44) growth hormone releasing hormone ("GHRH").
<400> 13
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Ser Asn Gln Glu Arg Gly Ala Arg Ala Arg Leu
           35                  40
<210> 14
   <211> 40
   <212> PRT
   <213> artificial sequence
<220>
   <223> This is the artificial sequence for GHRH (1-40)OH.
<220>
<221> MISC_FEATURE
   <222> (1)..(1)
   <223> Xaa at position 1 may be tyrosine, or histidine
<220>
<221> MISC_FEATURE
   <222> (2)..(2)
   <223> Xaa at position 2 may be alanine, valine, or isoleucine.
<220>
<221> MISC_FEATURE
   <222> (15)..(15)
   <223> Xaa at position 15 may be alanine, valine, or isoleucine.
<220>
<221> MISC_FEATURE
   <222> (27)..(27)
   <223> Xaa at position 27 may be methionine, or leucine.
<220>
<221> MISC_FEATURE
   <222> (28)..(28)
   <223> Xaa at position 28 may be serine or asparagine.
<220>
<221> MISC_FEATURE
   <222> (34)..(34)
   <223> Xaa at position 34 may be arginine or serine.
<220>
<221> MISC_FEATURE
   <222> (36)..(36)
   <223> Xaa at position 36 may be arginine or glutamine.
<220>
<221> MISC_FEATURE
   <222> (38)..(38)
   <223> Xaa at position 38 may be arginine or glutamine
<400> 14
   Xaa Xaa Asp Ala Ile Phe Thr Asn Ser Tyr Arg Lys Val Leu Xaa Gln
   1               5                   10                  15
   Leu Ser Ala Arg Lys Leu Leu Gln Asp Ile Xaa Xaa Arg Gln Gln Gly
               20                  25                  30
   Glu Xaa Asn Xaa Glu Xaa Gly Ala
           35                  40
<210> 15
   <211> 323
   <212> DNA
   <213> artificial sequence
<220>
   <223> This is a nucleic acid sequence of a eukaryotic promoter c5-12.
<400> 15
   cggccgtccg ccctcggcac catcctcacg acacccaaat atggcgacgg gtgaggaatg 60
   gtggggagtt atttttagag cggtgaggaa ggtgggcagg cagcaggtgt tggcgctcta 120
   aaaataactc ccgggagtta tttttagagc ggaggaatgg tggacaccca aatatggcga 180
   cggttcctca cccgtcgcca tatttgggtg tccgccctcg gccggggccg cattcctggg 240
   ggccgggcgg tgctcccgcc cgcctcgata aaaggctccg gggccggcgg cggcccacga 300
   gctacccgga ggagcgggag gcg 323
<210> 16
   <211> 190
   <212> DNA
   <213> artificial sequence
<220>
<223> Nucleic acid sequence of a human growth hormone poly A tail.
<400> 16
   gggtggcatc cctgtgaccc ctccccagtg cctctcctgg ccctggaagt tgccactcca 60
   gtgcccacca gccttgtcct aataaaatta agttgcatca ttttgtctga ctaggtgtcc 120
   ttctataata ttatggggtg gaggggggtg gtatggagca aggggcaagt tgggaagaca 180
   acctgtaggg 190
<210> 17
   <211> 795
   <212> DNA
   <213> artificial sequence
<220>
   <223> Nucleic acid sequence for antibiotic resistance gene kanamycin.
<400> 17
   atgattgaac aagatggatt gcacgcaggt tctccggccg cttgggtgga gaggctattc 60
   ggctatgact gggcacaaca gacaatcggc tgctctgatg ccgccgtgtt ccggctgtca 120
   gcgcaggggc gcccggttct ttttgtcaag accgacctgt ccggtgccct gaatgaactg 180
   caggacgagg cagcgcggct atcgtggctg gccacgacgg gcgttccttg cgcagctgtg 240
   ctcgacgttg tcactgaagc gggaagggac tggctgctat tgggcgaagt gccggggcag 300
   gatctcctgt catctcacct tgctcctgcc gagaaagtat ccatcatggc tgatgcaatg 360
   cggcggctgc atacgcttga tccggctacc tgcccattcg accaccaagc gaaacatcgc 420
   atcgagcgag cacgtactcg gatggaagcc ggtcttgtcg atcaggatga tctggacgaa 480
   gagcatcagg ggctcgcgcc agccgaactg ttcgccaggc tcaaggcgcg catgcccgac 540
   ggcgaggatc tcgtcgtgac tcatggcgat gcctgcttgc cgaatatcat ggtggaaaat 600
   ggccgctttt ctggattcat cgactgtggc cggctgggtg tggcggaccg ctatcaggac 660
   atagcgttgg ctacccgtga tattgctgaa gagcttggcg gcgaatgggc tgaccgcttc 720
   ctcgtgcttt acggtatcgc cgctcccgat tcgcagcgca tcgccttcta tcgccttctt 780
   gacgagttct tctga 795
<210> 18
   <211> 219
   <212> DNA
   <213> artificial sequence
<220>
   <223> Sequence for an analog porcine GHRH sequence.
<400> 18
   atggtgctct gggtgttctt ctttgtgatc ctcaccctca gcaacagctc ccactgctcc 60
   ccacctcccc ctttgaccct caggatgcgg cggcacgtag atgccatctt caccaacagc 120
   taccggaagg tgctggccca gctgtccgcc cgcaagctgc tccaggacat cctgaacagg 180
   cagcagggag agaggaacca agagcaagga gcataatga 219
<210> 19
   <211> 246
   <212> DNA
   <213> artificial sequence
<220>
   <223> Sequence for an analog mouse GHRH sequence.
<400> 19
   gccatggtgc tctgggtgct ctttgtgatc ctcatcctca ccagcggcag ccactgcagc 60
   ctgcctccca gccctcccct caggatgcag aggcacgtgg acgccatctt caccaccaac 120
   tacaggaagc tgctgagcca gctgtacgcc aggaaggtga tccaggacat catgaacaag 180
   cagggcgaga ggatccagga gcagagggcc aggctgagct gataagcttg cgatgagttc 240
   ttctaa 246
<210> 20
   <211> 234
   <212> DNA
   <213> artificial sequence
<220>
   <223> Sequence for an analog rat GHRH sequence.
<400> 20
   gccatggccc tgtgggtgtt cttcgtgctg ctgaccctga ccagcggaag ccactgcagc 60
   ctgcctccca gccctccctt cagggtgcgc cggcacgccg acgccatctt caccagcagc 120
   tacaggagga tcctgggcca gctgtacgct aggaagctcc tgcacgagat catgaacagg 180
   cagcagggcg agaggaacca ggagcagagg agcaggttca actgataagc ttgc 234
<210> 21
   <211> 29
   <212> DNA
   <213> artificial sequence
<220>
   <223> Nucleic acid sequence of a prokaryotic PNEO promoter.
<400> 21
   accttaccag agggcgcccc agctggcaa 29
<210> 22
   <211> 3534
   <212> DNA
   <213> artificial sequence
<220>
   <223> Plasmid vector having an analog GHRH sequence.
<400> 22
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggcacgt agatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga ggggggggcc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 23
   <211> 2722
   <212> DNA
   <213> artificial sequence
<220>
   <223> Plasmid vector having a codon optimized mouse GHRH sequence
<400> 23
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgctc 420
   tttgtgatcc tcatcctcac cagcggcagc cactgcagcc tgcctcccag ccctcccttc 480
   aggatgcaga ggcacgtgga cgccatcttc accaccaact acaggaagct gctgagccag 540
   ctgtacgcca ggaaggtgat ccaggacatc atgaacaagc agggcgagag gatccaggag 600
   cagagggcca ggctgagctg ataagcttat cggggtggca tccctgtgac ccctccccag 660
   tgcctctcct ggccctggaa gttgccactc cagtgcccac cagccttgtc ctaataaaat 720
   taagttgcat cattttgtct gactaggtgt ccttctataa tattatgggg tggagggggg 780
   tggtatggag caaggggcaa gttgggaaga caacctgtag ggctcgaggg ggggcccggt 840
   accagctttt gttcccttta gtgagggtta atttcgagct tggtcttccg cttcctcgct 900
   cactgactcg ctgcgctcgg tcgttcggct gcggcgagcg gtatcagctc actcaaaggc 960
   ggtaatacgg ttatccacag aatcagggga taacgcagga aagaacatgt gagcaaaagg 1020
   ccagcaaaag gccaggaacc gtaaaaaggc cgcgttgctg gcgtttttcc ataggctccg 1080
   cccccctgac gagcatcaca aaaatcgacg ctcaagtcag aggtggcgaa acccgacagg 1140
   actataaaga taccaggcgt ttccccctgg aagctccctc gtgcgctctc ctgttccgac 1200
   cctgccgctt accggatacc tgtccgcctt tctcccttcg ggaagcgtgg cgctttctca 1260
   tagctcacgc tgtaggtatc tcagttcggt gtaggtcgtt cgctccaagc tgggctgtgt 1320
   gcacgaaccc cccgttcagc ccgaccgctg cgccttatcc ggtaactatc gtcttgagtc 1380
   caacccggta agacacgact tatcgccact ggcagcagcc actggtaaca ggattagcag 1440
   agcgaggtat gtaggcggtg ctacagagtt cttgaagtgg tggcctaact acggctacac 1500
   tagaagaaca gtatttggta tctgcgctct gctgaagcca gttaccttcg gaaaaagagt 1560
   tggtagctct tgatccggca aacaaaccac cgctggtagc ggtggttttt ttgtttgcaa 1620
   gcagcagatt acgcgcagaa aaaaaggatc tcaagaagat cctttgatct tttctacggg 1680
   gtctgacgct cagctagcgc tcagaagaac tcgtcaagaa ggcgatagaa ggcgatgcgc 1740
   tgcgaatcgg gagcggcgat accgtaaagc acgaggaagc ggtcagccca ttcgccgcca 1800
   agctcttcag caatatcacg ggtagccaac gctatgtcct gatagcggtc cgccacaccc 1860
   agccggccac agtcgatgaa tccagaaaag cggccatttt ccaccatgat attcggcaag 1920
   caggcatcgc catgagtcac gacgagatcc tcgccgtcgg gcatgcgcgc cttgagcctg 1980
   gcgaacagtt cggctggcgc gagcccctga tgctcttcgt ccagatcatc ctgatcgaca 2040
   agaccggctt ccatccgagt acgtgctcgc tcgatgcgat gtttcgcttg gtggtcgaat 2100
   gggcaggtag ccggatcaag cgtatgcagc cgccgcattg catcagccat gatggatact 2160
   ttctcggcag gagcaaggtg agatgacagg agatcctgcc ccggcacttc gcccaatagc 2220
   agccagtccc ttcccgcttc agtgacaacg tcgagcacag ctgcgcaagg aacgcccgtc 2280
   gtggccagcc acgatagccg cgctgcctcg tcctgcagtt cattcagggc accggacagg 2340
   tcggtcttga caaaaagaac cgggcgcccc tgcgctgaca gccggaacac ggcggcatca 2400
   gagcagccga ttgtctgttg tgcccagtca tagccgaata gcctctccac ccaagcggcc 2460
   ggagaacctg cgtgcaatcc atcttgttca atcatgcgaa acgatcctca tcctgtctct 2520
   tgatcagatc ttgatcccct gcgccatcag atccttggcg gcaagaaagc catccagttt 2580
   actttgcagg gcttcccaac cttaccagag ggcgccccag ctggcaattc cggttcgctt 2640
   gctgtccata aaaccgccca gtctagcaac tgttgggaag ggcgatcgtg taatacgact 2700
   cactataggg cgaattggag ct 2722
<210> 24
   <211> 2725
   <212> DNA
   <213> artificial sequence
<220>
   <223> Plasmid vector having a codon optimized rat GHRH sequence
<400> 24
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg gggggccggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggccct gtgggtgttc 420
   ttcgtgctgc tgaccctgac cagcggaagc cactgcagcc tgcctcccag ccctcccttc 480
   agggtgcgcc ggcacgccga cgccatcttc accagcagct acaggaggat cctgggccag 540
   ctgtacgcta ggaagctcct gcacgagatc atgaacaggc agcagggcga gaggaaccag 600
   gagcagagga gcaggttcaa ctgataagct tatcggggtg gcatccctgt gacccctccc 660
   cagtgcctct cctggccctg gaagttgcca ctccagtgcc caccagcctt gtcctaataa 720
   aattaagttg catcattttg tctgactagg tgtccttcta taatattatg gggtggaggg 780
   gggtggtatg gagcaagggg caagttggga agacaacctg tagggctcga ggggggggcc 840
   ggtaccagct tttgttccct ttagtgaggg ttaatttcga gcttggtctt ccgcttcctc 900
   gctcactgac tcgctgcgct cggtcgttcg gctgcggcga gcggtatcag ctcactcaaa 960
   ggcggtaata cggttatcca cagaatcagg ggataacgca ggaaagaaca tgtgagcaaa 1020
   aggccagcaa aaggccagga accgtaaaaa ggccgcgttg ctggcgtttt tccataggct 1080
   ccgcccccct gacgagcatc acaaaaatcg acgctcaagt cagaggtggc gaaacccgac 1140
   aggactataa agataccagg cgtttccccc tggaagctcc ctcgtgcgct ctcctgttcc 1200
   gaccctgccg cttaccggat acctgtccgc ctttctccct tcgggaagcg tggcgctttc 1260
   tcatagctca cgctgtaggt atctcagttc ggtgtaggtc gttcgctcca agctgggctg 1320
   tgtgcacgaa ccccccgttc agcccgaccg ctgcgcctta tccggtaact atcgtcttga 1380
   gtccaacccg gtaagacacg acttatcgcc actggcagca gccactggta acaggattag 1440
   cagagcgagg tatgtaggcg gtgctacaga gttcttgaag tggtggccta actacggcta 1500
   cactagaaga acagtatttg gtatctgcgc tctgctgaag ccagttacct tcggaaaaag 1560
   agttggtagc tcttgatccg gcaaacaaac caccgctggt agcggtggtt tttttgtttg 1620
   caagcagcag attacgcgca gaaaaaaagg atctcaagaa gatcctttga tcttttctac 1680
   ggggtctgac gctcagctag cgctcagaag aactcgtcaa gaaggcgata gaaggcgatg 1740
   cgctgcgaat cgggagcggc gataccgtaa agcacgagga agcggtcagc ccattcgccg 1800
   ccaagctctt cagcaatatc acgggtagcc aacgctatgt cctgatagcg gtccgccaca 1860
   cccagccggc cacagtcgat gaatccagaa aagcggccat tttccaccat gatattcggc 1920
   aagcaggcat cgccatgagt cacgacgaga tcctcgccgt cgggcatgcg cgccttgagc 1980
   ctggcgaaca gttcggctgg cgcgagcccc tgatgctctt cgtccagatc atcctgatcg 2040
   acaagaccgg cttccatccg agtacgtgct cgctcgatgc gatgtttcgc ttggtggtcg 2100
   aatgggcagg tagccggatc aagcgtatgc agccgccgca ttgcatcagc catgatggat 2160
   actttctcgg caggagcaag gtgagatgac aggagatcct gccccggcac ttcgcccaat 2220
   agcagccagt cccttcccgc ttcagtgaca acgtcgagca cagctgcgca aggaacgccc 2280
   gtcgtggcca gccacgatag ccgcgctgcc tcgtcctgca gttcattcag ggcaccggac 2340
   aggtcggtct tgacaaaaag aaccgggcgc ccctgcgctg acagccggaa cacggcggca 2400
   tcagagcagc cgattgtctg ttgtgcccag tcatagccga atagcctctc cacccaagcg 2460
   gccggagaac ctgcgtgcaa tccatcttgt tcaatcatgc gaaacgatcc tcatcctgtc 2520
   tcttgatcag atcttgatcc cctgcgccat cagatccttg gcggcaagaa agccatccag 2580
   tttactttgc agggcttccc aaccttacca gagggcgccc cagctggcaa ttccggttcg 2640
   cttgctgtcc ataaaaccgc ccagtctagc aactgttggg aagggcgatc gtgtaatacg 2700
   actcactata gggcgaattg gagct 2725
<210> 25
   <211> 2725
   <212> DNA
   <213> artificial sequence
<220>
   <223> This is the codon optimized HV-GHRH expression plasmid.
<400> 25
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttctttgtga tcctcaccct cagcaacagc tcccactgct ccccacctcc ccctttgacc 480
   ctcaggatgc ggcggcacgt agatgccatc ttcaccaaca gctaccggaa ggtgctggcc 540
   cagctgtccg cccgcaagct gctccaggac atcctgaaca ggcagcaggg agagaggaac 600
   caagagcaag gagcataatg acatcaagct tatcggggtg gcatccctgt gacccctccc 660
   cagtgcctct cctggccctg gaagttgcca ctccagtgcc caccagcctt gtcctaataa 720
   aattaagttg catcattttg tctgactagg tgtccttcta taatattatg gggtggaggg 780
   gggtggtatg gagcaagggg caagttggga agacaacctg tagggctcga gggggggccc 840
   ggtaccagct tttgttccct ttagtgaggg ttaatttcga gcttggtctt ccgcttcctc 900
   gctcactgac tcgctgcgct cggtcgttcg gctgcggcga gcggtatcag ctcactcaaa 960
   ggcggtaata cggttatcca cagaatcagg ggataacgca ggaaagaaca tgtgagcaaa 1020
   aggccagcaa aaggccagga accgtaaaaa ggccgcgttg ctggcgtttt tccataggct 1080
   ccgcccccct gacgagcatc acaaaaatcg acgctcaagt cagaggtggc gaaacccgac 1140
   aggactataa agataccagg cgtttccccc tggaagctcc ctcgtgcgct ctcctgttcc 1200
   gaccctgccg cttaccggat acctgtccgc ctttctccct tcgggaagcg tggcgctttc 1260
   tcatagctca cgctgtaggt atctcagttc ggtgtaggtc gttcgctcca agctgggctg 1320
   tgtgcacgaa ccccccgttc agcccgaccg ctgcgcctta tccggtaact atcgtcttga 1380
   gtccaacccg gtaagacacg acttatcgcc actggcagca gccactggta acaggattag 1440
   cagagcgagg tatgtaggcg gtgctacaga gttcttgaag tggtggccta actacggcta 1500
   cactagaaga acagtatttg gtatctgcgc tctgctgaag ccagttacct tcggaaaaag 1560
   agttggtagc tcttgatccg gcaaacaaac caccgctggt agcggtggtt tttttgttta 1620
   caagcagcag attacgcgca gaaaaaaagg atctcaagaa gatcctttga tcttttctac 1680
   ggggtctgac gctcagctag cgctcagaag aactcgtcaa gaaggcgata gaaggcgatg 1740
   cgctgcgaat cgggagcggc gataccgtaa agcacgagga agcggtcagc ccattcgccg 1800
   ccaagctctt cagcaatatc acgggtagcc aacgctatgt cctgatagcg gtccgccaca 1860
   cccagccggc cacagtcgat gaatccagaa aagcggccat tttccaccat gatattcggc 1920
   aagcaggcat cgccatgagt cacgacgaga tcctcgccgt cgggcatgcg cgccttgagc 1980
   ctggcgaaca gttcggctgg cgcgagcccc tgatgctctt cgtccagatc atcctgatcg 2040
   acaagaccgg cttccatccg agtacgtgct cgctcgatgc gatgtttcgc ttggtggtcg 2100
   aatgggcagg tagccggatc aagcgtatgc agccgccgca ttgcatcagc catgatggat 2160
   actttctcgg caggagcaag gtgagatgac aggagatcct gccccggcac ttcgcccaat 2220
   agcagccagt cccttcccgc ttcagtgaca acgtcgagca cagctgcgca aggaacgccc 2280
   gtcgtggcca gccacgatag ccgcgctgcc tcgtcctgca gttcattcag ggcaccggac 2340
   aggtcggtct tgacaaaaag aaccgggcgc ccctgcgctg acagccggaa cacggcggca 2400
   tcagagcagc cgattgtctg ttgtgcccag tcatagccga atagcctctc cacccaagcg 2460
   gccggagaac ctgcgtgcaa tccatcttgt tcaatcatgc gaaacgatcc tcatcctgtc 2520
   tcttgatcag atcttgatcc cctgcgccat cagatccttg gcggcaagaa agccatccag 2580
   tttactttgc agggcttccc aaccttacca gagggcgccc cagctggcaa ttccggttcg 2640
   cttgctgtcc ataaaaccgc ccagtctagc aactgttggg aagggcgatc gtgtaatacg 2700
   actcactata gggcgaattg gagct 2725
<210> 26
   <211> 2721
   <212> DNA
   <213> artificial sequence
<220>
   <223> This is the codon optimized pig-GHRH expression plasmid.
<400> 26
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttctttgtga tcctcaccct cagcaacagc tcccactgct ccccacctcc ccctttgacc 480
   ctcaggatgc ggcggtatgc agatgccatc ttcaccaaca gctaccggaa ggtgctgggc 540
   cagctgtccg cccgcaagct gctccaggac atcatgagca ggcagcaggg agagaggaac 600
   caagagcaag gagcataatg aaagcttatc ggggtggcat ccctgtgacc cctccccagt 660
   gcctctcctg gccctggaag ttgccactcc agtgcccacc agccttgtcc taataaaatt 720
   aagttgcatc attttgtctg actaggtgtc cttctataat attatggggt ggaggggggt 780
   ggtatggagc aaggggcaag ttgggaagac aacctgtagg gctcgagggg gggcccggta 840
   ccagcttttg ttccctttag tgagggttaa tttcgagctt ggtcttccgc ttcctcgctc 900
   actgactcgc tgcgctcggt cgttcggctg cggcgagcgg tatcagctca ctcaaaggcg 960
   gtaatacggt tatccacaga atcaggggat aacgcaggaa agaacatgtg agcaaaaggc 1020
   cagcaaaagg ccaggaaccg taaaaaggcc gcgttgctgg cgtttttcca taggctccgc 1080
   ccccctgacg agcatcacaa aaatcgacgc tcaagtcaga ggtggcgaaa cccgacagga 1140
   ctataaagat accaggcgtt tccccctgga agctccctcg tgcgctctcc tgttccgacc 1200
   ctgccgctta ccggatacct gtccgccttt ctcccttcgg gaagcgtggc gctttctcat 1260
   agctcacgct gtaggtatct cagttcggtg taggtcgttc gctccaagct gggctgtgtg 1320
   cacgaacccc ccgttcagcc cgaccgctgc gccttatccg gtaactatcg tcttgagtcc 1380
   aacccggtaa gacacgactt atcgccactg gcagcagcca ctggtaacag gattagcaga 1440
   gcgaggtatg taggcggtgc tacagagttc ttgaagtggt ggcctaacta cggctacact 1500
   agaagaacag tatttggtat ctgcgctctg ctgaagccag ttaccttcgg aaaaagagtt 1560
   ggtagctctt gatccggcaa acaaaccacc gctggtagcg gtggtttttt tgtttacaag 1620
   cagcagatta cgcgcagaaa aaaaggatct caagaagatc ctttgatctt ttctacgggg 1680
   tctgacgctc agctagcgct cagaagaact cgtcaagaag gcgatagaag gcgatgcgct 1740
   gcgaatcggg agcggcgata ccgtaaagca cgaggaagcg gtcagcccat tcgccgccaa 1800
   gctcttcagc aatatcacgg gtagccaacg ctatgtcctg atagcggtcc gccacaccca 1860
   gccggccaca gtcgatgaat ccagaaaagc ggccattttc caccatgata ttcggcaagc 1920
   aggcatcgcc atgagtcacg acgagatcct cgccgtcggg catgcgcgcc ttgagcctgg 1980
   cgaacagttc ggctggcgcg agcccctgat gctcttcgtc cagatcatcc tgatcgacaa 2040
   gaccggcttc catccgagta cgtgctcgct cgatgcgatg tttcgcttgg tggtcgaatg 2100
   ggcaggtagc cggatcaagc gtatgcagcc gccgcattgc atcagccatg atggatactt 2160
   tctcggcagg agcaaggtga gatgacagga gatcctgccc cggcacttcg cccaatagca 2220
   gccagtccct tcccgcttca gtgacaacgt cgagcacagc tgcgcaagga acgcccgtcg 2280
   tggccagcca cgatagccgc gctgcctcgt cctgcagttc attcagggca ccggacaggt 2340
   cggtcttgac aaaaagaacc gggcgcccct gcgctgacag ccggaacacg gcggcatcag 2400
   agcagccgat tgtctgttgt gcccagtcat agccgaatag cctctccacc caagcggccg 2460
   gagaacctgc gtgcaatcca tcttgttcaa tcatgcgaaa cgatcctcat cctgtctctt 2520
   gatcagatct tgatcccctg cgccatcaga tccttggcgg caagaaagcc atccagttta 2580
   ctttgcaggg cttcccaacc ttaccagagg gcgccccagc tggcaattcc ggttcgcttg 2640
   ctgtccataa aaccgcccag tctagcaact gttgggaagg gcgatcgtgt aatacgactc 2700
   actatagggc gaattggagc t 2721
<210> 27
   <211> 2716
   <212> DNA
   <213> artificial sequence
<220>
   <223> This is the codon optimized dog-GHRH expression plasmid.
<400> 27
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttcctggtga tcctcaccct cagcagtggt tcccactctt ccccgccatc cctgcccatc 480
   agaatccctc ggtatgcaga cgccatcttc accaacagct accggaaggt gctgggccag 540
   ctgtccgccc gcaagctcct scaggacatc atgagccggc agcagggaga gagaaaccgg 600
   gagcaaggag catagtaagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc ggcaaacaaa ccaccgctgg tagcggtggt ttttttgttt acaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 28
   <211> 2716
   <212> DNA
   <213> artificial sequence
<220>
   <223> This is the codon optimized bovine-GHRH expression plasmid.
<400> 28
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct gtgggtgttc 420
   ttcctggtga ccctgaccct gagcagcggc tcccacggct ccctgccctc ccagcctctg 480
   cgcatccctc gctacgccga cgccatcttc accaacagct accgcaaggt gctcggccag 540
   ctcagcgccc gcaagctcct gcaggacatc atgaaccggc agcagggcga gcgcaaccag 600
   gagcagggag cctgataagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc ggcaaacaaa ccaccgctgg tagcggtggt ttttttgttt acaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 29
   <211> 2716
   <212> DNA
   <213> artificial sequence
<220>
   <223> This is the codon optimized cat-GHRH expression plasmid.
<400> 29
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttcctggtga tcctcacccs ssacagtggc tcccactgct ccccgccatc cctgcccctc 480
   agaatgcctc ggtatgcaga tgccatcttc accaacagct accggaaggt gctgggtcag 540
   ctgtctgccc gcaagctact gcaggacatc atgagcagac agcagggaga gagaaaccag 600
   gagcaaggag cataataagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc ggcaaacaaa ccaccgctgg tagcggtggt ttttttgttt acaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 30
   <211> 2739
   <212> DNA
   <213> artificial sequence
<220>
   <223> This is the codon optimized TI-GHRH expression plasmid.
<400> 30
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttctttgtga tcctcaccct cagcaacagc tcccactgct ccccacctcc ccctttgacc 480
   ctcaggatgc ggcggtatat cgatgccatc ttcaccaaca gctaccggaa ggtgctggcc 540
   cagctgtccg cccgcaagct gctccaggac atcctgaaca ggcagcaggg agagaggaac 600
   caagagcaag gagcataatg actgcaggaa ttcgatatca agcttatcgg ggtggcatcc 660
   ctgtgacccc tccccagtgc ctctcctggc cctggaagtt gccactccag tgcccaccag 720
   ccttgtccta ataaaattaa gttgcatcat tttgtctgac taggtgtcct tctataatat 780
   tatggggtgg aggggggtgg tatggagcaa ggggcaagtt gggaagacaa cctgtagggc 840
   tcgagggggg gcccggtacc agcttttgtt ccctttagtg agggttaatt tcgagcttgg 900
   tcttccgctt cctcgctcac tgactcgctg cgctcggtcg ttcggctgcg gcgagcggta 960
   tcagctcact caaaggcggt aatacggtta tccacagaat caggggataa cgcaggaaag 1020
   aacatgtgag caaaaggcca gcaaaaggcc aggaaccgta aaaaggccgc gttgctggcg 1080
   tttttccata ggctccgccc ccctgacgag catcacaaaa atcgacgctc aagtcagagg 1140
   tggcgaaacc cgacaggact ataaagatac caggcgtttc cccctggaag ctccctcgtg 1200
   cgctctcctg ttccgaccct gccgcttacc ggatacctgt ccgcctttct cccttcggga 1260
   agcgtggcgc tttctcatag ctcacgctgt aggtatctca gttcggtgta ggtcgttcgc 1320
   tccaagctgg gctgtgtgca cgaacccccc gttcagcccg accgctgcgc cttatccggt 1380
   aactatcgtc ttgagtccaa cccggtaaga cacgacttat cgccactggc agcagccact 1440
   ggtaacagga ttagcagagc gaggtatgta ggcggtgcta cagagttctt gaagtggtgg 1500
   cctaactacg gctacactag aagaacagta tttggtatct gcgctctgct gaagccagtt 1560
   accttcggaa aaagagttgg tagctcttga tccggcaaac aaaccaccgc tggtagcggt 1620
   ggtttttttg tttacaagca gcagattacg cgcagaaaaa aaggatctca agaagatcct 1680
   ttgatctttt ctacggggtc tgacgctcag ctagcgctca gaagaactcg tcaagaaggc 1740
   gatagaaggc gatgcgctgc gaatcgggag cggcgatacc gtaaagcacg aggaagcggt 1800
   cagcccattc gccgccaagc tcttcagcaa tatcacgggt agccaacgct atgtcctgat 1860
   agcggtccgc cacacccagc cggccacagt cgatgaatcc agaaaagcgg ccattttcca 1920
   ccatgatatt cggcaagcag gcatcgccat gagtcacgac gagatcctcg ccgtcgggca 1980
   tgcgcgcctt gagcctggcg aacagttcgg ctggcgcgag cccctgatgc tcttcgtcca 2040
   gatcatcctg atcgacaaga ccggcttcca tccgagtacg tgctcgctcg atgcgatgtt 2100
   tcgcttggtg gtcgaatggg caggtagccg gatcaagcgt atgcagccgc cgcattgcat 2160
   cagccatgat ggatactttc tcggcaggag caaggtgaga tgacaggaga tcctgccccg 2220
   gcacttcgcc caatagcagc cagtcccttc ccgcttcagt gacaacgtcg agcacagctg 2280
   cgcaaggaac gcccgtcgtg gccagccacg atagccgcgc tgcctcgtcc tgcagttcat 2340
   tcagggcacc ggacaggtcg gtcttgacaa aaagaaccgg gcgcccctgc gctgacagcc 2400
   ggaacacggc ggcatcagag cagccgattg tctgttgtgc ccagtcatag ccgaatagcc 2460
   tctccaccca agcggccgga gaacctgcgt gcaatccatc ttgttcaatc atgcgaaacg 2520
   atcctcatcc tgtctcttga tcagatcttg atcccctgcg ccatcagatc cttggcggca 2580
   agaaagccat ccagtttact ttgcagggct tcccaacctt accagagggc gccccagctg 2640
   gcaattccgg ttcgcttgct gtccataaaa ccgcccagtc tagcaactgt tgggaagggc 2700
   gatcgtgtaa tacgactcac tatagggcga attggagct 2739
<210> 31
   <211> 2716
   <212> DNA
   <213> artificial sequence
<220>
   <223> This is the codon optimized ovine-GHRH expression plasmid.
<400> 31
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct gtgggtgttc 420
   ttcctggtga ccctgaccct gagcagcgga agccacggca gcctgcccag ccagcccctg 480
   aggatcccta ggtacgccga cgccatcttc accaacagct acaggaagat cctgggccag 540
   ctgagcgcta ggaagctcct gcaggacatc atgaacaggc agcagggcga gaggaaccag 600
   gagcagggcg cctgataagc ttatcggggt ggcatccctg tgacccctcc ccagtgcctc 660
   tcctggccct ggaagttgcc actccagtgc ccaccagcct tgtcctaata aaattaagtt 720
   gcatcatttt gtctgactag gtgtccttct ataatattat ggggtggagg ggggtggtat 780
   ggagcaaggg gcaagttggg aagacaacct gtagggctcg agggggggcc cggtaccagc 840
   ttttgttccc tttagtgagg gttaatttcg agcttggtct tccgcttcct cgctcactga 900
   ctcgctgcgc tcggtcgttc ggctgcggcg agcggtatca gctcactcaa aggcggtaat 960
   acggttatcc acagaatcag gggataacgc aggaaagaac atgtgagcaa aaggccagca 1020
   aaaggccagg aaccgtaaaa aggccgcgtt gctggcgttt ttccataggc tccgcccccc 1080
   tgacgagcat cacaaaaatc gacgctcaag tcagaggtgg cgaaacccga caggactata 1140
   aagataccag gcgtttcccc ctggaagctc cctcgtgcgc tctcctgttc cgaccctgcc 1200
   gcttaccgga tacctgtccg cctttctccc ttcgggaagc gtggcgcttt ctcatagctc 1260
   acgctgtagg tatctcagtt cggtgtaggt cgttcgctcc aagctgggct gtgtgcacga 1320
   accccccgtt cagcccgacc gctgcgcctt atccggtaac tatcgtcttg agtccaaccc 1380
   ggtaagacac gacttatcgc cactggcagc agccactggt aacaggatta gcagagcgag 1440
   gtatgtaggc ggtgctacag agttcttgaa gtggtggcct aactacggct acactagaag 1500
   aacagtattt ggtatctgcg ctctgctgaa gccagttacc ttcggaaaaa gagttggtag 1560
   ctcttgatcc ggcaaacaaa ccaccgctgg tagcggtggt ttttttgttt acaagcagca 1620
   gattacgcgc agaaaaaaag gatctcaaga agatcctttg atcttttcta cggggtctga 1680
   cgctcagcta gcgctcagaa gaactcgtca agaaggcgat agaaggcgat gcgctgcgaa 1740
   tcgggagcgg cgataccgta aagcacgagg aagcggtcag cccattcgcc gccaagctct 1800
   tcagcaatat cacgggtagc caacgctatg tcctgatagc ggtccgccac acccagccgg 1860
   ccacagtcga tgaatccaga aaagcggcca ttttccacca tgatattcgg caagcaggca 1920
   tcgccatgag tcacgacgag atcctcgccg tcgggcatgc gcgccttgag cctggcgaac 1980
   agttcggctg gcgcgagccc ctgatgctct tcgtccagat catcctgatc gacaagaccg 2040
   gcttccatcc gagtacgtgc tcgctcgatg cgatgtttcg cttggtggtc gaatgggcag 2100
   gtagccggat caagcgtatg cagccgccgc attgcatcag ccatgatgga tactttctcg 2160
   gcaggagcaa ggtgagatga caggagatcc tgccccggca cttcgcccaa tagcagccag 2220
   tcccttcccg cttcagtgac aacgtcgagc acagctgcgc aaggaacgcc cgtcgtggcc 2280
   agccacgata gccgcgctgc ctcgtcctgc agttcattca gggcaccgga caggtcggtc 2340
   ttgacaaaaa gaaccgggcg cccctgcgct gacagccgga acacggcggc atcagagcag 2400
   ccgattgtct gttgtgccca gtcatagccg aatagcctct ccacccaagc ggccggagaa 2460
   cctgcgtgca atccatcttg ttcaatcatg cgaaacgatc ctcatcctgt ctcttgatca 2520
   gatcttgatc ccctgcgcca tcagatcctt ggcggcaaga aagccatcca gtttactttg 2580
   cagggcttcc caaccttacc agagggcgcc ccagctggca attccggttc gcttgctgtc 2640
   cataaaaccg cccagtctag caactgttgg gaagggcgat cgtgtaatac gactcactat 2700
   agggcgaatt ggagct 2716
<210> 32
   <211> 2725
   <212> DNA
   <213> artificial sequence
<220>
   <223> This is the codon optimized chicken-GHRH expression plasmid.
<400> 32
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggccct gtgggtgttc 420
   tttgtgctgc tgaccctgac ctccggaagc cactgcagcc tgccacccag cccacccttc 480
   cgcgtcaggc gccacgccga cggcatcttc agcaaggcct accgcaagct cctgggccag 540
   ctgagcgcac gcaactacct gcacagcctg atggccaagc gcgtgggcag cggactggga 600
   gacgaggccg agcccctgag ctgataagct tatcggggtg gcatccctgt gacccctccc 660
   cagtgcctct cctggccctg gaagttgcca ctccagtgcc caccagcctt gtcctaataa 720
   aattaagttg catcattttg tctgactagg tgtccttcta taatattatg gggtggaggg 780
   gggtggtatg gagcaagggg caagttggga agacaacctg tagggctcga gggggggccc 840
   ggtaccagct tttgttccct ttagtgaggg ttaatttcga gcttggtctt ccgcttcctc 900
   gctcactgac tcgctgcgct cggtcgttcg gctgcggcga gcggtatcag ctcactcaaa 960
   ggcggtaata cggttatcca cagaatcagg ggataacgca ggaaagaaca tgtgagcaaa 1020
   aggccagcaa aaggccagga accgtaaaaa ggccgcgttg ctggcgtttt tccataggct 1080
   ccgcccccct gacgagcatc acaaaaatcg acgctcaagt cagaggtggc gaaacccgac 1140
   aggactataa agataccagg cgtttccccc tggaagctcc ctcgtgcgct ctcctgttcc 1200
   gaccctgccg cttaccggat acctgtccgc ctttctccct tcgggaagcg tggcgctttc 1260
   tcatagctca cgctgtaggt atctcagttc ggtgtaggtc gttcgctcca agctgggctg 1320
   tgtgcacgaa ccccccgttc agcccgaccg ctgcgcctta tccggtaact atcgtcttga 1380
   gtccaacccg gtaagacacg acttatcgcc actggcagca gccactggta acaggattag 1440
   cagagcgagg tatgtaggcg gtgctacaga gttcttgaag tggtggccta actacggcta 1500
   cactagaaga acagtatttg gtatctgcgc tctgctgaag ccagttacct tcggaaaaag 1560
   agttggtagc tcttgatccg gcaaacaaac caccgctggt agcggtggtt tttttgttta 1620
   caagcagcag attacgcgca gaaaaaaagg atctcaagaa gatcctttga tcttttctac 1680
   ggggtctgac gctcagctag cgctcagaag aactcgtcaa gaaggcgata gaaggcgatg 1740
   cgctgcgaat cgggagcggc gataccgtaa agcacgagga agcggtcagc ccattcgccg 1800
   ccaagctctt cagcaatatc acgggtagcc aacgctatgt cctgatagcg gtccgccaca 1860
   cccagccggc cacagtcgat gaatccagaa aagcggccat tttccaccat gatattcggc 1920
   aagcaggcat cgccatgagt cacgacgaga tcctcgccgt cgggcatgcg cgccttgagc 1980
   ctggcgaaca gttcggctgg cgcgagcccc tgatgctctt cgtccagatc atcctgatcg 2040
   acaagaccgg cttccatccg agtacgtgct cgctcgatgc gatgtttcgc ttggtggtcg 2100
   aatgggcagg tagccggatc aagcgtatgc agccgccgca ttgcatcagc catgatggat 2160
   actttctcgg caggagcaag gtgagatgac aggagatcct gccccggcac ttcgcccaat 2220
   agcagccagt cccttcccgc ttcagtgaca acgtcgagca cagctgcgca aggaacgccc 2280
   gtcgtggcca gccacgatag ccgcgctgcc tcgtcctgca gttcattcag ggcaccggac 2340
   aggtcggtct tgacaaaaag aaccgggcgc ccctgcgctg acagccggaa cacggcggca 2400
   tcagagcagc cgattgtctg ttgtgcccag tcatagccga atagcctctc cacccaagcg 2460
   gccggagaac ctgcgtgcaa tccatcttgt tcaatcatgc gaaacgatcc tcatcctgtc 2520
   tcttgatcag atcttgatcc cctgcgccat cagatccttg gcggcaagaa agccatccag 2580
   tttactttgc agggcttccc aaccttacca gagggcgccc cagctggcaa ttccggttcg 2640
   cttgctgtcc ataaaaccgc ccagtctagc aactgttggg aagggcgatc gtgtaatacg 2700
   actcactata gggcgaattg gagct 2725
<210> 33
   <211> 2700
   <212> DNA
   <213> artificial sequence
<220>
   <223> This is the optimized plasmid for Horse GHRH.
<400> 33
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttctttgtga tcctcaccct cagcaacagc tcccactgct ccccacctcc ccctttgacc 480
   ctcaggatgc ggcggtatgc agatgccatc ttcaccaaca gctaccggaa ggtgctgggc 540
   cagctgtccg cccgcaagct gctgcaggac atcatgagca ggcagcaggg agagagcaac 600
   caagagcgag gagcataatg aaagcttatc ggggtggcat ccctgtgacc cctccccagt 660
   gcctctcctg gccctggaag ttgccactcc agtgcccacc agccttgtcc taataaaatt 720
   aagttgcatc attttgtctg actaggtgtc cttctataat attatggggt ggaggggggt 780
   ggtatggagc aaggggcaag ttgggaagac aacctgtagg gctcgagggg gggcccggta 840
   ccagcttttg ttccctttag tgagggttaa tttcgagctt ggtcttccgc ttcctcgctc 900
   actgactcgc tgcgctcggt cgttcggctg cggcgagcgg tatcagctca ctcaaaggcg 960
   gtaatacggt tatccacaga atcaggggat aacgcaggaa agaacatgtg agcaaaaggc 1020
   cagcaaaagg ccaggaaccg taaaaaggcc gcgttgctgg cgtttttcca taggctccgc 1080
   ccccctgacg agcatcacaa aaatcgacgc tcaagtcaga ggtggcgaaa cccgacagga 1140
   ctataaagat accaggcgtt tccccctgga agctccctcg tgcgctctcc tgttccgacc 1200
   ctgccgctta ccggatacct gtccgccttt ctcccttcgg gaagcgtggc gctttctcat 1260
   agctcacgct gtaggtatct cagttcggtg taggtcgttc gctccaagct gggctgtgtg 1320
   cacgaacccc ccgttcagcc cgaccgctgc gccttatccg gtaactatcg tcttgagtcc 1380
   aacccggtaa gacacgactt atcgccactg gcagcagcca ctggtaacag gattagcaga 1440
   gcgaggtatg taggcggtgc tacagagttc ttgaagtggt ggcctaacta cggctacact 1500
   agaagaacag tatttggtat ctgcgctctg ctgaagccag ttaccttcgg aaaaagagtt 1560
   ggtagctctt gatccggcaa acaaaccacc gctggtagcg gtggtttttt tgtttacaag 1620
   cagcagatta cgcgcagaaa aaaggatctc aagaagatcc tttgatcttt tctacggggt 1680
   tctgacgctc agctagcgct cagaagaact cgtcaagaag gcgatagaag gcgatgcgct 1740
   gcgaatcggg agcggcgata ccgtaaagca cgaggaagcg gtcagcccat tcgccgccaa 1800
   gctcttcagc aatatcacgg gtagccaacg ctatgtcctg atagcggtcc gccacaccca 1860
   gccggccaca gtcgatgaat ccagaaaagc ggccattttc caccatgata ttcggcaagc 1920
   aggcatcgcc atgagtcacg acgagatcct cgccgtcggg catgcgcgcc ttgagcctgg 1980
   cgaacagttc ggctggcgcg agcccctgat gctcttcgtc cagatcatcc tgatcgacaa 2040
   gaccggcttc catccgagta cgtgctcgct cgatgcgatg tttcgcttgg tggtcgaatg 2100
   ggcaggtagc cggatcaagc gtatgcagcc gccgcattgc atcagccatg atggatactt 2160
   tctcggcagg agcaaggtga gatgacagga gatcctgccc cggcacttcg cccaatagca 2220
   gccagtccct tcccgcttca gtgacaacgt cgagcacagc tgcgcaagga acgcccgtcg 2280
   tggccagcca cgatagccgc gctgcctcgt cctgcagttc attcagggca ccggacaggt 2340
   cggtcttgac aaaaagaacc gggcgcccct gcgctgacag ccggaacacg gcggcatcag 2400
   agcagccgat tgtctgttgt gcccagtcat agccgaatag cctctccacc caagcggccg 2460
   gagaacctgc gtgcaatcca tcttgttcaa tcatgcgaaa cgatcctcat cctgtctctt 2520
   gatcagatct tgatcccctg cgccatcaga tccttggcgg caagaaagcc atccagttta 2580
   ctttgcaggg cttcccaacc ttaccagagg gcgccccagc tggcaattcc ggttcgcttg 2640
   ctgtccataa aaccgcccag tctagcaact gttgggaagg gcgatcgtgt aatacgactc 2700
<210> 34
   <211> 2721
   <212> DNA
   <213> artificial sequence
<220>
   <223> This is the codon optimized Human-GHRH expression plasmid.
<400> 34
   ccaccgcggt ggcggccgtc cgccctcggc accatcctca cgacacccaa atatggcgac 60
   gggtgaggaa tggtggggag ttatttttag agcggtgagg aaggtgggca ggcagcaggt 120
   gttggcgctc taaaaataac tcccgggagt tatttttaga gcggaggaat ggtggacacc 180
   caaatatggc gacggttcct cacccgtcgc catatttggg tgtccgccct cggccggggc 240
   cgcattcctg ggggccgggc ggtgctcccg cccgcctcga taaaaggctc cggggccggc 300
   ggcggcccac gagctacccg gaggagcggg aggcgccaag cggatcccaa ggcccaactc 360
   cccgaaccac tcagggtcct gtggacagct cacctagctg ccatggtgct ctgggtgttc 420
   ttctttgtga tcctcaccct cagcaacagc tcccactgct ccccacctcc ccctttgacc 480
   ctcaggatgc ggcggtatgc agatgccatc ttcaccaaca gctaccggaa ggtgctgggc 540
   cagctgtccg cccgcaagct gctgcaggac atcatgagca ggcagcaggg agagagcaac 600
   caagagcgag gagcataatg aaagcttatc ggggtggcat ccctgtgacc cctccccagt 660
   gcctctcctg gccctggaag ttgccactcc agtgcccacc agccttgtcc taataaaatt 720
   aagttgcatc attttgtctg actaggtgtc cttctataat attatggggt ggaggggggt 780
   ggtatggagc aaggggcaag ttgggaagac aacctgtagg gctcgagggg gggcccggta 840
   ccagcttttg ttccctttag tgagggttaa tttcgagctt ggtcttccgc ttcctcgctc 900
   actgactcgc tgcgctcggt cgttcggctg cggcgagcgg tatcagctca ctcaaaggcg 960
   gtaatacggt tatccacaga atcaggggat aacgcaggaa agaacatgtg agcaaaaggc 1020
   cagcaaaagg ccaggaaccg taaaaaggcc gcgttgctgg cgtttttcca taggctccgc 1080
   ccccctgacg agcatcacaa aaatcgacgc tcaagtcaga ggtggcgaaa cccgacagga 1140
   ctataaagat accaggcgtt tccccctgga agctccctcg tgcgctctcc tgttccgacc 1200
   ctgccgctta ccggatacct gtccgccttt ctcccttcgg gaagcgtggc gctttctcat 1260
   agctcacgct gtaggtatct cagttcggtg taggtcgttc gctccaagct gggctgtgtg 1320
   cacgaacccc ccgttcagcc cgaccgctgc gccttatccg gtaactatcg tcttgagtcc 1380
   aacccggtaa gacacgactt atcgccactg gcagcagcca ctggtaacag gattagcaga 1440
   gcgaggtatg taggcggtgc tacagagttc ttgaagtggt ggcctaacta cggctacact 1500
   agaagaacag tatttggtat ctgcgctctg ctgaagccag ttaccttcgg aaaaagagtt 1560
   ggtagctctt gatccggcaa acaaaccacc gctggtagcg gtggtttttt tgtttacaag 1620
   cagcagatta cgcgcagaaa aaaaggatct caagaagatc ctttgatctt ttctacgggg 1680
   tctgacgctc agctagcgct cagaagaact cgtcaagaag gcgatagaag gcgatgcgct 1740
   gcgaatcggg agcggcgata ccgtaaagca cgaggaagcg gtcagcccat tcgccgccaa 1800
   gctcttcagc aatatcacgg gtagccaacg ctatgtcctg atagcggtcc gccacaccca 1860
   gccggccaca gtcgatgaat ccagaaaagc ggccattttc caccatgata ttcggcaagc 1920
   aggcatcgcc atgagtcacg acgagatcct cgccgtcggg catgcgcgcc ttgagcctgg 1980
   cgaacagttc ggctggcgcg agcccctgat gctcttcgtc cagatcatcc tgatcgacaa 2040
   gaccggcttc catccgagta cgtgctcgct cgatgcgatg tttcgcttgg tggtcgaatg 2100
   ggcaggtagc cggatcaagc gtatgcagcc gccgcattgc atcagccatg atggatactt 2160
   tctcggcagg agcaaggtga gatgacagga gatcctgccc cggcacttcg cccaatagca 2220
   gccagtccct tcccgcttca gtgacaacgt cgagcacagc tgcgcaagga acgcccgtcg 2280
   tggccagcca cgatagccgc gctgcctcgt cctgcagttc attcagggca ccggacaggt 2340
   cggtcttgac aaaaagaacc gggcgcccct gcgctgacag ccggaacacg gcggcatcag 2400
   agcagccgat tgtctgttgt gcccagtcat agccgaatag cctctccacc caagcggccg 2460
   gagaacctgc gtgcaatcca tcttgttcaa tcatgcgaaa cgatcctcat cctgtctctt 2520
   gatcagatct tgatcccctg cgccatcaga tccttggcgg caagaaagcc atccagttta 2580
   ctttgcaggg cttcccaacc ttaccagagg gcgccccagc tggcaattcc ggttcgcttg 2640
   ctgtccataa aaccgcccag tctagcaact gttgggaagg gcgatcgtgt aatacgactc 2700
   actatagggc gaattggagc t 2721
<210> 35
   <211> 225
   <212> DNA
   <213> artificial sequence
<220>
   <223> Sequence for an analog bovine GHRH sequence.
<400> 35
   gccatggtgc tgtgggtgtt cttcctggtg accctgaccc tgagcagcgg ctcccacggc 60
   tccctgccct cccagcctct gcgcatccct cgctacgccg acgccatctt caccaacagc 120
   taccgcaagg tgctcggcca gctcagcgcc cgcaagctcc tgcaggacat catgaaccgg 180
   cagcagggcg agcgcaacca ggagcaggga gcctgataag cttgc 225
<210> 36
   <211> 225
   <212> DNA
   <213> artificial sequence
<220>
   <223> Sequence for an analog ovine GHRH sequence.
<400> 36
   gccatggtgc tgtgggtgtt cttcctggtg accctgaccc tgagcagcgg aagccacggc 60
   agcctgccca gccagcccct gaggatccct aggtacgccg acgccatctt caccaacagc 120
   tacaggaaga tcctgggcca gctgagcgct aggaagctcc tgcaggacat catgaacagg 180
   cagcagggcg agaggaacca ggagcagggc gcctgataag cttgc 225
<210> 37
   <211> 246
   <212> DNA
   <213> artificial sequence
<220>
   <223> Sequence for an analog chicken GHRH sequence.
<400> 37
   gccatggtgc tctgggtgct ctttgtgatc ctcatcctca ccagcggcag ccactgcagc 60
   ctgcctccca gccctccctt caggatgcag aggcacgtgg acgccatctt caccaccaac 120
   tacaggaagc tgctgagcca gctgtacgcc aggaaggtga tccaggacat catgaacaag 180
   cagggcgaga ggatccagga gcagagggcc aggctgagct gataagcttg cgatgagttc 240
   ttctaa 246
<210> 38
   <211> 190
   <212> DNA
   <213> artificial sequence
<220>
   <223> Nucleic acid sequence of human growth hormone poly A tail.
<400> 38
   gggtggcatc cctgtgaccc ctccccagtg cctctcctgg ccctggaagt tgccactcca 60
   gtgcccacca gccttgtcct aataaaatta agttgcatca ttttgtctga ctaggtgtcc 120
   ttctataata ttatggggtg gaggggggtg gtatggagca aggggcaagt tgggaagaca 180
   acctgtaggg 190
<210> 39
   <211> 55
   <212> DNA
   <213> artificial sequence
<220>
   <223> Nucleic acid sequence of human growth hormone 5' UTR
<400> 39
   caaggcccaa ctccccgaac cactcagggt cctgtggaca gctcacctag ctgcc 55
<210> 40
   <211> 782
   <212> DNA
   <213> artificial sequence
<220>
   <223> Nucleic acid sequence of a plasmid pUC-18 origin of replication
<400> 40
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   tt 782
<210> 41
   <211> 5
   <212> DNA
   <213> artificial sequence
<220>
   <223> This is a NEO ribosomal binding site
<400> 41
   tcctc 5

## Description

This application claims priority to U.S. Provisional Patent Application, Serial Number 60/590,739, entitled "GROWTH HORMONE RELEASING HORMONE ENHANCES VACCINATION RESPONSE" filed on July 23, 2004, having Ruxandra Draghia-Akli, Patricia A. Brown, Amir S. Khan and William C. Davis, listed as the inventors.

This invention pertains to compositions and methods of: vaccinating a subject; enhancing the vaccination efficiency; preparing a subject prior to vaccination; and improving the clinical outcome after infectious challenge in a subject that has been vaccinated. More specifically, the invention pertains to delivering into a tissue of the subject a nucleic acid expression construct that encodes a growth-hormone-releasing-hormone ("GHRH") before or concomitantly with delivering a vaccine to the subject, wherein, GHRH is expressed *in vivo* in the subject and the subject comprises a human, pig, cow, bird or any other animal species receiving a vaccine.

Infectious disease remains a significant problem in both humans and animals. Thus, age-appropriate antibiotic selection and evaluation of the clinical effectiveness of the specific vaccine as well as other immune-enhancing therapies are required. Substantial efforts have addressed the prevention rather than treatment of disease. The yearly market for vaccination is over $20 billion. Numerous reports indicate an interdependent relationship between the neuroendocrine and the immune systems. Hypothalamic GHRH stimulates growth hormone ("GH") secretion from the anterior pituitary gland, but recent studies have also demonstrated the immunomodulatory properties of this peptide (Siejka et al., 2004). The importance of GHRH in the modulation of immune status under physiological and pathological conditions (Marshall et al., 2001) has been described, both through stimulation of the GH/insulin-like growth factor-I ("IGF-I") axis and directly as an immune modulator (Dialynas et al., 1999; Khorram et al., 2001). GHRH is integral in the development and regulation of the immune system. Detail is still lacking, however, on exactly how GHRH mediates those effects or the impact of GHRH treatment on vaccination and pathogen challenge.

### Growth Hormone Releasing Hormone ("GHRH") and Growth

**Hormone ("GH") Axis:** To better understand utilizing GHRH plasmid-mediated supplementation as a method to enhance a specific vaccination response and to improve the clinical outcome after an infectious challenge, the mechanisms and current understanding of the GHRH/GH axis will be addressed. Although not wanting to be bound by theory, the central role of GH is controlling somatic growth in humans and other vertebrates. The physiologically relevant pathways regulating GH secretion from the pituitary are fairly well known. The GH pathway genes include: (1) ligands, such as GH and IGF-I; (2) transcription factors such as prophet of pit 1, or prop 1, and pit 1: (3) agonists and antagonists, such as GHRH and somatostatin ("SS"), respectively; and (4) receptors, such as GHRH receptor ("GHRH-R") and the GH receptor ("GH-R"). These genes are expressed in different organs and tissues, including the hypothalamus, pituitary, liver, and bone. Effective and regulated expression of the GH pathway is essential for optimal linear growth, as well as homeostasis of carbohydrate, protein, and fat metabolism. GH synthesis and secretion from the anterior pituitary is stimulated by GHRH and inhibited by somatostatin, both hypothalamic hormones. GH increases production of IGF-I, primarily in the liver, and other target organs. IGF-I and GH, in turn, feedback on the hypothalamus and pituitary to inhibit GHRH and GH release. GH elicits both direct and indirect actions on peripheral tissues, the indirect effects being mediated mainly by IGF-I.

**GHRH and the Immune Function:** Plasmid-mediated GHRH supplementation has been shown to have a variety of immunostimulatory effects in animals with depressed immune systems due to illness or various treatment regimens (Dorshkind and Horseman, 2001). Studies indicate that cells of the immune system produce GHRH, GH and IGF-I (Burgess et al., 1999) suggesting that immune function might be regulated by both autocrine and paracrine mechanisms. It has also been suggested that the increased morbidity in the elderly, such as respiratory disease, may be causally related to changes that occur with aging: decreased GH/IGF-I production, reduced IGF-I availability and decreased immune surveillance, especially T-cell mediated (Gelato, 1996; Krishnaraj et al., 1998). Conversely, administration of GHRH or its analogs in the elderly has resulted in profound immuno-enhancing effects, both short- and long-term after therapy. These effects include an increased number of lymphocytes, monocytes, B-cells as well as cells expressing T-cell receptor αβ and T-cell receptor γδ (Khorram et al., 1997). In immunocompromised patients, e.g., after bone marrow transplantation, IGF-I administration can enhance lymphoid and myeloid reconstitution (Alpdogan et al., 2003). Also, studies of animal models of disease and vaccination show that *in vivo* administration of GH can effectively prime macrophages and increase the resistance to pathogens (Sakai et al., 1997).

Several studies in different animal models and human have shown that GHRH has an immune stimulatory effect, both through stimulation of the GH axis and directly as an immune-modulator (Dialynas et al., 1999; Khorram et al., 2001). GH has been known to enhance immune responses, whether directly or through the IGF-I, induced by GH. Recently, a GH secretagogue ("GHS") was found to induce the production of GH by the pituitary gland, but also determined a statistically significant increase in thymic cellularity and differentiation in old mice. When inoculated with a transplantable lymphoma cell line, EL4, the treated old mice showed statistically significant resistance to the initiation of tumors and the subsequent metastases. Generation of CTL to EL4 cells was also enhanced in the treated mice, suggesting that GHS has a considerable immune enhancing effect (Koo et al., 2001). The immune function is also modulated by IGF-I, which has two major effects on B cell development: potentiation and maturation, and as a B-cell proliferation cofactor that works together with interlukin-7. These activities were identified through the use of anti-IGF-I antibodies, antisense sequences to IGF-I, and the use of recombinant IGF-I to substitute for the activity. The treatment of mice with recombinant IGF-I confirmed these observations as it increased the number of pre-B and mature B cells in bone marrow (Jardieu et al., 1994). The mature B cell remained sensitive to IGF-I as immunoglobulin production was also stimulated by IGF-I *in vitro* and *in vivo* (Robbins et al., 1994).

In aging mammals, the GHRH-GH-IGF-I axis undergoes considerable decrement having reduced GH secretion and IGF-I production associated with a loss of skeletal muscle mass (sarcopenia), osteoporosis, arthritis, increased fat deposition and decreased lean body mass (Caroni and Schneider, 1994; Veldhuis et al., 1997). It has been demonstrated that the development of these changes can be offset by recombinant GH therapy. A therapy that would address both the increase risk of infection and the wasting would be a major step forward in the well-being and quality of life of patients.

The production of recombinant proteins in the last 2 decades provided a useful tool for the treatment of many diverse conditions. For example, GH-deficiencies in short stature children, anabolic agent in burn, sepsis, and AIDS patients. However, resistance to GH action has been reported in malnutrition and infection. Current GH therapy has several shortcomings, however, including frequent subcutaneous or intravenous injections, insulin resistance and impaired glucose tolerance (Rabinovsky et al., 1992); children are also vulnerable to premature epiphyseal closure and slippage of the capital femoral epiphysis (Liu and LeRoith, 1999). A "slow-release" form of GH (from Genentech) has been developed that only requires injections every 14 days. However, this GH product appears to perturb the normal physiological pulsatile GH profile, and is also associated with frequent side effects.

**Growth Hormone Releasing Hormone versus Growth Hormone or Growth Hormone Releasing Peptides ("GHRP"):** GH and GHRH are currently administered therapeutically as recombinant proteins. Current knowledge about the interaction between GH and its receptor suggests that the molecular heterogeneity of circulating GH may have important homeostasis implications. It has been suggested that adverse effects including insulin resistance, may result from the fact that exogenous GH elevates the basal GH serum levels and abolishes the natural GH episodic pulses. Studies have shown that continuous infusion with GHRH restores normal GH pulsatile pattern, without desensitization of GHRH receptors or depletion of GH supplies in humans, sheep or pigs (Dubreuil et al., 1990; Vance et al., 1989; Vance et al., 1985). At the same time, this system is capable of feed-back, which is totally abolished in the GH therapies. Virtually no side effects have been reported for GHRH therapies (Thorner et al., 1986a). Thus, GHRH therapy may be more physiological than GH therapy.

GHRPs are used in clinics to stimulate short term GH and IGF-I in humans. Hexarelin, a potent and well-studied GHRP, is capable of causing profound GH release in normal individuals. The GH response to hexarelin in humans becomes appreciably attenuated following long-term administration. Although this attenuation is partial and reversible, it could seriously limit the potential long-term therapeutic use of hexarelin and similar agents (Rahim and Shalet, 1998). Long-term therapy with hexarelin, in association with a vaccine, is needed to stimulate immune function. With the development of GH-releasing agents and their use in human subjects, it is clear that these agents are not specific for GH release. More recent studies in humans have demonstrated that acute increases in adrenocorticotrophic hormone (ACTH) (Ghigo et al., 1999), cortisol and prolactin (PRL) (Svensson and Bengtsson, 1999) have occurred after administration of GHRPs (hexarelin, MK-0677) (Schleim et al., 1999). The potential adverse effects of repeated episodes of transient (even minor) hyperprolactinaemia and hypercortisolaemia during long-term therapy with GHRPs and similar agents raise concern, require further study, and are undesirable in patients facing an infectious challenge (Rahim et al., 1999).

In contrast, essentially no side effects have been reported for recombinant GHRH therapies. Extracranially secreted GHRH, as mature peptide or truncated molecules (as seen with pancreatic islet cell tumors and variously located carcinoids) are often biologically active and can even produce acromegaly (Esch et al., 1982; Thomer et al., 1984). Administration of recombinant GHRH to GH-deficient children or adult humans augments IGF-I levels, increases GH secretion proportionally to the GHRH dose, yet still invokes a response to bolus doses of recombinant GHRH (Bercu and Walker, 1997). Thus, GHRH administration represents a more physiological alternative of increasing subnormal GH and IGF-I levels (Corpas et al., 1993).

Although recombinant GHRH protein therapy entrains and stimulates normal cyclical GH secretion with virtually no side effects, the short half-life of GHRH *in vivo* requires frequent (one to three times a day) intravenous, subcutaneous or intranasal (requiring 300-fold higher dose) administration. Thus, as a chronic treatment, GHRH administration is not practical.

Wild type GHRH has a relatively short half-life in the circulatory system, both in humans (Frohman et al., 1984) and in farm animals. After 60 minutes of incubation in plasma 95% of the GHRH(1-44)NH2 is degraded, while incubation of the shorter (1-40)OH form of the hormone, under similar conditions, shows only a 77% degradation of the peptide after 60 minutes of incubation (Frohman et al., 1989). Incorporation of cDNA coding for a particular protease-resistant GHRH analog in a therapeutic nucleic acid vector results in a molecule with a longer half-life in serum, increased potency, and provides greater GH release in plasmid-injected animals (Draghia-Akli et al., 1999). Mutagenesis *via* amino acid replacement of protease sensitive amino acids prolongs the serum half-life of the GHRH molecule. Furthermore, the enhancement of biological activity of GHRH is achieved by using super-active analogs that may increase its binding affinity to specific receptors (Draghia-Akli et al., 1999).

**Growth Hormone ("GH") and Growth Hormone Releasing Hormone ("GHRH") in Farm animals:** The administration of recombinant GH or recombinant GH has been used in subjects for many years, but not as a pathway to stimulate the response after vaccination, or to improve the clinical outcome after an infectious challenge. More specifically, recombinant GH treatment in farm animals has been shown to enhance lean tissue deposition and/or milk production, while increasing feed efficiency (Etherton et al., 1986; Klindt et al., 1998). Numerous studies have shown that recombinant GH markedly reduces the amount of carcass fat; and consequently the quality of products increases. However, chronic GH administration has practical, economical and physiological limitations that potentially mitigate its usefulness and effectiveness (Chung et al., 1985; Gopinath and Etherton, 1989b). Experimentally, recombinant GH-releasing hormone ("GHRH") has been used as a more physiological alternative. The use of GHRH in large animal species (e.g. pigs or cattle) not only enhances growth performance and milk production, but more importantly, the efficiency of production from both a practical and metabolic perspective (Dubreuil et al., 1990; Farmer et al., 1992). For example, the use of recombinant GHRH in lactating sows has beneficial effects on growth of the weanling pigs, yet optimal nutritional and hormonal conditions are needed for GHRH to exert its full potential (Farmer et al., 1996). Administration of GHRH and GH stimulate milk production, with an increase in feed to milk conversion. This therapy enhances growth primarily by increasing lean body mass (Lapierre et al., 1991; van Rooij et al., 2000) with overall improvement in feed efficiency. Hot and chilled carcass weights are increased and carcass lipid (percent of soft-tissue mass) is decrease by administration of GHRH and GH (Etherton et al., 1986).

**Transgene Delivery and *in vivo* Expression:** Although not wanting to be bound by theory, the delivery of a specific transgene to somatic tissue to correct inborn or acquired deficiencies and imbalances is possible. Such transgene-based delivery offers a number of advantages over the administration of recombinant proteins. These advantages include: the conservation of native protein structure; improved biological activity; avoidance of systemic toxicities; and avoidance of infectious and toxic impurities. Because the protein is synthesized and secreted continuously into the circulation, plasmid-mediated therapy allows for prolonged production of the protein in a therapeutic range. In contrast, the primary limitation of using recombinant protein is the limited bio-availability of protein after each administration.

In a plasmid-based expression system, a non-viral vector may comprise of a synthetic transgene delivery system in addition to the nucleic acid encoding the therapeutic genetic product. In this way, the risks associated with the use of most viral vectors can be avoided, including the expression of viral proteins that can induce immune responses against the target tissues or the viral vector and the possibility of DNA mutations or activations of oncogenes. The non-viral expression vector products generally have low toxicity due to the use of "species-specific" components for gene delivery, which minimizes the risks of plasmid-targeted immunogenicity and loss of expression. Additionally, no significant integration of plasmid sequences above the rate of spontaneous mutation into host chromosomes has been reported *in vivo* to date, so that this type of therapy should neither activate oncogenes nor inactivate tumor suppressor genes. As episomal systems residing outside the chromosomes, plasmids have defined pharmacokinetics and elimination profiles, leading to a finite duration of gene expression in target tissues. Plasmid vectors are simple to manufacture using good manufacturing practice techniques. They have a low risk to benefit ratio when compared to viral vectors, as stated on March 13-14, 2003 in a workshop sponsored by the American Society of Gene Therapy (ASGT) and the Food and Drug Administration's Center for Biologics Evaluation and Research (FDA/CBER) (Frederickson et al., 2003).

Direct plasmid DNA gene transfer is currently the basis of many emerging nucleic acid therapy strategies and does not require viral components or lipid particles (Aihara and Miyazaki, 1998; Muramatsu et al., 2001). Skeletal muscle is target tissue, because muscle fiber has a long life span and can be transduced by circular DNA plasmids that are expressed in immunocompetent hosts (Davis et al., 1993; Tripathy et al., 1996). Plasmid DNA constructs are attractive candidates for direct therapy into the subjects skeletal muscle because the constructs are well-defined entities that are biochemically stable and have been used successfully for many years (Acsadi et al., 1991; Wolff et al., 1990). The relatively low expression levels of an encoded product that are achieved after direct plasmid DNA injection are sometimes sufficient to indicate bioactivity of secreted peptides (Danko and Wolff, 1994; Tsurumi et al., 1996). Previous reports showed that human GHRH cDNA could be delivered to muscle by a plasmid in mice where it transiently stimulated GH secretion to a modest extent over a period of two weeks (Draghia-Akli et al., 1997).

**Plasmid-mediated GHRH supplementation stimulates immune function:** Preliminary studies in healthy dogs suggested that a single administration of a GHRH plasmid into skeletal muscle will ensure physiologic GHRH expression for several months (Draghia-Akli et al., 2003a). An initial study was designed to assess the ability of the plasmid-based GHRH treatment to produce beneficial effects in geriatric or cancer-afflicted companion dogs, and to assess long-term safety of the treatment regimen. A muscle-specific GHRH-expressing plasmid to 16 dogs afflicted with cancer was administered. The initial 56-day evaluation (Draghia-Akli et al., 2002a) demonstrated increased serum IGF-I concentrations, an indicator of GHRH bioactivity. A significant increase in circulating lymphocyte levels was found in treated animals. A further pilot study was conducted with severely debilitated geriatric and companion dogs with spontaneously occurring tumors. In this case, IGF-I levels were found to be elevated more than 365 days post-treatment. For the longitudinal continuation of this study, dogs that could be analyzed for at least 180 days post-treatment were included. Increases in weight, activity level and exercise tolerance in addition to improvement and maintenance of hematological parameters were observed. The overall long-term assessment of the treated dogs showed improvement in quality of life that was maintained throughout the study period. These results suggest a role for plasmid-mediated GHRH treatment in reversing the catabolic processes associated with aging and cancer anemia and/or cachexia, and suggest that the improved well-being may be associated with stimulation of immune function.

**Plasmid delivery and electroporation:** Efforts have been made to enhance the delivery of plasmid DNA to cells by physical means including electroporation, sonoporation, and pressure. Although not wanting to be bound by theory, the administration of a nucleic acid construct by electroporation involves the application of a pulsed electric field to create transient pores in the cellular membrane without causing permanent damage to the cell, which allows exogenous molecules to enter the cell (Smith and Nordstrom, 2000). By adjusting the electrical pulse generated by an electroporetic system, nucleic acid molecules can travel through passageways or pores in the cell that are created during the procedure. United States Patent 5,704,908 titled "Electroporation and iontophoresis catheter with porous balloon," issued on January 6, 1998 with Hofmann et al., listed as inventors describes an electroporation apparatus for delivering molecules to cells at a selected location within a cavity in the body of a patient. Similar pulse voltage injection devices are also described in: United States Patent 5,702,359 titled "Needle electrodes for mediated delivery of drugs and genes," issued on December 30, 1997, with Hofmann, et al., listed as inventors; United States Patent 5,439,440 titled "Electroporation system with voltage control feedback for clinical applications," issued on August 8, 1995 with Hofmann listed as inventor; PCT application WO/96/12520 titled "Electroporetic Gene and Drug Therapy by Induced Electric Fields," published on May 5, 1996 with Hofmann et al., listed as inventors; PCT application WO/96/12006 titled "Flow Through Electroporation Apparatus and Method," published on April 25, 1996 with Hofmann et al., listed as inventors; PCT application WO/95/19805 titled "Electroporation and Iontophoresis Apparatus and Method For insertion of Drugs and genes into Cells," published on July 27, 1995 with Hofmann listed as inventor; and PCT application WO/97/07826 titled "In Vivo Electroporation of Cells," published on March 6, 1997, with Nicolau et al., listed as inventors.

Recently, significant progress to enhance plasmid delivery *in vivo* and subsequently to achieve physiological levels of a secreted protein was obtained using the electroporation technique. Electroporation has been used very successfully to transfect tumor cells after injection of plasmid (Lucas et al., 2002; Matsubara et al., 2001)) or to deliver the anti-tumor drug bleomycin to cutaneous and subcutaneous tumors in humans (Gehl et al., 1998; Heller et al., 1996). Electroporation also has been extensively used in mice (Lesbordes et al., 2002; Lucas et al., 2001; Vilquin et al., 2001), rats (Terada et al., 2001; Yasui et al., 2001), and dogs (Fewell et al., 2001) to deliver therapeutic genes that encode for a variety of hormones, cytokines or enzymes. Previous studies using GHRH showed that plasmid therapy with electroporation is scalable and represents a promising approach to induce production and regulated secretion of proteins in large animals and humans (Draghia-Akli et al., 1999; Draghia-Akli et al., 2002c). Electroporation also has been extensively used in rodents and other small animals (Bettan et al., 2000; Yin and Tang, 2001). Intramuscular injection of plasmid followed by electroporation has been used successfully in ruminants for vaccination purposes (Babiuk et al., 2003; Tollefsen et al., 2003). It has been observed that the electrode configuration affects the electric field distribution, and subsequent results (Gehl et al., 1999; Miklavcic et al., 1998). Although not wanting to be bound by theory, needle electrodes give consistently better results than external caliper electrodes in a large animal model, and can be used for humans.

The ability of electroporation to enhance plasmid uptake into the skeletal muscle has been well documented. Similarly, plasmids formulated with poly-L-glutamate ("PLG") or polyvinylpyrrolidone ("PVP") were observed to have an increase in plasmid transfection, which consequently increased the expression of a desired transgene. For example, plasmids formulated with PLG or PVP were observed to increase gene expression to up to 10 fold in the skeletal muscle of mice, rats, and dogs (Fewell et al., 2001; Mumper et al., 1998). Although not wanting to be bound by theory, the anionic polymer sodium PLG enhances plasmid uptake at low plasmid concentrations and reduces any possible tissue damage caused by the procedure. PLG is a stable compound and it is resistant to relatively high temperatures (Dolnik et al., 1993). PLG has been used to increase stability of anti-cancer drugs (Li et al., 2000) and as "glue" to close wounds or to prevent bleeding from tissues during wound and tissue repair (Otani et al., 1996; Otani et al., 1998). PLG has been used to increase stability in vaccine preparations (Matsuo et al., 1994) without increasing their immunogenicity. PLG also has been used as an anti-toxin after antigen inhalation or exposure to ozone (Fryer and Jacoby, 1993).

Although not wanting to be bound by theory, PLG increases the transfection of the plasmid during the electroporation process, not only by stabilizing the plasmid DNA and facilitating the intracellular transport through the membrane pores, but also through an active mechanism. For example, positively charged surface proteins on the cells could complex the negatively charged PLG linked to plasmid DNA through protein-protein interactions. When an electric field is applied, the surface proteins reverse direction and actively internalize the DNA molecules, a process that substantially increases the transfection efficiency. Furthermore, PLG will prevent the muscle damage associated with *in vivo* plasmid delivery (Draghia-Akli et al., 2002b) and will increase plasmid stability *in vitro* prior to injection. There are studies directed to electroporation of eukaryotic cells with linear DNA (McNally et al., 1988; Neumann et al., 1982) (Toneguzzo et al., 1988) (Aratani et al., 1992; Nairn et al., 1993; Xie and Tsong, 1993; Yorifuji and Mikawa, 1990), but these examples illustrate transfection into cell suspensions, cell cultures, and the like, and such transfected cells are not present in a somatic tissue.

U.S. Patent No. 4,956,288 is directed to methods for preparing recombinant host cells containing high copy number of a foreign DNA by electroporating a population of cells in the presence of the foreign DNA, culturing the cells, and killing the cells having a low copy number of the foreign DNA.

Although not wanting to be bound by theory, a GHRH cDNA can be delivered to muscle of mice injectable myogenic expression vector where it can transiently stimulate GH secretion over a period of two weeks (Draghia-Akli et al., 1997). This injectable vector system was optimized by incorporating a powerful synthetic muscle promoter (Li et al., 1999) coupled with a novel protease-resistant GHRH molecule with a substantially longer half-life and greater GH secretory activity (pSP-HV-GHRH) (Draghia-Akli et al., 1999). Highly efficient electroporation technology was optimized to deliver the nucleic acid construct to the skeletal muscle of an animal (Draghia-Akli et al., 2002b). Using this combination of vector design and electric pulses plasmid delivery method, the inventors were able to show increased growth and favorably modified body composition in pigs (Draghia-Akli et al., 1999; Draghia-Akli et al., 2003b). The modified GHRH nucleic acid constructs increased red blood cell production in companion animals with cancer and cancer treatment-associated anemia (Draghia-Akli et al., 2002a). In pigs, available data suggested that the modified porcine HV-GHRH analog (SEQID#1) was more potent in promoting growth and positive body composition changes than the wild-type porcine GHRH (Draghia-Akli et al., 1999).

Administering novel GHRH analog proteins (U.S. Pat Nos. 5,847,066; 5846,936; 5,792,747; 5,776,901; 5,696,089; 5,486,505; 5,137,872; 5,084,442, 5,036,045; 5,023,322; 4,839,344; 4,410,512, RE33,699) or synthetic or naturally occurring peptide fragments of GHRH (U.S. Pat. Nos. 4,833,166; 4,228,158; 4,228,156; 4,226,857; 4,224,316; 4,223,021; 4,223,020; 4,223,019) for the purpose of increasing release of growth hormone have been reported. A GHRH analog containing the following mutations has been reported (U.S. Patent No. 5,846,936): Tyr at position 1 to His; Ala at position 2 to Val, Leu, or others; Asn at position 8 to Gln, Ser, or Thr; Gly at position 15 to Ala or Leu; Met at position 27 to Nle or Leu; and Ser at position 28 to Asn. The GHRH analog is the subject of United States Patent 6,551,996 titled "Super-active porcine growth hormone releasing hormone analog," issued on April 22, 2003 with Schwartz, et al., listed as inventors ("the '996 Patent"), which teaches application of a GHRH analog containing mutations that improve the ability to elicit the release of growth hormone. In addition, the '996 Patent application relates to the treatment of growth deficiencies; the improvement of growth performance; the stimulation of production of growth hormone in an animal at a greater level than that associated with normal growth; and the enhancement of growth utilizing the administration of growth hormone releasing hormone analog.

In summary, enhancing vaccination response and potency and improving the clinical outcome of a subject after an infectious challenge were previously uneconomical and restricted in scope. The related art has shown that it is possible to improve these different conditions in a limited capacity utilizing recombinant protein technology, but these treatments have some significant drawbacks. It has also been taught that nucleic acid expression constructs that encode recombinant proteins are viable solutions to the problems of frequent injections and high cost of traditional recombinant therapy. There is a need in the art to expanded treatments for subjects with a disease by utilizing nucleic acid expression constructs that are delivered into a subject and express stable therapeutic proteins *in vivo*.

### SUMMARY

The current invention pertains to claim 1 and claim 16. Specific embodiments of the invention pertain to delivering into a tissue of the subject a nucleic acid expression construct that encodes a growth-hormone-releasing-hormone (GHRH") before or concomitantly with delivering a vaccine to a subject in need of vaccination, wherein the GHRH is expressed *in vivo* in the subject and the subject comprises a human, pig, cow, bird or any other animal species receiving a vaccine.

The current invention is useful for a method of preparing a subject in need of vaccination. This method comprises delivering into a tissue of the subject a nucleic acid expression construct that encodes an *in vivo* expressed GHRH. The preferred GHRH of this invention comprises a sequence that is at least 90% identical to the encoded GHRH of SEQID#14, and the preferred nucleic acid expression constructs comprise a sequence that is at least 97% identical to mouse pAV0202 (SEQID#23); rat pAV0203 (SEQID#24); HV-GHRH pAV0224 (SEQID#25); pig-wt-GHRH pAV0225 (SEQID#26); dog pAV0235 (SEQID#27); bovine pAV0236 (SEQID#28); cat pAV0238 (SEQID#29); TI-GHRH pAV0239 (SEQID#30); ovine pAV0240 (SEQID#31); chicken pAV0241 (SEQID#32); horse pAV0249 (SEQID#33) or human pAV0226 (SEQID#34). This invention encompasses vaccines that comprise: killed microorganisms; live attenuated organisms; subunit antigens; toxoid antigens; conjugate antigens or other type of vaccine that when introduced into a subjects body produces immunity to a specific disease by causing the activation of the immune system, antibody formation, and/or creating of a T-cell and/or B-cell response. However preferred embodiments of the invention comprise vaccinations having: bovine herpesvirus-1 ("IBR"); bovine virus diarrhea ("BVD"); parainfluenza 3; respiratory syncytial virus; Leptospira canicola; Leptospira grippotyphosa, Leptospira hardjo; Leptospira icterohaemorrhagiae; Leptospira Pomona bacterins; mycoplasma hyopneumonia; mycoplasma hyopneumonia; or combinations thereof.

Generally the nucleic acid expression construct can be delivered into the subject up to 1 year before the subject is vaccinated, however, that time can vary. For example, in a preferred embodiment, the nucleic acid expression construct is delivered about 0 to about 14 days before the subject is vaccinated. One preferred method of delivering the nucleic acid expression construct into the tissue of the subject comprises tissue electroporation. Many methods of tissue electroporation have been described previously, however, a preferred tissue electroporation method comprises: penetrating the tissue in the subject with a plurality of needle electrodes, wherein the plurality of needle electrodes are arranged in a spaced relationship and the tissue of the subject comprise muscle cells; introducing the nucleic acid expression construct into the tissue between the plurality of needle electrodes in an amount in a range of about 0.01 - 5 mg; and applying an electrical pulse to the plurality of needle electrodes, wherein the electrical pulse allow the nucleic acid expression construct to traverse a muscle cell membrane. Additionally, the nucleic acid expression construct may also comprise a transfection-facilitating polypeptide or a charged polypeptide (e.g. poly-L-glutamate).

Also described herein is a method for vaccinating a subject. This method comprises: delivering into a tissue of the subject a nucleic acid expression construct that encodes a growth-hormone-releasing-hormone ("GHRH"); and then providing a vaccine to the subject in an amount effective to induce anti-vaccine antibodies in the subject. The preferred GHRH expression constructs are described above. The vaccine can be provided either concomitantly or after a period of time following delivery of the GHRH nucleic acid expression construct to the subject. In preferred embodiments, the vaccine is delivered up to a year after delivery of the GHRH nucleic acid expression construct. This invention encompasses vaccines that comprise: killed microorganisms; live attenuated organisms; subunit antigens; toxoid antigens; conjugate antigens or other type of vaccine that when introduced into a subjects body produces immunity to a specific disease by causing the activation of the immune system, antibody formation, and/or creating of a T-cell and/or B-cell response. However preferred embodiments of the invention comprise the vaccinations described above. In more preferred embodiments, the vaccine is delivered about 7 days to about 14 days after delivery of the GHRH nucleic acid expression construct. In another preferred embodiment, the GHRH nucleic acid expression construct is delivered using tissue electroporation, with a transfection-facilitating polypeptide.

Also described herein is a method of improving the clinical outcome, after an infectious challenge, of a vaccinated subject having arthritis. The method comprises: penetrating a muscle tissue in the subject with a plurality of needle electrodes, wherein the plurality of needle electrodes are arranged in a spaced relationship; delivering into the muscle tissue of the subject a nucleic acid expression construct that encodes a growth-hormone-releasing-hormone ("GHRH"), such that an amount of expressed GHRH is effective to enhance the vaccination response; and applying an electrical pulse to the plurality of needle electrodes, wherein the electrical pulse allows the nucleic acid expression construct to traverse a muscle cell membrane. The preferred range of 0.01-5 mg of nucleic acid expression construct having a defined concentration of poly-L-glutamate polypeptide is delivered into the muscle tissue of the subject, and the nucleic acid expression construct comprises a sequence that encodes a polypeptide having an amino acid sequence that is at least 90% identical to the encoded GHRH of SEQID#14. The preferred nucleic acid expression constructs comprise sequence that is at least 97% identical to mouse pAV0202 (SEQID#23); rat pAV0203 (SEQID#24); HV-GHRH pAV0224 (SEQID#25); pig-wt-GHRH pAV0225 (SEQID#26); dog pAV0235 (SEQID#27); bovine pAV0236 (SEQID#28); cat pAV0238 (SEQID#29); TI-GHRH pAV0239 (SEQID#30); ovine pAV0240 (SEQID#31); chicken pAV0241 (SEQID#32); horse pAV0249 (SEQID#33) or human pAV0226 (SEQID#34). The preferred vaccines comprise: killed microorganisms; live attenuated organisms; subunit antigens; toxoid antigens; conjugate antigens or other type of vaccine that when introduced into a subjects body produces immunity to a specific disease by causing the activation of the immune system, antibody formation, and/or creating of a T-cell and/or B-cell response.

A fourth aspect of the current invention comprises a composition having both a nucleic acid expression construct that encodes a growth-hormone-releasing-hormone ("GHRH") and a vaccine. The preferred nucleic acid expression constructs comprise sequence that is at least 97% identical to mouse pAV0202 (SEQID#23); rat pAV0203 (SEQID#24); HV-GHRH pAV0224 (SEQID#25); pig-wt-GHRH pAV0225 (SEQID#26); dog pAV0235 (SEQID#27); bovine pAV0236 (SEQID#28); cat pAV0238 (SEQID#29); TI-GHRH pAV0239 (SEQID#30); ovine pAV0240 (SEQID#31); chicken pAV0241 (SEQID#32); horse pAV0249 (SEQID#33) or human pAV0226 (SEQID#34). The preferred vaccines comprise: killed microorganisms; live attenuated organisms; subunit antigens; toxoid antigens; conjugate antigens or other type of vaccine that when introduced into a subjects body produces immunity to a specific disease by causing the activation of the immune system, antibody formation, and/or creating of a T-cell and/or B-cell response.

### BRIEF DESCRIPTION OF THE DRAWINGS:

**Figure 1** shows plasma levels of a secreted alkaline phosphatase ("SEAP") protein following injection of a pig with different concentrations of a SEAP expressing plasmid;

**Figure 2** shows glucose and insulin levels in control and pSP-HV-GHRH treated animals;

**Figure 3** Panel A and Panel B show the percentage of CD2⁺ cells and CD4⁺CD45R⁺ naïve T cells at day 0 and 18 after treatment, Panel C shows the ratio of CD45R⁺/CD45R⁻ naïve T cells at 300 days post-treatment, wherein the values are presented as means ± SEM, * *P* < 0.001;

**Figure 4** shows that the CD4⁺/CD8⁺ ratio is significantly increased 14 days after vaccination with a Surround-9 way vaccine (Biocor) in cows that received the GHRH plasmid, when compared to control animals, that had a decrease in the CD4⁺/CD8⁺ ratio during the same period of time, *P* < 0.05;

**Figure 5** shows that the relative proportion of naïve T-cells is increased at 14 days after the Surround 9-way (Biocor) vaccination in animals that received the GHRH plasmid when compared to control animals, *P* < 0.05;

**Figure 6** shows the body condition scores in heifers treated with pSP-HV-GHRH versus controls at 60 to 80 DIM. Body condition scores differed between treatment groups, *P* < 0.0001;

**Figure 7** shows the weight of the animals treated with the pSP-HV-GHRH expressing plasmid compared with controls at different time points after injection;

**Figure 8** shows a restriction map of pAV0224 expression plasmid;

**Figure 9** shows a restriction map of pAV0225 expression plasmid;

**Figure 10** shows a restriction map of pAV0235 expression plasmid;

**Figure 11** shows a restriction map of pAV0236 expression plasmid;

**Figure 12** shows a restriction map of pAV0238 expression plasmid;

**Figure 13** shows a restriction map of pAV0239 expression plasmid;

**Figure 14** shows a restriction map of pAV0240 expression plasmid;

**Figure 15** shows a restriction map of pAV0241expression plasmid;

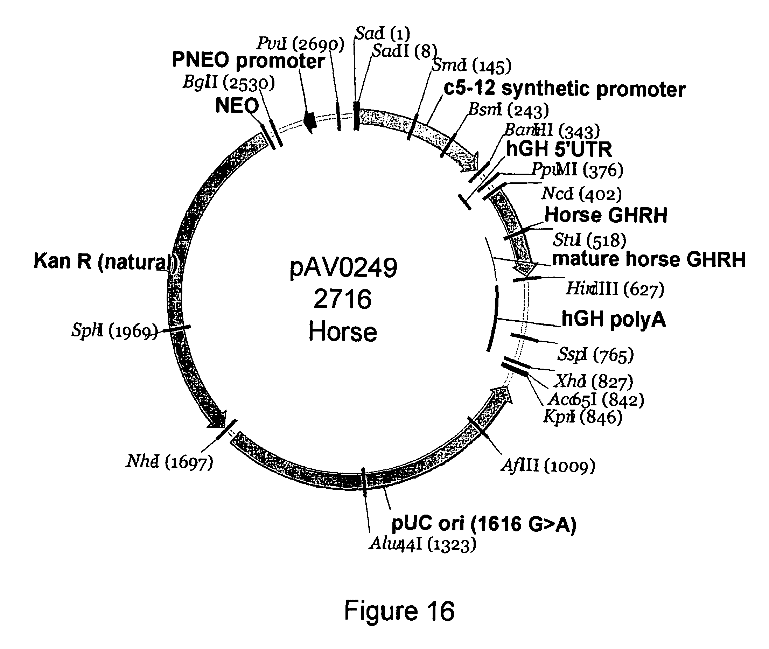

**Figure 16** shows a restriction map of pAV0249 expression plasmid; and

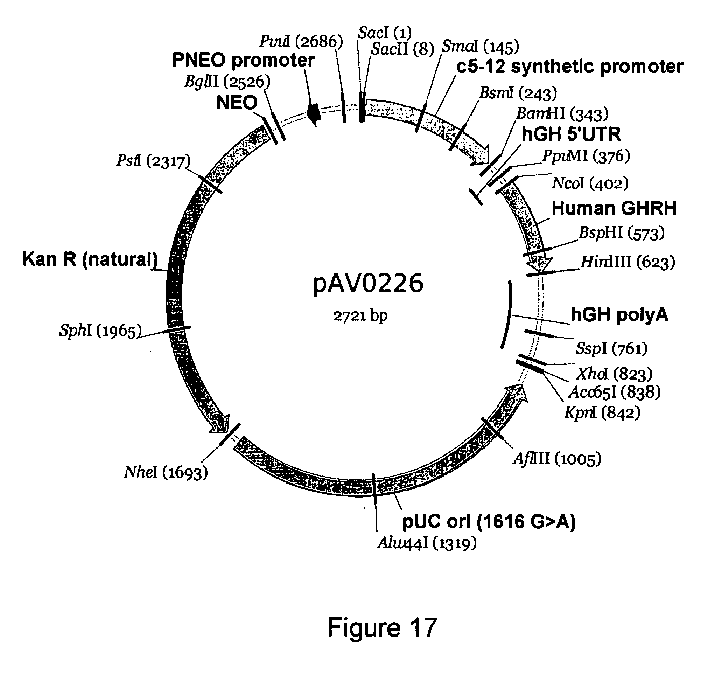

**Figure 17** shows a restriction map of pAV0226 expression plasmid.

The term "a" or "an" as used herein in the specification may mean one or more. As used herein in the claim(s), when used in conjunction with the word "comprising", the words "a" or "an" may mean one or more than one. As used herein "another" may mean at least a second or more.

The term "analog" as used herein includes any mutant of GHRH, or synthetic or naturally occurring peptide fragments of GHRH, such as HV-GHRH (SEQID#1), pig-GHRH (SEQID#2), bovine-GHRH (SEQID#3), dog-GHRH (SEQID#4), cat-GHRH (SEQID#5), TI-GHRH (SEQID#6), ovine-GHRH (SEQID#7), chicken-GHRH (SEQID#8), horse-GHRH (SEQID#9), TV-GHRH (SEQID#11), 15/27/28-GHRH (SEQID#12), human GHRH (1-44)NH₂ (SEQID#13), human GHRH(1-40)OH (SEQID#10) forms, or any shorter form to no less than (1-29) amino acids.

The term "bodily fat proportion" as used herein is defined as the body fat mass divided by the total body weight.

The term "body condition score" (BCS) as used herein is defined as a method to evaluate the overall nutrition and management of horses or any other farm animal.

The term "cassette" as used herein is defined as one or more transgene expression vectors.

The term "cell-transfecting pulse" as used herein is defined as a transmission of a force which results in transfection of a vector, such as a linear DNA fragment, into a cell. In some embodiments, the force is from electricity, as in electroporation, or the force is from vascular pressure.

The term "coding region" as used herein refers to any portion of the DNA sequence that is transcribed into messenger RNA (mRNA) and then translated into a sequence of amino acids characteristic of a specific polypeptide.

The term "delivery" or "delivering" as used herein is defined as a means of introducing a material into a tissue, a subject, a cell or any recipient, by means of chemical or biological process, injection, mixing, electroporation, sonoporation, or combination thereof, either under or without pressure.

The term "chronically ill" as used herein is defined as patients with conditions as chronic obstructive pulmonary disease, chronic heart failure, stroke, dementia, rehabilitation after hip fracture, chronic renal failure, arthritis, rheumatoid arthritis, and multiple disorders in the elderly, with doctor visits and/or hospitalization once a month for at least two years.

The term "donor-subject" as used herein refers to any species of the animal kingdom wherein cells have been removed and maintained in a viable state for any period of time outside the subject.

The term "donor-cells" as used herein refers to any cells that have been removed and maintained in a viable state for any period of time outside the donor-subject.

The term "electroporation" as used herein refers to a method that utilized electric pulses to deliver a nucleic acid sequence into cells.

The terms "electrical pulse" and "electroporation" as used herein refer to the administration of an electrical current to a tissue or cell for the purpose of taking up a nucleic acid molecule into a cell. A skilled artisan recognizes that these terms are associated with the terms "pulsed electric field" "pulsed current device" and "pulse voltage device." A skilled artisan recognizes that the amount and duration of the electrical pulse is dependent on the tissue, size, and overall health of the recipient subject, and furthermore knows how to determine such parameters empirically.

The term "encoded GHRH" as used herein is a biologically active polypeptide of growth hormone releasing hormone.

The term "enhanced vaccination response" as used herein comprises the enhanced immunity to a specific disease by allowing a faster activation of the immune system, faster antibody formation, higher antibody titers, enhancement of a T-cell response and/or enhancement of a B-cell response.

The term "functional biological equivalent" of GHRH as used herein is a polypeptide that has a distinct amino acid sequence from a wild type GHRH polypeptide while simultaneously having similar or improved biological activity when compared to the GHRH polypeptide. The functional biological equivalent may be naturally occurring or it may be modified by an individual. A skilled artisan recognizes that the similar or improved biological activity as used herein refers to facilitating and/or releasing growth hormone or other pituitary hormones. A skilled artisan recognizes that in some embodiments the encoded functional biological equivalent of GHRH is a polypeptide that has been engineered to contain a distinct amino acid sequence while simultaneously having similar or improved biological activity when compared to the GHRH polypeptide. Methods known in the art to engineer such a sequence include site-directed mutagenesis.

The term "growth hormone" ("GH") as used herein is defined as a hormone that relates to growth and acts as a chemical messenger to exert its action on a target cell. In a specific embodiment, the growth hormone is released by the action of growth hormone releasing hormone.

The term "growth hormone releasing hormone" ("GHRH") as used herein is defined as a hormone that facilitates or stimulates release of growth hormone, and in a much lesser extent other pituitary hormones, such as prolactin.

The term "heterologous nucleic acid sequence" as used herein is defined as a DNA sequence comprising differing regulatory and expression elements.

The term "immunotherapy" as used herein refers to any treatment that promotes or enhances the body's immune system to build protective antibodies that will reduce the symptoms of a medical condition, prevent the development in a subject of an infectious condition and/or lessen the need for medications.

The term "modified cells" as used herein is defined as the cells from a subject that have an additional nucleic acid sequence introduced into the cell.

The term "modified-donor-cells" as used herein refers to any donor-cells that have had a GHRH-encoding nucleic acid sequence delivered.

The term "molecular switch" as used herein refers to a molecule that is delivered into a subject that can regulate transcription of a gene.

The term "nucleic acid expression construct" as used herein refers to any type of an isolated genetic construct comprising a nucleic acid coding for a RNA capable of being transcribed. The term "expression vector" can also be used interchangeably herein. In specific embodiments, the isolated nucleic acid expression construct comprises: a promoter; a nucleotide sequence of interest; and a 3' untranslated region; wherein the promoter, the nucleotide sequence of interest, and the 3' untranslated region are operatively linked; and *in vivo* expression of the nucleotide sequence of interest is regulated by the promoter. The term "DNA fragment" as used herein refers to a substantially double stranded DNA molecule. Although the fragment may be generated by any standard molecular biology means known in the art, in some embodiments the DNA fragment or expression construct is generated by restriction digestion of a parent DNA molecule. The terms "expression vector," "expression cassette," or "expression plasmid" can also be used interchangeably. Although the parent molecule may be any standard molecular biology DNA reagent, in some embodiments the parent DNA molecule is a plasmid.

The term "operatively linked" as used herein refers to elements or structures in a nucleic acid sequence that are linked by operative ability and not physical location. The elements or structures are capable of, or characterized by accomplishing a desired operation. It is recognized by one of ordinary skill in the art that it is not necessary for elements or structures in a nucleic acid sequence to be in a tandem or adjacent order to be operatively linked.

The term "poly-L-glutamate ("PLG")" as used herein refers to a biodegradable polymer of L-glutamic acid that is suitable for use as a vector or adjuvant for DNA transfer into cells with or without electroporation.

The term "post-injection" as used herein refers to a time period following the introduction of a nucleic acid cassette that contains heterologous nucleic acid sequence encoding GHRH or a biological equivalent thereof into the cells of the subject and allowing expression of the encoded gene to occur while the modified cells are within the living organism.

The term "plasmid" as used herein refers generally to a construction comprised of extra-chromosomal genetic material, usually of a circular duplex of DNA that can replicate independently of chromosomal DNA. Plasmids, or fragments thereof, may be used as vectors. Plasmids are double-stranded DNA molecule that occur or are derived from bacteria and (rarely) other microorganisms. However, mitochondrial and chloroplast DNA, yeast killer and other cases are commonly excluded.

The term "plasmid mediated gene supplementation" as used herein refers a method to allow a subject to have prolonged exposure to a therapeutic range of a therapeutic protein by utilizing a nucleic acid-expression construct *in vivo*.

The term "pulse voltage device," or "pulse voltage injection device" as used herein relates to an apparatus that is capable of causing or causes uptake of nucleic acid molecules into the cells of an organism by emitting a localized pulse of electricity to the cells. The cell membrane then destabilizes, forming passageways or pores. Conventional devices of this type are calibrated to allow one to select or adjust the desired voltage amplitude and the duration of the pulsed voltage. The primary importance of a pulse voltage device is the capability of the device to facilitate delivery of compositions of the invention, particularly linear DNA fragments, into the cells of the organism.

The term "plasmid backbone" as used herein refers to a sequence of DNA that typically contains a bacterial origin of replication, and a bacterial antibiotic selection gene, which are necessary for the specific growth of only the bacteria that are transformed with the proper plasmid. However, there are plasmids, called mini-circles, that lack both the antibiotic resistance gene and the origin of replication (Darquet et al., 1997; Darquet et al., 1999; Soubrier et al., 1999). The use of *in vitro* amplified expression plasmid DNA (i.e. non-viral expression systems) avoids the risks associated with viral vectors. The non-viral expression systems products generally have low toxicity due to the use of "species-specific" components for gene delivery, which minimizes the risks of immunogenicity generally associated with viral vectors. One aspect of the current invention is that the plasmid backbone does not contain viral nucleotide sequences.

The term "promoter" as used herein refers to a sequence of DNA that directs the transcription of a gene. A promoter may direct the transcription of a prokaryotic or eukaryotic gene. A promoter may be "inducible", initiating transcription in response to an inducing agent or, in contrast, a promoter may be "constitutive", whereby an inducing agent does not regulate the rate of transcription. A promoter may be regulated in a tissue-specific or tissue-preferred manner, such that it is only active in transcribing the operable linked coding region in a specific tissue type or types.

The term "quality of life" or "health related quality of life" of a subject as used herein refers to those attributes valued by patients and their owners, including: their resultant comfort and well-being; the extent to which they are able to maintain reasonable physical, emotional, and intellectual function; and the degree to which they retain their ability to participate in valued activities within the family, in the workplace, and in the community.

The term "welfare" of a subject as used herein refers at a state of being or doing well, performing tasks and activities at functional levels; condition of health, happiness, and comfort; well-being; prosperity.

The term "replication element" as used herein comprises nucleic acid sequences that will lead to replication of a plasmid in a specified host. One skilled in the art of molecular biology will recognize that the replication element may include, but is not limited to a selectable marker gene promoter, a ribosomal binding site, a selectable marker gene sequence, and a origin of replication.

The term "residual linear plasmid backbone" as used herein comprises any fragment of the plasmid backbone that is left at the end of the process making the nucleic acid expression plasmid linear.

The term "recipient-subject" as used herein refers to any species of the animal kingdom wherein modified-donor-cells can be introduced from a donor-subject.

The term "regulator protein" as used herein refers to any protein that can be used to control the expression of a gene, and that is increasing the rate of transcription in response to an inducing agent.

The term "secretagogue" as used herein refers to an agent that stimulates secretion. For example, a growth hormone secretagogue is any molecule that stimulates the release of growth hormone from the pituitary when delivered into an animal. Growth hormone releasing hormone is a growth hormone secretagogue.

The terms "subject" or "animal" as used herein refers to any species of the animal kingdom. In preferred embodiments, it refers more specifically to humans and domesticated animals used for: pets (*e.g*. cats, dogs, *etc*.); work (*e.g*. horses, *etc*.); food (cows, chicken, fish, lambs, pigs, etc); and all others known in the art.

The term "tissue" as used herein refers to a collection of similar cells and the intercellular substances surrounding them. A skilled artisan recognizes that a tissue is an aggregation of similarly specialized cells for the performance of a particular function. For the scope of the present invention, the term tissue does not refer to a cell line, a suspension of cells, or a culture of cells. In a specific embodiment, the tissue is electroporated *in vivo.* In another embodiment, the tissue is not a plant tissue. A skilled artisan recognizes that there are four basic tissues in the body: 1) epithelium; 2) connective tissues, including blood, bone, and cartilage; 3) muscle tissue; and 4) nerve tissue. In a specific embodiment, the methods and compositions are directed to transfer of linear DNA into a muscle tissue by electroporation.

The term "therapeutic element" as used herein comprises nucleic acid sequences that will lead to an *in vivo* expression of an encoded gene product. One skilled in the art of molecular biology will recognize that the therapeutic element may include, but is not limited to a promoter sequence, a transgene, a poly A sequence, or a 3' or 5' UTR.

The term "transfects" as used herein refers to introduction of a nucleic acid into a eukaryotic cell. In some embodiments, the cell is not a plant tissue or a yeast cell.

The term "vector" as used herein refers to any vehicle that delivers a nucleic acid into a cell or organism. Examples include plasmid vectors, viral vectors, liposomes, or cationic lipids. The term also refers to a construction comprised of genetic material designed to direct transformation of a targeted cell by delivering a nucleic acid sequence into that cell. A vector may contain multiple genetic elements positionally and sequentially oriented with other necessary elements such that an included nucleic acid cassette can be transcribed and when necessary translated in the transfected cells. These elements are operatively linked. The term "expression vector" refers to a DNA plasmid that contains all of the information necessary to produce a recombinant protein in a heterologous cell.

The term "viral backbone" as used herein refers to a nucleic acid sequence that does not contain a promoter, a gene, and a 3' poly A signal or an untranslated region, but contain elements including, but not limited at site-specific genomic integration Rep and inverted terminal repeats ("ITRs") or the binding site for the tRNA primer for reverse transcription, or a nucleic acid sequence component that induces a viral immunogenicity response when inserted in vivo, allows integration, affects specificity and activity of tissue specific promoters, causes transcriptional silencing or poses safety risks to the subject.

The term "vascular pressure pulse" refers to a pulse of pressure from a large volume of liquid to facilitate uptake of a vector into a cell. A skilled artisan recognizes that the amount and duration of the vascular pressure pulse is dependent on the tissue, size, and overall health of the recipient animal, and furthermore knows how to determine such parameters empirically.

The term "vaccine" as used herein refers to any preparation of killed microorganisms, live attenuated organisms, subunit antigens, toxoid antigens, conjugate antigens or other type of antigenic molecule that when introduced into a subjects body produces immunity to a specific disease by causing the activation of the immune system, antibody formation, and/or creating of a T-cell and/or B-cell response. Generally vaccines against microorganisms are directed toward at least part of a virus, bacteria, parasite, mycoplasma, or other infectious agent.

Efficacy of vaccination or immunization for specific pathogens and the clinical outcome after an infectious challenge are of extraordinary importance for both human and animal medicine. One specific embodiment of the current invention is a method of enhancing the response to vaccination. The method comprises: penetrating a muscle tissue in the subject with a plurality of needle electrodes, wherein the plurality of needle electrodes are arranged in a spaced relationship; delivering into the muscle tissue of the subject a nucleic acid expression construct that encodes a growth-hormone-releasing-hormone ("GHRH"), such that an amount of expressed GHRH is effective to enhance the response to a specific vaccination; and applying an electrical pulse to the plurality of needle electrodes, wherein the electrical pulse allows the nucleic acid expression construct to traverse a muscle cell membrane. A range of 0.01-5 mg of nucleic acid expression construct with a defined concentration of poly-L-glutamate polypeptide is delivered into the muscle tissue of the subject, and the nucleic acid expression construct comprises a sequence that encodes a polypeptide having an amino acid sequence that is at least 90% identical to the encoded GHRH of SEQID#14. The preferred subject comprises a human, a ruminant animal, a food animal, a horse, or a work animal. While there are many indicators of enhanced response to vaccination, a few examples comprise: increased specific antibody titer, more rapid response after an infectious challenge, an improved clinical outcome, or a combination thereof. Other specific embodiments of this invention encompass various modes of delivering into the tissue of the subject the nucleic acid expression construct (e.g. an electroporation method, a viral vector, in conjunction with a carrier, by parenteral route, or a combination thereof).

A second preferred embodiment includes the nucleic acid expression construct being delivered in a single dose, and the single dose comprising a total of about a 0.01-5 mg of nucleic acid expression construct. Generally the nucleic acid expression construct is delivered into a tissue of the subject comprising diploid cells (e.g. muscle cells).

In a third specific embodiment the nucleic acid expression construct used for transfection comprises a wt porcine-GHRH plasmid (SEQID#26). Other specific embodiments utilize other nucleic acid expression constructs (e.g. an optimized bovine GHRH plasmid, pAV0236 (SEQID#28); a TI-GHRH plasmid, pAV0239 (SEQID#30); HV-GHRH plasmid, pAV0224 (SEQID#25); ovine GHRH plasmid, pAV0240 (SEQID#31); chicken GHRH plasmid, pAV0241 (SEQID#32); dog GHRH plasmid, pAV0235 (SEQID#27); cat GHRH plasmid, pAV0238 (SEQID#29); horse GHRH plasmid, pAV0249 (SEQID#33), human GHRH plasmid, pAV0226 (SEQID#34).

In a fourth specific embodiment, the nucleic acid expression construct further comprises, a transfection-facilitating polypeptide (e.g. a charged polypeptide, or poly-L-glutamate). After delivering the nucleic acid expression construct into the tissues of the subject, expression of the encoded GHRH or functional biological equivalent thereof is initiated. The encoded GHRH comprises a biologically active polypeptide; and the encoded functional biological equivalent of GHRH is a polypeptide that has been engineered to contain a distinct amino acid sequence while simultaneously having similar or improved biologically activity when compared to the GHRH polypeptide. One embodiment of a specific encoded GHRH or functional biological equivalent thereof is of formula (SEQID#14). The animal comprises a human, a food animal, a work animal (e.g. a pig, cow, sheep, goat or chicken), or a pet (e.g. dog, cat, horse).

Also described herein are methods useful for improving the clinical outcome of a patient after an infectious challenge. The general method comprises treating a subject with plasmid-mediated gene supplementation. The method comprises delivering a nucleic acid expression construct that encodes a growth-hormone-releasing-hormone ("GHRH") or functional biological equivalent thereof into a tissue, such as a muscle, of the subject. Specific embodiments of this invention are directed toward improving the vaccination response in treated subjects by plasmid mediated GHRH supplementation. Plasmid injection can precede or be concomitant with the specific vaccination. The subsequent *in vivo* expression of the GHRH or biological equivalent in the subject is sufficient to improve vaccination response. Thus, if an infectious challenge occurs at a later date, the clinical outcome is significantly improved. It is also possible to enhance this method by placing a plurality of electrodes in a selected tissue, then delivering nucleic acid expression construct to the selected tissue in an area that interposes the plurality of electrodes, and applying a cell-transfecting pulse (e.g. electrical) to the selected tissue in an area of the selected tissue where the nucleic acid expression construct was delivered. However, the cell-transfecting pulse need not be an electrical pulse, a different method, such as vascular pressure pulse can also be utilized. Electroporation, direct injection, gene gun, or gold particle bombardment are also used in specific embodiments to deliver the nucleic acid expression construct encoding the GHRH or biological equivalent into the subject. The subject in this invention comprises an animal (e.g. a human, a pig, a horse, a cow, a mouse, a rat, a monkey, a sheep, a goat, a dog, or a cat).

Recombinant GH replacement therapy is widely used in agriculture and clinically, with beneficial effects, but generally, the doses are supra-physiological. Such elevated doses of recombinant GH are associated with deleterious side-effects, for example, up to 30% of the recombinant GH treated subjects develop at a higher frequency insulin resistance (Gopinath and Etherton, 1989a; Gopinath and Etherton, 1989b; Verhelst et al., 1997) or accelerated bone epiphysis growth and closure in pediatric patients (Blethen and Rundle, 1996). In addition, molecular heterogeneity of circulating GH may have important implications in growth and homeostasis (Satozawa et al., 2000; Tsunekawa et al., 1999; Wada et al., 1998). Unwanted side effects result from the fact that treatment with recombinant exogenous GH protein raises basal levels of GH and abolishes the natural episodic pulses of GH. In contradistinction, no side effects have been reported for recombinant GHRH therapies. The normal levels of GHRH in the pituitary portal circulation range from about 150-to-800 pg/ml, while systemic circulating values of the hormone are up to about 100-500 pg/ml. Some patients with acromegaly caused by extracranial tumors have level that is nearly 100 times as high (e.g. 50 ng/ml of immunoreactive GHRH) (Thorner et al., 1984). Long-term studies using recombinant GHRH therapies (1-5 years) in children and elderly humans have shown an absence of the classical GH side-effects, such as changes in fasting glucose concentration or, in pediatric patients, the accelerated bone epiphysal growth and closure or slipping of the capital femoral epiphysis (Chevalier et al., 2000) (Duck et al., 1992; Vittone et al., 1997).

Studies in humans, sheep or pigs showed that continuous infusion with recombinant GHRH protein restores the normal GH pattern without desensitizing GHRH receptors or depleting GH supplies (Dubreuil et al., 1990). As this system is capable of a degree of feed-back which is abolished in the GH therapies, GHRH recombinant protein therapy may be more physiological than GH therapy. However, due to the short half-life of GHRH *in vivo,* frequent (one to three times per day) intravenous, subcutaneous or intranasal (requiring 300-fold higher dose) administrations are necessary (Evans et al., 1985; Thomer et al., 1986b). Thus, as a chronic therapy, recombinant GHRH protein administration is not practical. A plasmid-mediated supplementation approach, however could overcome this limitations to GHRH use. The choice of GHRH for a gene therapeutic application is favored by the fact that the gene, cDNA and native and several mutated molecules have been characterized for humans, pig, cattle and other species (Bohlen et al., 1983; Guillemin et al., 1982); the cDNA of cat, dog and horse specific GHRH have been isolated. The measurement of therapeutic efficacy is straightforward and unequivocal.

Among the non-viral techniques for gene transfer *in vivo,* the direct injection of plasmid DNA into muscle is simple, inexpensive, and safe. The inefficient DNA uptake into muscle fibers after simple direct injection had led to relatively low expression levels (Prentice et al., 1994; Wells et al., 1997) In addition, the duration of the transgene expression has been short (Wolff et al., 1990). The most successful previous clinical applications have been confined to vaccines (Danko and Wolff, 1994; Tsurumi et al., 1996). Recently, significant progress to enhance plasmid delivery *in vivo* and subsequently to achieve physiological levels of a secreted protein was obtained using the electroporation technique. Electroporation has been used very successfully to transfect tumor cells after injection of plasmid (Lucas et al., 2002; Matsubara et al., 2001) or to deliver the anti-tumor drug bleomycin to cutaneous and subcutaneous tumors in humans (Gehl et al., 1998; Heller et al., 1996). Electroporation also has been extensively used in mice (Lesbordes et al., 2002; Lucas et al., 2001; Vilquin et al., 2001), rats (Terada et al., 2001; Yasui et al., 2001), and dogs (Fewell et al., 2001) to deliver therapeutic genes that encode for a variety of hormones, cytokines or enzymes. Our previous studies using growth hormone releasing hormone (GHRH) showed that plasmid therapy with electroporation is scalable and represents a promising approach to induce production and regulated secretion of proteins in large animals and humans (Draghia-Akli et al., 1999; Draghia-Akli et al., 2002c). Electroporation also has been extensively used in rodents and other small animals (Bettan et al., 2000; Yin and Tang, 2001). It has been observed that the electrode configuration affects the electric field distribution, and subsequent results (Gehl et al., 1999; Miklavcic et al., 1998). Preliminary experiments indicated that for a large animal or humans, needle electrodes give consistently better reproducible results than external caliper electrodes.

The ability of electroporation to enhance plasmid uptake into the skeletal muscle has been well documented, as described above. In addition, plasmid formulated with PLG or polyvinylpyrrolidone ("PVP") has been observed to increase gene transfection and consequently gene expression to up to 10 fold in the skeletal muscle of mice, rats and dogs (Fewell et al., 2001; Mumper et al., 1998). Although not wanting to be bound by theory, PLG will increase the transfection of the plasmid during the electroporation process, not only by stabilizing the plasmid DNA, and facilitating the intracellular transport through the membrane pores, but also through an active mechanism. For example, positively charged surface proteins on the cells could complex the negatively charged PLG linked to plasmid DNA through protein-protein interactions. When an electric field is applied, the surface proteins reverse direction and actively internalize the DNA molecules, process that substantially increases the transfection efficiency.

Although not wanting to be bound by theory, the plasmid supplementation approach to enhance the vaccination response and to improve the clinical outcome of a subject after an infectious challenge described herein offers advantages over the limitations of directly injecting recombinant GH or GHRH protein. Expression of GHRH or novel biological equivalents of GHRH can be directed by an expression plasmid controlled by a synthetic muscle-specific promoter. Expression of such GHRH or biological equivalent thereof elicited high GH and IGF-I levels in subjects that have had the encoding sequences delivered into the cells of the subject by intramuscular injection and *in vivo* electroporation. Although *in vivo* electroporation is the preferred method of introducing the heterologous nucleic acid encoding system into the cells of the subject, other methods exist and should be known by a person skilled in the art (*e*.*g*. electroporation, lipofectamine, calcium phosphate, *ex vivo* transformation, direct injection, DEAE dextran, sonication loading, receptor mediated transfection, microprojectile bombardment, etc.). For example, it may also be possible to introduce the nucleic acid sequence that encodes the GHRH or functional biological equivalent thereof directly into the cells of the subject by first removing the cells from the body of the subject or donor, maintaining the cells in culture, then introducing the nucleic acid encoding system by a variety of methods (*e.g*. electroporation, lipofectamine, calcium phosphate, *ex vivo* transformation, direct injection, DEAE dextran, sonication loading, receptor mediated transfection, microprojectile bombardment, etc.), and finally reintroducing the modified cells into the original subject or other host subject (the *ex vivo* method). The GHRH sequence can be cloned into an adenovirus vector or an adeno-associated vector and delivered by simple intramuscular injection, or intravenously or intra-arterially. Plasmid DNA carrying the GHRH sequence can be complexed with cationic lipids or liposomes and delivered intramuscularly, intravenously or subcutaneous.

Administration as used herein refers to the route of introduction of a vector or carrier of DNA into the body. Administration can be directly to a target tissue or by targeted delivery to the target tissue after systemic administration. In particular, the present invention can be used for improving the vaccination response in a subject by administration of the vector to the body in order to establishing controlled expression of any specific nucleic acid sequence within tissues at certain levels that are useful for plasmid-mediated supplementation. The preferred means for administration of vector and use of formulations for delivery are described above.

Muscle cells have the unique ability to take up DNA from the extracellular space after simple injection of DNA particles as a solution, suspension, or colloid into the muscle. Expression of DNA by this method can be sustained for several months. DNA uptake in muscle cells is further enhanced utilizing *in vivo* electroporation.

Delivery of formulated DNA vectors involves incorporating DNA into macromolecular complexes that undergo endocytosis by the target cell. Such complexes may include lipids, proteins, carbohydrates, synthetic organic compounds, or inorganic compounds. The characteristics of the complex formed with the vector (size, charge, surface characteristics, composition) determine the bioavailability of the vector within the body. Other elements of the formulation function as ligands that interact with specific receptors on the surface or interior of the cell. Other elements of the formulation function to enhance entry into the cell, release from the endosome, and entry into the nucleus.

Delivery can also be through use of DNA transporters. DNA transporters refer to molecules that bind to DNA vectors and are capable of being taken up by epidermal cells. DNA transporters contain a molecular complex capable of non-covalently binding to DNA and efficiently transporting the DNA through the cell membrane. It is preferable that the transporter also transport the DNA through the nuclear membrane. See, *e.g*., the following applications:
(1) Woo et al., U.S. Patent No. 6,150,168 entitled: "A DNA Transporter System and Method of Use;" (2) Woo et al., PCT/US93/02725, entitled "A DNA Transporter System and method of Use", filed Mar. 19, 1993; (3) Woo et al., U.S. Patent No. 6,177,554 "Nucleic Acid Transporter Systems and Methods of Use;" (4) Szoka et al., U.S. Patent No. 5,955,365 entitled "Self-Assembling Polynucleotide Delivery System;" and (5) Szoka et al., PCT/US93/03406, entitled "Self-Assembling Polynucleotide Delivery System", filed Apr. 5, 1993.

Another method of delivery involves a DNA transporter system. The DNA transporter system consists of particles containing several elements that are independently and non-covalently bound to DNA. Each element consists of a ligand that recognizes specific receptors or other functional groups such as a protein complexed with a cationic group that binds to DNA. Examples of cations which may be used are spermine, spermine derivatives, histone, cationic peptides and/or polylysine; one element is capable of binding both to the DNA vector and to a cell surface receptor on the target cell. Examples of such elements are organic compounds which interact with the asialoglycoprotein receptor, the folate receptor, the mannose-6-phosphate receptor, or the carnitine receptor. A second element is capable of binding both to the DNA vector and to a receptor on the nuclear membrane. The nuclear ligand is capable of recognizing and transporting a transporter system through a nuclear membrane. An example of such ligand is the nuclear targeting sequence from SV40 large T antigen or histone. A third element is capable of binding to both the DNA vector and to elements which induce episomal lysis. Examples include inactivated virus particles such as adenovirus, peptides related to influenza virus hemagglutinin, or the GALA peptide described in the Skoka patent cited above.

Administration may also involve lipids. The lipids may form liposomes which are hollow spherical vesicles composed of lipids arranged in unilamellar, bilamellar, or multilamellar fashion and an internal aqueous space for entrapping water soluble compounds, such as DNA, ranging in size from 0.05 to several microns in diameter. Lipids may be useful without forming liposomes. Specific examples include the use of cationic lipids and complexes containing DOPE which interact with DNA and with the membrane of the target cell to facilitate entry of DNA into the cell.

Gene delivery can also be performed by transplanting genetically engineered cells. For example, immature muscle cells called myoblasts may be used to carry genes into the muscle fibers. Myoblast genetically engineered to express recombinant human growth hormone can secrete the growth hormone into the animal's blood. Secretion of the incorporated gene can be sustained over periods up to 3 months.

Myoblasts eventually differentiate and fuse to existing muscle tissue. Because the cell is incorporated into an existing structure, it is not just tolerated but nurtured. Myoblasts can easily be obtained by taking muscle tissue from an individual who needs plasmid-mediated supplementation and the genetically engineered cells can also be easily put back with out causing damage to the patient's muscle. Similarly, keratinocytes may be used to delivery genes to tissues. Large numbers of keratinocytes can be generated by cultivation of a small biopsy. The cultures can be prepared as stratified sheets and when grafted to humans, generate epidermis which continues to improve in histotypic quality over many years. The keratinocytes are genetically engineered while in culture by transfecting the keratinocytes with the appropriate vector. Although keratinocytes are separated from the circulation by the basement membrane dividing the epidermis from the dermis, human keratinocytes secrete into circulation the protein produced.

Delivery may also involve the use of viral vectors. For example, an adenoviral vector may be constructed by replacing the E1 and E3 regions of the virus genome with the vector elements described in this invention including promoter, 5'UTR, 3'UTR and nucleic acid cassette and introducing this recombinant genome into 293 cells which will package this gene into an infectious virus particle. Virus from this cell may then be used to infect tissue *ex vivo* or *in vivo* to introduce the vector into tissues leading to expression of the gene in the nucleic acid cassette.

### VECTORS

The term "vector" is used to refer to a carrier nucleic acid molecule into which a nucleic acid sequence can be inserted for introduction into a cell wherein, in some embodiments, it can be replicated. A nucleic acid sequence can be native to the animal, or it can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), linear DNA fragments, and artificial chromosomes (*e*.*g*., YACs, BACs), although in a preferred embodiment the vector contains substantially no viral sequences. One of skill in the art would be well equipped to construct a vector through standard recombinant techniques.

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of anti-sense molecules or ribozymes. Expression vectors can contain a variety of "control sequences," which refer to nucleic acid sequences necessary for the transcription and possibly translation of an operatively linked coding sequence in a particular host cell. In addition to control sequences that govern transcription and translation, vectors and expression vectors may contain nucleic acid sequences that serve other functions as well and are described *infra*.

### PLASMID VECTORS

In certain embodiments, a linear DNA fragment from a plasmid vector is contemplated for use to transfect a eukaryotic cell, particularly a mammalian cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, *E. coli* is often transformed using derivatives of pBR322 or pUC, a plasmid derived from an E. *coli* species. pBR322 contains genes for ampicillin and tetracycline resistance and thus provides easy means for identifying transformed cells. Other plasmids contain genes for kanamycin or neomycin, or have a non-antibiotic selection mechanism. The pBR plasmid, or other microbial plasmid or phage should also contain, or be modified to contain, for example, promoters which can be used by the microbial organism for expression of its own proteins. A skilled artisan recognizes that any plasmid in the art may be modified for use in the methods of the present invention. In a specific embodiment, for example, a GHRH vector used for the therapeutic applications is synthetically produced and has a kanamycin resistance gene.

In addition, phage vectors containing replicon and control sequences that are compatible with the host microorganism can be used as transforming vectors in connection with these hosts. For example, the phage lambda GEM^{™}-11 may be utilized in making a recombinant phage vector which can be used to transform host cells, such as, for example, *E. coli* LE392.

Further useful plasmid vectors include pIN vectors (Inouye et al., 1985); and pGEX vectors, for use in generating glutathione S-transferase soluble fusion proteins for later purification and separation or cleavage. Other suitable fusion proteins are those with β-galactosidase, ubiquitin, and the like.

Bacterial host cells, for example, *E. coli*, comprising the expression vector, are grown in any of a number of suitable media, for example, LB. The expression of the recombinant protein in certain vectors may be induced, as would be understood by those of skill in the art, by contacting a host cell with an agent specific for certain promoters, e.g., by adding IPTG to the media or by switching incubation to a higher temperature. After culturing the bacteria for a further period, generally of between 2 and 24 h, the cells are collected by centrifugation and washed to remove residual media.

### PROMOTERS AND ENHANCERS

A promoter is a control sequence that is a region of a nucleic acid sequence at which initiation and rate of transcription of a gene product are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control" and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i*.*e*., 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the timidine kinase (tk) promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant, synthetic or heterologous promoter, which refers to a promoter that is not normally associated with a nucleic acid sequence in its natural environment. A recombinant, synthetic or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.,* containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression. The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB, http://www.epd.isb-sib.ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

Tables 1 and 2 list non-limiting examples of elements/promoters that may be employed, in the context of the present invention, to regulate the expression of a RNA. Table 2 provides non-limiting examples of inducible elements, which are regions of a nucleic acid sequence that can be activated in response to a specific stimulus.

| **TABLE 1** | |
|---|---|
| **Promoter and/or Enhancer** | |
| **Promoter/Enhancer** | **Relevant References** |
| β-Actin | (Kawamoto et al., 1988; Kawamoto et al., 1989) |
| Muscle Creatine Kinase (MCK) | (Horlick and Benfield, 1989; Jaynes et al., 1988) |
| Metallothionein (MTII) | (Inouye et al., 1994; Narum et al., 2001; Skroch et al., 1993) |
| Albumin | (Pinkert et al., 1987; Tronche et al., 1989) |
| β-Globin | (Tronche et al., 1990; Trudel and Costantini, 1987) |
| Insulin | (German et al., 1995; Ohlsson et al., 1991) |
| Rat Growth Hormone | (Larsen et al., 1986) |
| Troponin I (TN I) | (Lin et al., 1991; Yutzey and Konieczny, 1992) |
| Platelet-Derived Growth Factor | (Pech et al., 1989) |
| Duchenne Muscular Dystrophy | (Klamut et al., 1990; Klamut et al., 1996) |
| Cytomegalovirus (CMV) | (Boshart et al., 1985; Dorsch-Hasler et al., 1985) |
| Synthetic muscle specific promoters | (Draghia-Akli et al., 1999; Draghia-Akli et al., 2002c; Li et al., 1999) |

| **TABLE 2** | |
|---|---|
| **Element/Inducer** | |
| **Element** | **Inducer** |
| MT II | Phorbol Ester (TFA) |
| | Heavy metals |
| MMTV (mouse mammary tumor virus) | Glucocorticoids |
| β-Interferon | Poly(rI)x / Poly(rc) |
| Adenovirus 5 E2 | E1A |
| Collagenase | Phorbol Ester (TPA) |
| Stromelysin | Phorbol Ester (TPA) |
| SV40 | Phorbol Ester (TPA) |
| Murine MX Gene | Interferon, Newcastle Disease Virus |
| GRP78 Gene | A23187 |
| α-2-Macroglobulin | IL-6 |
| Vimentin | Serum |
| MHC Class I Gene H-2κb | Interferon |
| HSP70 | E1A, SV40 Large T Antigen |
| Proliferin | Phorbol Ester-TPA |
| Tumor Necrosis Factor α | PMA |
| Thyroid Stimulating Hormone α Gene | Thyroid Hormone |

The identity of tissue-specific promoters or elements, as well as assays to characterize their activity, is well known to those of skill in the art. Non-limiting examples of such regions include the human LIMK2 gene (Nomoto et al., 1999), the somatostatin receptor 2 gene (Kraus et al., 1998), murine epididymal retinoic acid-binding gene (Lareyre et al., 1999), human CD4 (Zhao-Emonet et al., 1998), mouse alpha2 (XI) collagen (Liu et al., 2000; Tsumaki et al., 1998), D1A dopamine receptor gene (Lee et al., 1997), insulin-like growth factor II (Dai et al., 2001; Wu et al., 1997), and human platelet endothelial cell adhesion molecule-1 (Almendro et al., 1996).

In a preferred embodiment, a synthetic muscle promoter is utilized, such as SPc5-12 (Li et al., 1999), which contains a proximal serum response element ("SRE") from skeletal α-actin, multiple MEF-2 sites, MEF-1 sites, and TEF-1 binding sites, and greatly exceeds the transcriptional potencies of natural myogenic promoters. The uniqueness of such a synthetic promoter is a significant improvement over, for instance, issued patents concerning a myogenic promoter and its use (e.g. U.S. Pat. No. 5,374,544) or systems for myogenic expression of a nucleic acid sequence (e.g. U.S. Pat. No. 5,298,422). In a preferred embodiment, the promoter utilized in the invention does not get shut off or reduced in activity significantly by endogenous cellular machinery or factors. Other elements, including trans-acting factor binding sites and enhancers may be used in accordance with this embodiment of the invention. In an alternative embodiment, a natural myogenic promoter is utilized, and a skilled artisan is aware how to obtain such promoter sequences from databases including the National Center for Biotechnology Information ("NCBI") GenBank database or the NCBI PubMed site. A skilled artisan is aware that these databases may be utilized to obtain sequences or relevant literature related to the present invention.

### INITIATION SIGNALS AND INTERNAL RIBOSOME BINDING SITES

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon should be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites ("IRES") elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Samow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

### MULTIPLE CLONING SITES

Vectors can include a MCS, which is a nucleic acid region that contains multiple restriction enzyme sites, any of which can be used in conjunction with standard recombinant technology to digest the vector (see, for example, (Carbonelli et al., 1999; Cocea, 1997; Levenson et al., 1998)) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### SPLICING SITES

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, (Chandler et al., 1997).

### TERMINATION SIGNALS

The vectors or constructs of the present invention will generally comprise at least one termination signal. A "termination signal" or "terminator" is comprised of the DNA sequences involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 A residues ("polyA") to the 3' end of the transcript. RNA molecules modified with this polyA tail appear to more stable and are translated more efficiently. Thus, in other embodiments involving eukaryotes, it is preferred that that terminator comprises a signal for the cleavage of the RNA, and it is more preferred that the terminator signal promotes polyadenylation of the message. The terminator and/or polyadenylation site elements can serve to enhance message levels and to minimize read through from the cassette into other sequences.

Terminators contemplated for use in the invention include any known terminator of transcription described herein or known to one of ordinary skill in the art, including but not limited to, for example, the termination sequences of genes, such as for example the bovine growth hormone terminator or viral termination sequences, such as for example the SV40 terminator. In certain embodiments, the termination signal may be a lack of transcribable or translatable sequence, such as due to a sequence truncation.

### POLYADENYLATION SIGNALS

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal, skeletal alpha actin 3'UTR or the human or bovine growth hormone polyadenylation signal, convenient and known to function well in various target cells. Polyadenylation may increase the stability of the transcript or may facilitate cytoplasmic transport.

### ORIGINS OF REPLICATION

In order to propagate a vector in a host cell, it may contain one or more origins of replication sites (often termed "ori"), which is a specific nucleic acid sequence at which replication is initiated. Alternatively an autonomously replicating sequence ("ARS") can be employed if the host cell is yeast.

### SELECTABLE AND SCREENABLE MARKERS

In certain embodiments of the invention, cells containing a nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker, for instance kanamycin.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase ("tk") or chloramphenicol acetyltransferase ("CAT") may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### MUTAGENESIS

Where employed, mutagenesis was accomplished by a variety of standard, mutagenic procedures. Mutation is the process whereby changes occur in the quantity or structure of an organism. Mutation can involve modification of the nucleotide sequence of a single gene, blocks of genes or whole chromosome. Changes in single genes may be the consequence of point mutations which involve the removal, addition or substitution of a single nucleotide base within a DNA sequence, or they may be the consequence of changes involving the insertion or deletion of large numbers of nucleotides.

Mutations can arise spontaneously as a result of events such as errors in the fidelity of DNA replication or the movement of transposable genetic elements (transposons) within the genome. They also are induced following exposure to chemical or physical mutagens. Such mutation-inducing agents include ionizing radiations, ultraviolet light and a diverse array of chemical such as alkylating agents and polycyclic aromatic hydrocarbons all of which are capable of interacting either directly or indirectly (generally following some metabolic biotransformations) with nucleic acids. The DNA lesions induced by such environmental agents may lead to modifications of base sequence when the affected DNA is replicated or repaired and thus to a mutation. Mutation also can be site-directed through the use of particular targeting methods.

### SITE-DIRECTED MUTAGENESIS

Structure-guided site-specific mutagenesis represents a powerful tool for the dissection and engineering of protein-ligand interactions (Wells, 1996, Braisted *et al*., 1996). The technique provides for the preparation and testing of sequence variants by introducing one or more nucleotide sequence changes into a selected DNA.

Site-specific mutagenesis uses specific oligonucleotide sequences which encode the DNA sequence of the desired mutation, as well as a sufficient number of adjacent, unmodified nucleotides. In this way, a primer sequence is provided with sufficient size and complexity to form a stable duplex on both sides of the deletion junction being traversed. A primer of about 17 to 25 nucleotides in length is preferred, with about 5 to 10 residues on both sides of the junction of the sequence being altered.

The technique typically employs a bacteriophage vector that exists in both a single-stranded and double-stranded form. Vectors useful in site-directed mutagenesis include vectors such as the M13 phage. These phage vectors are commercially available and their use is generally well known to those skilled in the art. Double-stranded plasmids are also routinely employed in site-directed mutagenesis, which eliminates the step of transferring the gene of interest from a phage to a plasmid.

In general, one first obtains a single-stranded vector, or melts two strands of a double-stranded vector, which includes within its sequence a DNA sequence encoding the desired protein or genetic element. An oligonucleotide primer bearing the desired mutated sequence, synthetically prepared, is then annealed with the single-stranded DNA preparation, taking into account the degree of mismatch when selecting hybridization conditions. The hybridized product is subjected to DNA polymerizing enzymes such as *E. coli* polymerase I (Klenow fragment) in order to complete the synthesis of the mutation-bearing strand. Thus, a heteroduplex is formed, wherein one strand encodes the original non-mutated sequence, and the second strand bears the desired mutation. This heteroduplex vector is then used to transform appropriate host cells, such as *E. coli* cells, and clones are selected that include recombinant vectors bearing the mutated sequence arrangement.

Comprehensive information on the functional significance and information content of a given residue of protein can best be obtained by saturation mutagenesis in which all 19 amino acid substitutions are examined. The shortcoming of this approach is that the logistics of multi-residue saturation mutagenesis are daunting (Warren *et al*., 1996, Brown *et al*., 1996; Zeng *et al*., 1996; Burton and Barbas, 1994; Yelton *et al*., 1995; Jackson *et al*., 1995; Short *et al*., 1995; Wong *et al*., 1996; Hilton *et al*., 1996). Hundreds, and possibly even thousands, of site specific mutants should be studied. However, improved techniques make production and rapid screening of mutants much more straightforward. See also, U.S. Patents 5,798,208 and 5,830,650, for a description of "walk-through" mutagenesis. Other methods of site-directed mutagenesis are disclosed in U.S. Patents 5,220,007; 5,284,760; 5,354,670; 5,366,878; 5,389,514; 5,635,377; and 5,789,166.

### ELECTROPORATION

In certain embodiments of the present invention, a nucleic acid is introduced into an organelle, a cell, a tissue or an organism *via* electroporation. Electroporation involves the exposure of a suspension of cells and DNA to a high-voltage electric discharge. In some variants of this method, certain cell wall-degrading enzymes, such as pectin-degrading enzymes, are employed to render the target recipient cells more susceptible to transformation by electroporation than untreated cells (U.S. Patent No.5,384,253). Alternatively, recipient cells can be made more susceptible to transformation by mechanical wounding and other methods known in the art.

Transfection of eukaryotic cells using electroporation has been quite successful. Mouse pre-B lymphocytes have been transfected with human kappa-immunoglobulin genes (Potter et al., 1984), and rat hepatocytes have been transfected with the chloramphenicol acetyltransferase gene (Tur-Kaspa et al., 1986) in this manner.

The underlying phenomenon of electroporation is believed to be the same in all cases, but the exact mechanism responsible for the observed effects has not been elucidated. Although not wanting to be bound by theory, the overt manifestation of the electroporative effect is that cell membranes become transiently permeable to large molecules, after the cells have been exposed to electric pulses. There are conduits through cell walls, which under normal circumstances maintain a resting transmembrane potential of circa 90 mV by allowing bi-directional ionic migration.

Although not wanting to be bound by theory, electroporation makes use of the same structures, by forcing a high ionic flux through these structures and opening or enlarging the conduits. In prior art, metallic electrodes are placed in contact with tissues and predetermined voltages, proportional to the distance between the electrodes are imposed on them. The protocols used for electroporation are defined in terms of the resulting field intensities, according to the formula ***E*=*V*/*d*,** where ("***E***") is the field, ("***V***") is the imposed voltage and ("***d***") is the distance between the electrodes.

The electric field intensity *E* has been a very important value in prior art when formulating electroporation protocols for the delivery of a drug or macromolecule into the cell of the subject. Accordingly, it is possible to calculate any electric field intensity for a variety of protocols by applying a pulse of predetermined voltage that is proportional to the distance between electrodes. However, a caveat is that an electric field can be generated in a tissue with insulated electrodes (i.e. flow of ions is not necessary to create an electric field). Although not wanting to be bound by theory, it is the current that is necessary for successful electroporation not electric field per se.

During electroporation, the heat produced is the product of the inter-electrode impedance, the square of the current, and the pulse duration. Heat is produced during electroporation in tissues and can be derived as the product of the inter-electrode current, voltage and pulse duration. The protocols currently described for electroporation are defined in terms of the resulting field intensities ***E***, which are dependent on short voltage pulses of unknown current. Accordingly, the resistance or heat generated in a tissue cannot be determined, which leads to varied success with different pulsed voltage electroporation protocols with predetermined voltages. The ability to limit heating of cells across electrodes can increase the effectiveness of any given electroporation voltage pulsing protocol. For example, prior art teaches the utilization of an array of six needle electrodes utilizing a predetermined voltage pulse across opposing electrode pairs. This situation sets up a centralized pattern during an electroporation event in an area where congruent and intersecting overlap points develop. Excessive heating of cells and tissue along electroporation path will kill the cells, and limit the effectiveness of the protocol. However, symmetrically arranged needle electrodes without opposing pairs can produce a decentralized pattern during an electroporation event in an area where no congruent electroporation overlap points can develop.

Controlling the current flow between electrodes allows one to determine the relative heating of cells. Thus, it is the current that determines the subsequent effectiveness of any given pulsing protocol and not the voltage across the electrodes. Predetermined voltages do not produce predetermined currents, and prior art does not provide a means to determine the exact dosage of current, which limits the usefulness of the technique. Thus, controlling an maintaining the current in the tissue between two electrodes under a threshold will allow one to vary the pulse conditions, reduce cell heating, create less cell death, and incorporate macromolecules into cells more efficiently when compared to predetermined voltage pulses.

Overcoming the above problem by providing a means to effectively control the dosage of electricity delivered to the cells in the inter-electrode space by precisely controlling the ionic flux that impinges on the conduits in the cell membranes. The precise dosage of electricity to tissues can be calculated as the product of the current level, the pulse length and the number of pulses delivered. Thus, a specific embodiment of the present invention can deliver the electroporative current to a volume of tissue along a plurality of paths without, causing excessive concentration of cumulative current in any one location, thereby avoiding cell death owing to overheating of the tissue.

Although not wanting to be bound by theory, the nature of the voltage pulse to be generated is determine by the nature of tissue, the size of the selected tissue and distance between electrodes. It is desirable that the voltage pulse be as homogenous as possible and of the correct amplitude. Excessive field strength results in the lysing of cells, whereas a low field strength results in reduced efficacy of electroporation. Some electroporation devices utilize the distance between electrodes to calculate the electric field strength and predetermined voltage pulses for electroporation. This reliance on knowing the distance between electrodes is a limitation to the design of electrodes. Because the programmable current pulse controller will determine the impedance in a volume of tissue between two electrodes, the distance between electrodes is not a critical factor for determining the appropriate electrical current pulse. Therefore, an alternative embodiment of a needle electrode array design would be one that is non-symmetrical. In addition, one skilled in the art can imagine any number of suitable symmetrical and non-symmetrical needle electrode arrays that do not deviate from the spirit and scope of the invention. The depth of each individual electrode within an array and in the desired tissue could be varied with comparable results. In addition, multiple injection sites for the macromolecules could be added to the needle electrode array.

One example of an electroporation device that may be used to effectively facilitate the introduction of a macromolecule into cells of a selected tissue of a subject was described in U.S. Patent application 10/657,725 filed on 9/08/2003, titled "Constant Current Electroporation Device And Methods Of Use," with Smith et al., listed as inventors. The electroporation device comprises an electro-kinetic device ("EKD") whose operation is specified by software or firmware. The EKD produces a series of programmable constant-current pulse patterns between electrodes in an array based on user control and input of the pulse parameters and allows the storage and acquisition of current waveform data. The electroporation device also comprises a replaceable electrode disk having an array of needle electrodes, a central injection channel for an injection needle, and a removable guide disk.

### RESTRICTION ENZYMES

In some embodiments of the present invention, a linear DNA fragment is generated by restriction enzyme digestion of a parent DNA molecule. Examples of restriction enzymes are provided below.

| **Name** | **Recognition Sequence** |
|---|---|
| AatII | GACGTC |
| Acc65 I | GGTACC |
| Acc I | GTMKAC |
| Aci I | CCGC |
| Acl I | AACGTT |
| Afe I | AGCGCT |
| Afl II | CTTAAG |
| Afl III | ACRYGT |

| Age I | ACCGGT |
|---|---|
| Alu I | AGCT |
| Alw I | GGATC |
| AlwN I | CAGNNNCTG |
| Apa I | GGGCCC |
| ApaL I | GTGCAC |
| Apo I | RAATTY |
| Asc I | GGCGCGCC |
| Ase I | ATTAAT |
| Ava I | CYCGRG |
| Ava II | GGWCC |
| Avr II | CCTAGG |
| BamH I | GGATCC |
| Ban I | GGYRCC |
| Ban II | GRGCYC |
| Bbs I | GAAGAC |
| Bbv I | GCAGC |
| BbvC I | CCTCAGC |
| BciV I | GTATCC |
| Bcl I | TGATCA |
| Bfa I | CTAG |
| Bgl I | GCCNNNNNGGC |
| Bgl II | AGATCT |
| Blp I | GCTNAGC |
| Bmr I | ACTGGG |
| Bpm I | CTGGAG |
| BsaA I | YACGTR |
| BsaB I | GATNNNNATC |
| BsaH I | GRCGYC |
| Bsa I | GGTCTC |
| BsaJ I | CCNNGG |
| BsaW I | WCCGGW |
| BseR I | GAGGAG |
| Bsg I | GTGCAG |
| BsiE I | CGRYCG |
| BsiHKA I | GWGCWC |
| BsiW I | CGTACG |
| BsmA I | GTCTC |
| BsmB I | CGTCTC |
| BsmF I | GGGAC |
| Bsm I | GAATGC |
| BsoB I | CYCGRG |
| Bsp1286 I | GDGCHC |
| BspD I | ATCGAT |
| BspE I | TCCGGA |
| BspH I | TCATGA |
| BspM I | ACCTGC |
| BsrB I | CCGCTC |
| BsrD I | GCAATG |
| BsrF I | RCCGGY |
| BsrG I | TGTACA |
| Bsr I | ACTGG |
| BssH II | GCGCGC |
| BssK I | CCNGG |
| Bst4C I | ACNGT |
| BssS I | CACGAG |
| BstB I | TTCGAA |
| BstE II | GGTNACC |
| BstF5 I | GGATGNN |
| BstN I | CCWGG |
| BstU I | CGCG |
| BstY I | RGATCY |
| BstZ17 I | GTATAC |
| Bsu36 I | CCTNAGG |
| Btg I | CCPuPyGG |
| Btr I | CACGTG |
| Cac8 I | GCNNGC |
| Cla I | ATCGAT |
| Dde I | CTNAG |
| Dpn I | GATC |
| Dpn II | GATC |
| Dra I | TTTAAA |
| Eae I | YGGCCR |
| Eag I | CGGCCG |
| Ear I | CTCTTC |
| Eci I | GGCGGA |
| EcoO109 I | RGGNCCY |
| EcoR I | GAATTC |
| EcoR V | GATATC |
| Fau I | CCCGCNNNN |
| Fnu4H I | GCNGC |
| Fok I | GGATG |
| Fse I | GGCCGGCC |
| Fsp I | TGCGCA |
| Hac II | RGCGCY |
| Hae III | GGCC |
| Hga I | GACGC |
| Hha I | GCGC |
| Hinc II | GTYRAC |
| Hind III | AAGCTT |
| Hinf I | GANTC |
| HinP1 I | GCGC |
| Hpa I | GTTAAC |
| Hpa II | CCGG |
| Hph I | GGTGA |
| Kas I | GGCGCC |
| Kpn I | GGTACC |
| Mbo I | GATC |
| Mbo II | GAAGA |
| Mfe I | CAATTG |
| Mlu I | ACGCGT |
| Mnl I | CCTC |
| Msc I | TGGCCA |
| Mse I | TTAA |
| MspA1 I | CMGCKG |
| Msp I | CCGG |
| Nae I | GCCGGC |
| Nar I | GGCGCC |
| Nci I | CCSGG |
| Nco I | CCATGG |
| Nde I | CATATG |
| NgoMI V | GCCGGC |
| Nhe I | GCTAGC |
| Nla III | CATG |
| Nla IV | GGNNCC |
| Not I | GCGGCCGC |
| Nru I | TCGCGA |
| Nsi I | ATGCAT |
| Nsp I | RCATGY |
| Pac I | TTAATTAA |
| PaeR7 I | CTCGAG |
| Pci I | ACATGT |
| PleI | GAGTC |
| Pme I | GTTTAAAC |
| Pml I | CACGTG |
| PpuM I | RGGWCCY |
| Psi I | TTATAA |
| PspG I | CCWGG |
| PspOM I | GGGCCC |
| Pst I | CTGCAG |
| Pvu I | CGATCG |
| Pvu II | CAGCTG |
| Rsa I | GTAC |
| Rsr II | CGGWCCG |
| Sac I | GAGCTC |
| Sac II | CCGCGG |
| Sal I | GTCGAC |
| Sap I | GCTCTTC |
| Sau3A I | GATC |
| Sau96 I | GGNCC |
| Sbf I | CCTGCAGG |
| Sca I | AGTACT |
| ScrF I | CCNGG |
| SexA I | ACCWGGT |
| SfaN I | GCATC |
| Sfc I | CTRYAG |
| Sfo I | GGCGCC |
| SgrA I | CRCCGGYG |
| Sma I | CCCGGG |
| Sml I | CTYRAG |
| SnaB I | TACGTA |
| Spe I | ACTAGT |
| Sph I | GCATGC |
| Ssp I | AATATT |
| Stu I | AGGCCT |
| Sty I | CCWWGG |
| Swa I | ATTTAAAT |
| Taq I | TCGA |
| Tfi I | GAWTC |
| Tli I | CTCGAG |
| Tse I | GCWGC |
| Top45 I | GTSAC |
| Top509 I | AATT |
| TspR I | CAGTG |
| Tthlll I | GACNNNGTC |
| Xba I | TCTAGA |
| Xho I | CTCGAG |
| Xma I | CCCGGG |
| Xmn I | GAANNNNTTC |

The term "restriction enzyme digestion" of DNA as used herein refers to catalytic cleavage of the DNA with an enzyme that acts only at certain locations in the DNA. Such enzymes are called restriction endonucleases, and the sites for which each is specific is called a restriction site. The various restriction enzymes used herein are commercially available and their reaction conditions, cofactors, and other requirements as established by the enzyme suppliers are used. Restriction enzymes commonly are designated by abbreviations composed of a capital letter followed by other letters representing the microorganism from which each restriction enzyme originally was obtained and then a number designating the particular enzyme. In general, about 1µg of plasmid or DNA fragment is used with about 1-2 units of enzyme in about 20µl of buffer solution. Appropriate buffers and substrate amounts for particular restriction enzymes are specified by the manufacturer. Restriction enzymes are used to ensure plasmid integrity and correctness.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### EXAMPLE 1

### CONSTRUCTION OF DNA VECTORS AND METHODS IN ANIMAL SUBJECT

**DNA constructs:** In order to enhance the vaccination response and to improve the clinical outcome of subjects after an infectious challenge it was first necessary to design several GHRH constructs. Briefly, the plasmid vectors contained the muscle specific synthetic promoter SPc5-12 (SEQID#15)(Li et al., 1999) attached to a wild type species-specific or analog GHRH. Some wild-type GHRH sequences were cloned in our laboratory (dog, cat and horse); others (chicken, ovine, bovine, porcine, human) were synthesized according to the specialized literature. The analog GHRH sequences were generated by site directed mutagenesis as described (Draghia-Akli et al., 1999). Briefly, mammalian GHRH analog cDNA's were generated by site directed mutagenesis of GHRH cDNA (SEQID#18) (Altered Sites II *in vitro* Mutagenesis System, Promega, Madison, WI), and cloned into the BamHI/ Hind III sites of pSPc5-12, to generate the specific GHRH construct. The 3' untranslated region (3'UTR) of growth hormone was cloned downstream of GHRH cDNA. The resultant plasmids contained mammalian analog coding region for GHRH, and the resultant amino acid sequences were not naturally present in mammals. Although not wanting to be bound by theory, the enhancement of the vaccination response and the improvement of the clinical outcome of subjects after an infectious challenge are determined ultimately by the circulating levels of GHRH hormones. Several different plasmids encoded different mutated or wild type amino acid sequences of GHRH or functional biological equivalents thereof, for example:

| **Plasmid** | **Encoded Amino Acid Sequence** |
|---|---|
| **HV-GHRH(SEQID#1):** | **HV**DAIFTNSYRKVL**A**QLSARKLLQD**ILN**RQQGE**R**N**Q**E**Q**GA-OH |
| **Pig-GHRH(SEQID#2):** | **YA**DAIFTNSYRKVL**G**QLSARKLLQDI**MS**RQQGERN**Q**E**Q**GA-OH |
| **Bovine-GHRH (SEQID#3):** | **YA**DAIFTNSYRKVL**G**QLSARKLLQDI**MN**RQQGERN**Q**E**Q**GA-OH |
| **Dog-GHRH (SEQID#4):** | **YA**DAIFTNSYRKVL**G**QLSARKLLQDI**MS**RQQGE**R**NRE**Q**GA-OH |
| **Cat-GHRH (SEQID#5):** | **YA**DAIFTNSYRKVL**G**QLSARKLLQDI**MS**RQQGE**R**N**Q**E**Q**GA-OH |
| **TI-GHRH(SEQID#6):** | **YI**DAIFTNSYRKVL**A**QLSARKLLQDIL**N**RQQGE**R**N**Q**E**Q**GA-OH |
| **Ovine-GHRH (SEQID#7):** | **YA**DAIFTNSYRKILGQLSARKLLQDIMNRQQGERNQEQGA-OH |
| **Chicken-GHRH (SEQID#8):** | HADGIFSKAYRKLLGQLSARNYLHSLMAKRVGSGLGDEAEPLS-OH |
| **Horse-GHRH (partial) (SEQID#9):** | -ADAIFTNNYRKVLGQLSARKILQDIMSR-----------OH |
| **Human-GHRH(SEQID#10):** | **YA**DAIFTNSYRKVL**G**QLSARKLLQDI**MS**RQQGESN**Q**E**R**GA-OH |
| **TV-GHRH(SEQID#11):** | **YV**DAIFTNSYRKV**LA**QLSARKLLQDI**LN**RQQGE**R**NQE**Q**GA-OH |
| TA-15/27/28**-GHRH(SEQID#12):** | **YA**DAIFTNSYRKVL**A**QLSARKLLQDI**LN**RQQGE**R**N**Q**E**Q**GA-OH |

In general, the encoded GHRH or functional biological equivalent thereof is of formula:
-**X₁**-**X₂**-DAIFTNSYRKVL-**X₃**-QLSARKLLQDI-**X₄**-**X₅**-RQQGE-**X₆**-N-**X₇**-E-**X₈**-GA-OH (SEQID#14)

wherein: **X₁** is a D-or L-isomer of an amino acid selected from the group consisting of tyrosine ("Y"), or histidine ("H"); **X₂** is a D-or L-isomer of an amino acid selected from the group consisting of alanine ("A"), valine ("V"), or isoleucine ("I"); **X₃** is a D-or L-isomer of an amino acid selected from the group consisting of alanine ("A") or glycine ("G"); **X₄** is a D-or L-isomer of an amino acid selected from the group consisting of methionine ("M"), or leucine ("L"); **X₅** is a D-or L-isomer of an amino acid selected from the group consisting of serine ("S") or asparagines ("N"); **X₆** is a D- or L-isomer of an amino acid selected from the group consisting of arginine ("R"), or serine ("S"); **X₇** is a D- or L-isomer of an amino acid selected from the group consisting of arginine ("R"), or glutamine ("Q"); and **X₈** is a D- or L-isomer of an amino acid selected from the group consisting of arginine ("R"), or glutamine ("Q").

The plasmids described above do not contain polylinker, IGF-I gene, a skeletal alpha-actin promoter or a skeletal alpha actin 3' UTR /NCR. Furthermore, these plasmids were introduced by muscle injection, followed by *in vivo* electroporation, as described below.

In terms of "functional biological equivalents", it is well understood by the skilled artisan that, inherent in the definition of a "biologically functional equivalent" protein and/or polynucleotide, is the concept that there is a limit to the number of changes that may be made within a defined portion of the molecule while retaining a molecule with an acceptable level of equivalent biological activity. Functional biological equivalents are thus defined herein as those proteins (and poly-nucleotides) in selected amino acids (or codons) may be substituted. A peptide comprising a functional biological equivalent of GHRH is a polypeptide that has been engineered to contain distinct amino acid sequences while simultaneously having similar or improved biologically activity when compared to GHRH. For example one biological activity of GHRH is to facilitate GH secretion in the subject.

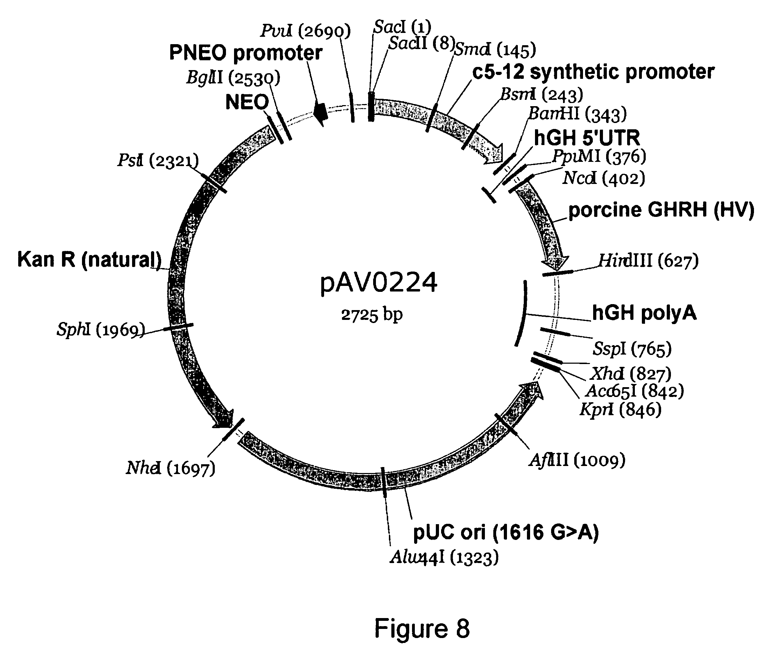

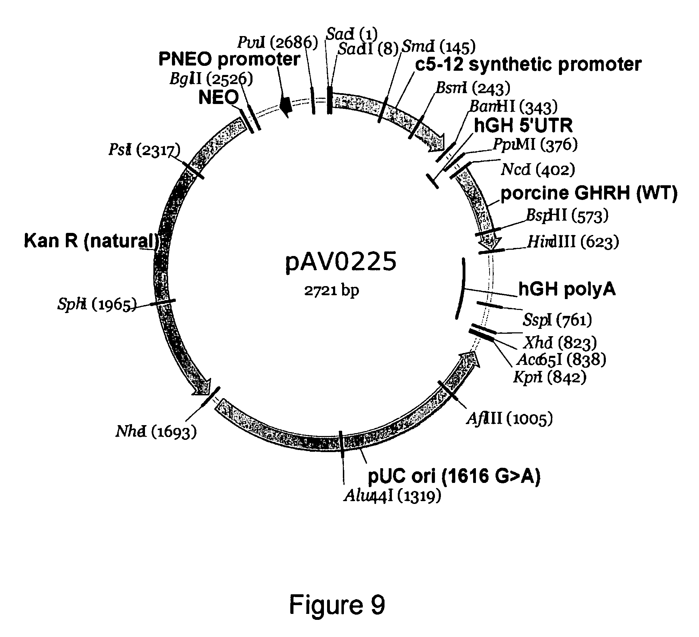

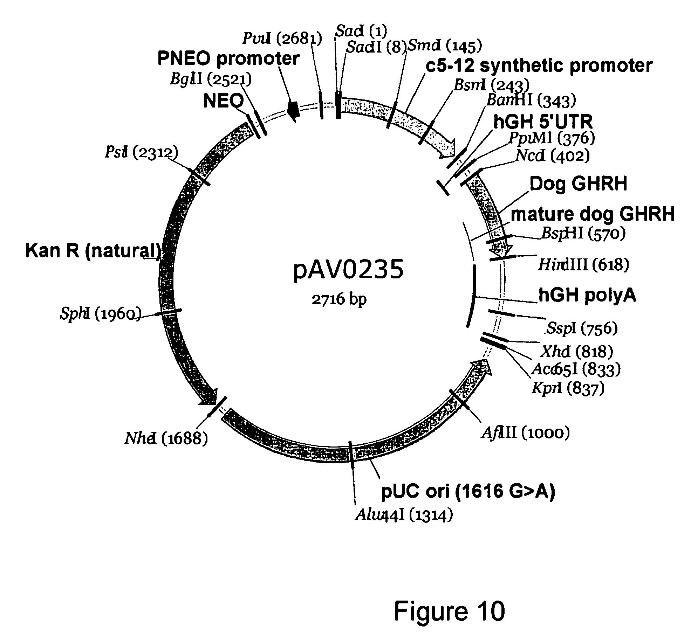

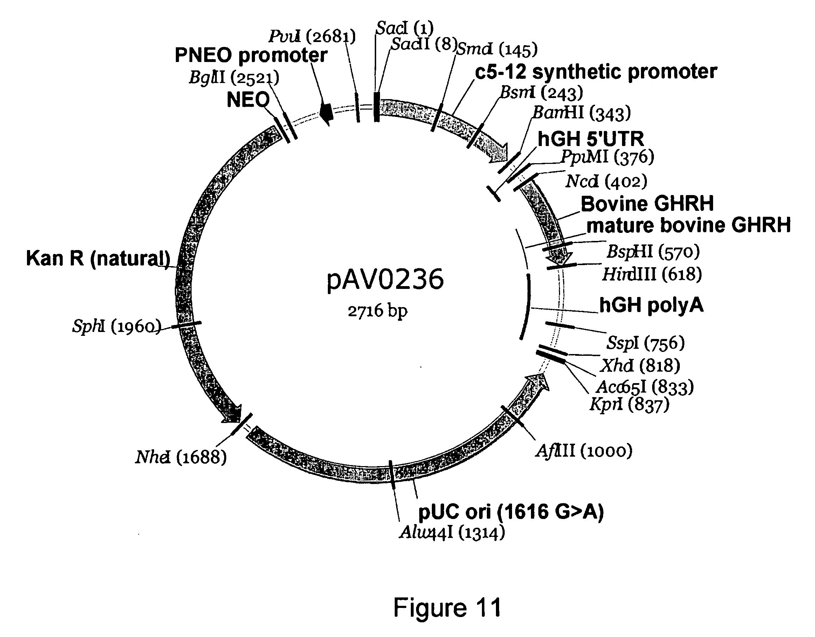

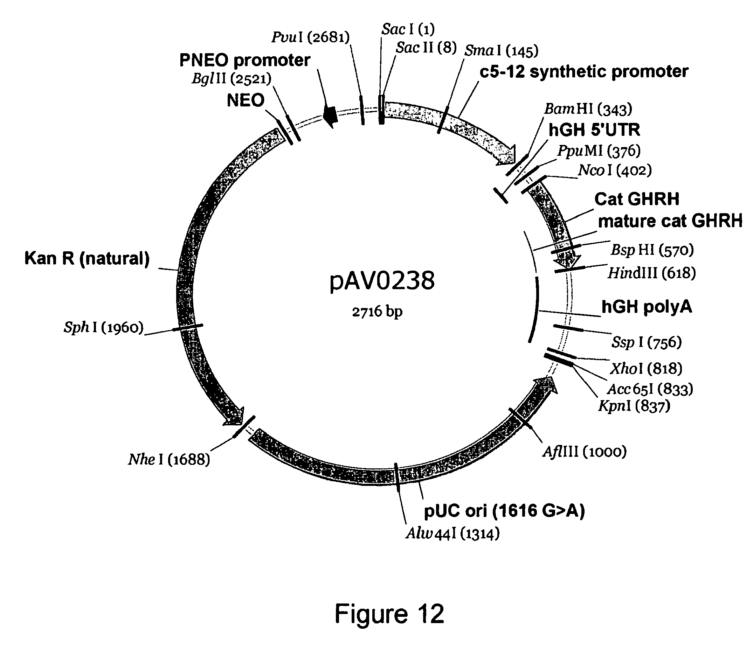

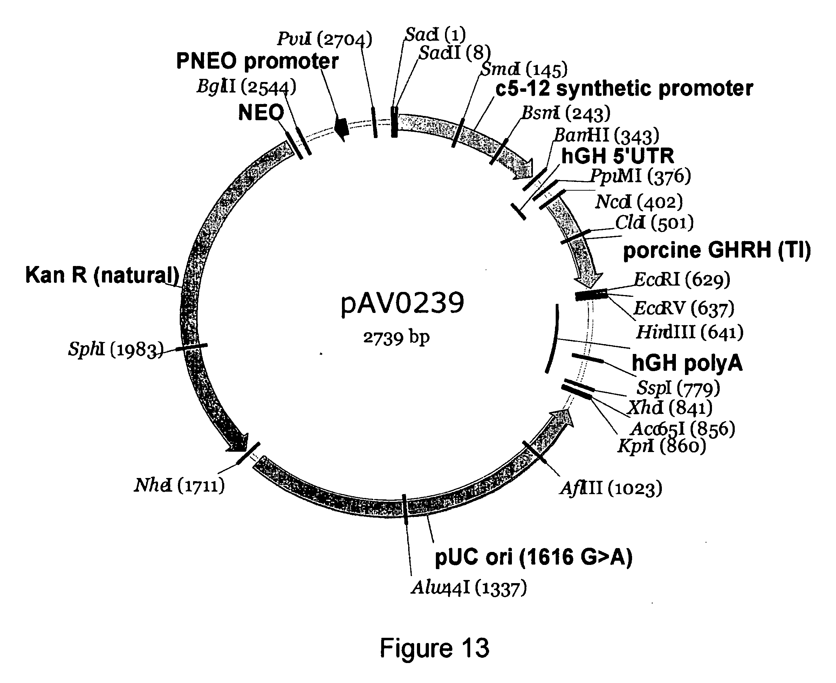

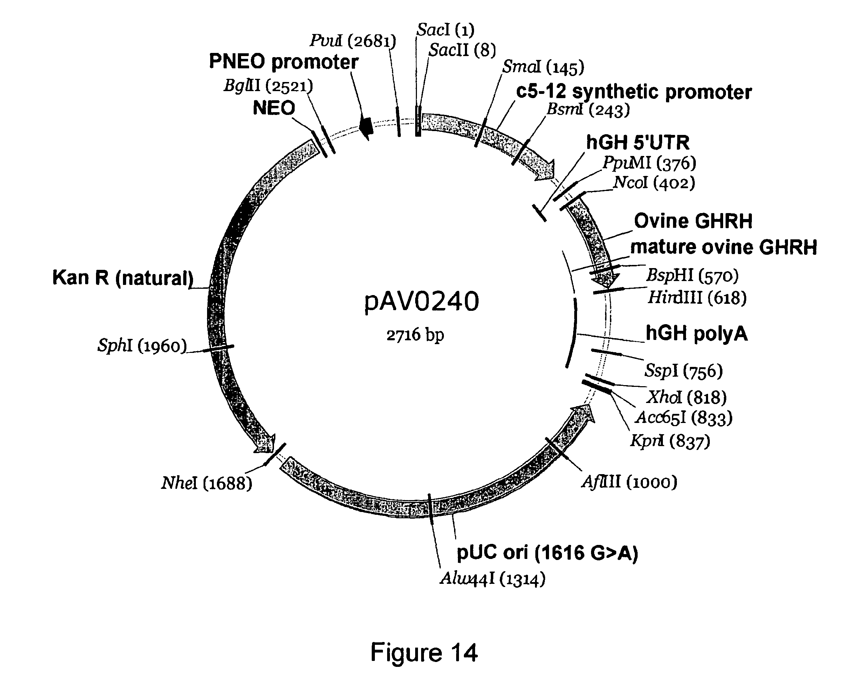

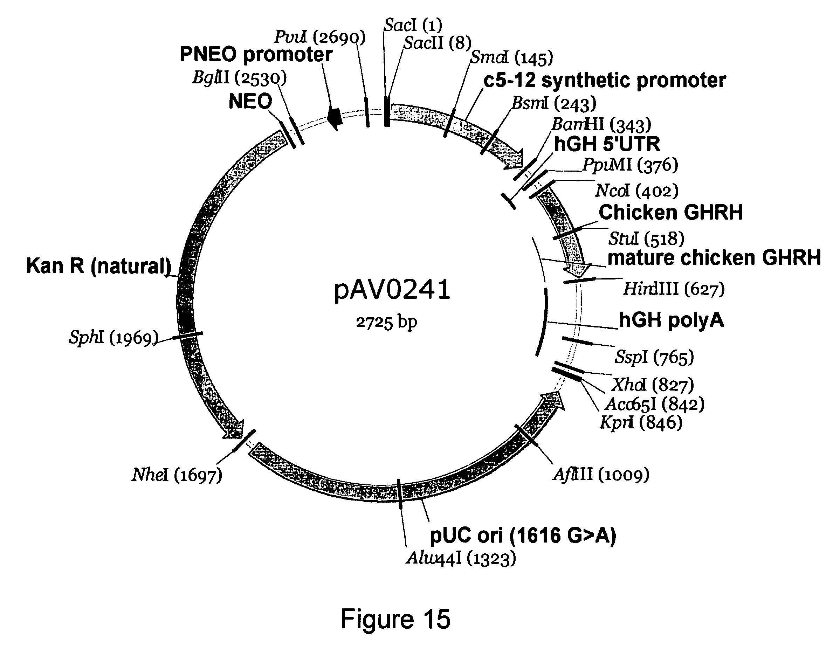

**Optimized Plasmid Backbone.** One aspect of the current invention is the optimized plasmid backbone. The synthetic plasmids presented below contain eukaryotic sequences that are synthetically optimized for species specific mammalian transcription. An existing pSP-HV-GHRH plasmid ("pAV0125") (SEQID#22), was synthetically optimized to form a new plasmid (SEQID#25). The plasmid pAV0125 was described in U.S. Patent 6,551,996 titled "Super-active porcine growth hormone releasing hormone analog," issued on April 22, 2003 with Schwartz, et al., listed as inventors ("the Schwartz '996 Patent"), which teaches application of a GHRH analog containing mutations that improve the ability to elicit the release of growth hormone. This 3,534 bp plasmid pAV0125 (SEQID #22) contains a plasmid backbone with various component from different commercially available plasmids, for example, a synthetic promoter SPc5-12 (SEQID#15), a modified porcine GHRH sequence (SEQID#18), and a 3'end of human growth hormone (SEQID#38). Other specific examples of optimized synthetic plasmids include an optimized wt-porcine GHRH plasmid, pAV0225 (SEQID#26) Figure 8; dog GHRH plasmid, pAV0235 (SEQID#27) Figure 9; bovine GHRH plasmid, pAV0236 (SEQID#28) Figure 10; cat GHRH plasmid, pAV0238 (SEQID#29) Figure 11; a TI-GHRH plasmid, pAV0239 (SEQID#30) Figure 12; ovine GHRH plasmid, pAV0240 (SEQID#31) Figure 13; chicken GHRH plasmid, pAV0241 (SEQID#32) Figure 14; horse GHRH plasmid, pAV0249 (SEQID#33) Figure 15; human GHRH plasmid (SEQID#34). The therapeutic encoded gene for such optimized plasmids may also include optimized nucleic acid sequences that encode modified GHRH molecules or functional biological equivalents thereof.

### EXAMPLE 2

### PLASMID INJECTION AND ELECTROPORATION CAN BE USED TO DELIVER ANTIGENS TO LARGE ANIMALS

Young hybrid pigs of mixed gender, 3 to 6 weeks of age, with weights between 15 and 40 kg, were used (n = 6 to 7/group/experiment). Animals were group housed in pens with *ad libitum* access to 24% protein diet (Producers Cooperative Association, Bryan, TX) and water. Plasmid was obtained using a commercially available kit (Qiagen Inc., Chatsworth, CA, USA). Endotoxin levels were at less than 0.01 EU/µg, as measured by Kinetic Chromagenic LAL (Endosafe, Charleston, SC). Plasmid preparations were diluted in sterile water and formulated 1% weight/weight with poly-L-glutamate sodium salt (MW=10.5 kDa average) (Sigma, St. Louis, MO). On Day 0 of the experiment, the animals were manually restrained and a secreted alkaline phosphatase (SEAP) expressing plasmid solution was directly injected through the intact skin into the semimembranosus muscle using a 21-gauge needle, in the center of the 5 pin electrode array. All major surface blood vessels were avoided when finding an appropriate injection site. At the pre-determined time interval after plasmid injection, in our case 80 milliseconds, electroporation was initiated: 5 pulses of 52 milliseconds in length, 0.4-1 Amp electric field intensity, 1 second between pulses. Animals were maintained in accordance with NIH, USDA and Animal Welfare Act guidelines.

*Blood collection:* On days 0, 3, 7, and 10 of each experiment, animals were weighed at 8:30 AM and blood was collected by jugular vein puncture into MICROTAINER serum separator tubes. Blood was allowed to clot for 10 to 15 min at room temperature and subsequently centrifuged at 3000 x g for 10 min and the serum stored at -80°C until further analysis.

*Secreted embryonic alkaline phosphatase assay:* Serum samples were thawed and 50µL was assayed for SEAP activity using the Phospha-Light Chemiluminescent Reporter Assay Kit (Applied Biosystems, Bedford, MA), per manufacturer instructions. The lower limit of detection for the assay is 3pg/mL. More concentrated serum samples were diluted 1:10 in mouse serum before assaying for SEAP activity. All samples were read using LUMIstar Galaxy luminometer (BMG Labtechnologies, Offenburg, Germany).

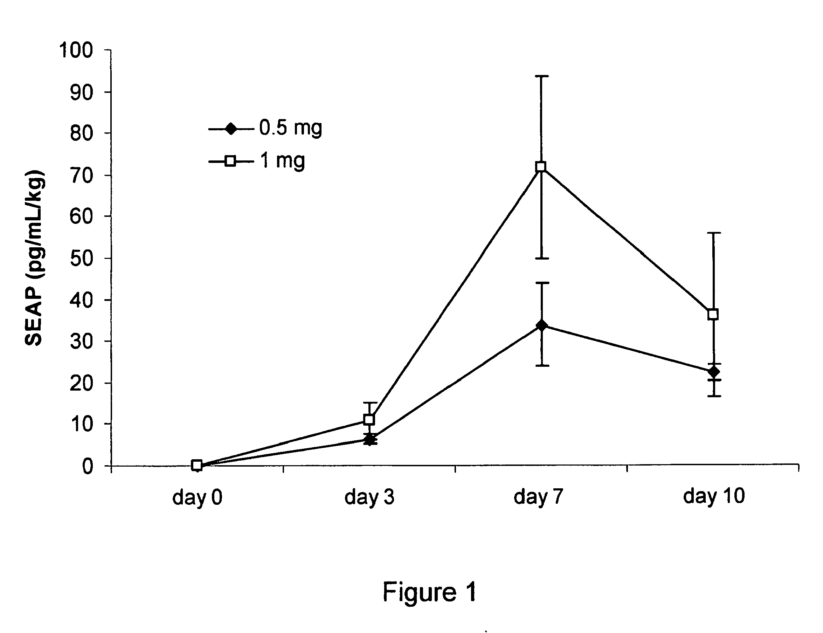

The SEAP protein is immunogenic in pigs, and the immune-mediated clearance of the protein does occur within 10 to 14 days after plasmid delivery (Figure 1) when direct muscle injection followed by electroporation is used. Thus, the levels of SEAP expression can be studied only over a 2-week period and interpreted as a reliable measure of gene expression following intramuscular plasmid transfer. Nevertheless, the technique could be used to deliver other types of molecules that specifically enhance the immune function in a subject. As a measurable vaccination response usually occurs approximately 14 days after the vaccination, it is reasonable that a treatment with an immuno-modulator, as GHRH, could occur before the vaccination, or concomitant with the vaccination procedure.

### EXAMPLE 3

### GHRH ADMINISTRATION IN COWS

Thirty-two primiparous Holstein cows, 18 to 20 months of age, with an average weight of 547 ± 43kg, were treated with 2.5mg pSP-HV-GHRH once during the last trimester of gestation and designated as the treated group. Similarly, 20 pregnant heifers from the same source and of the same breed and age did not receive plasmid treatment and served as controls. Animals calved at age 23 months ± 24 days. Cows were housed in a free stall barn fitted with fans equipped with water misters for evaporative cooling and exposed to natural daylight. The herd was fed a silage-based total mixed ration *ad libitum* twice daily. Each cow was fitted with a transponder/pedometer that allowed for automatic identification upon entering the stall. At the conclusion of this experiment, all animals treated with plasmid were disposed of in such a manner that their tissues did not enter the food chain. All milk and tissues produced by treated animals were destroyed and did not enter the human food chain. Animal protocols were conducted in accordance with the National Research Council's Guide for the Care and Use of Laboratory Animals.

***Intramuscular injection of plasmid DNA*.** The endotoxin-free plasmid (Qiagen Inc., Chatsworth, CA, USA) preparation of pSPc5-12-HV-GHRH was diluted in water to 5mg/mL and formulated with poly-L-glutamate 1% wt/wt. Cows were given a total quantity of 2.5mg pSP-HV-GHRH intramuscularly in the trapezius muscle using a 21G needle (Becton-Dickinson, Franklin Lacks, NJ). Two min after injection, the injected muscle was electroporated using Advisys's EKD device, and using the following conditions: 5 pulses, 1 Amp, 52 milliseconds/pulse, as described (Draghia-Akli et al., 2002b). The voltage changes with the change in resistance of the tissue during the electroporation (to maintain constant current), and it has been recorded to be between 80 and 120 V/cm. For all injections, 2cm needles were inserted through the skin into the muscle. Animals were observed immediately after injection and 24 h later for any adverse effects at the electroporation site.

***Weight, body condition and hoof scores.*** Before treatment, heifers were weighed on the same calibrated scale (Priefert cattle squeeze-chute connected to a Weigh Tronix 915A indicator and WP233 printer, Central City Scale, Central City, NE) and randomly assigned to groups. Two independent dairy animal scientists (Texas A&M University) that were blinded to the treatment groups assessed body condition scores prior to treatment, between 60 and 80 DIM and between 100 and 120 DIM. Cows were scored by both observing and handling the backbone, loin, and rump areas (Rodenburg, 1996), with possible BCS ranging from 1 (very thin cow) to 5 (a severely over-conditioned cow). Hoof scores were measured prior to plasmid-GHRH treatment and at 60 DIM. Hoof scores included a 0 (no hoof problem) to 4 (severe hoof problem) evaluation of each foot. Each hoof was assigned an additional 2 points for abscesses eventually present (1 point), and the necessary treatments at any given time (1 point). Possible hoof scores ranged from 0 (no problem) to 24 (severe hoof problems at all 4 feet, abscesses, and intense treatment needed for each hoof).

***Complete blood counts and immune markers.*** Whole blood from all heifers was collected in EDTA and submitted for complete blood count analysis (Texas Veterinary Medical Diagnostic Laboratory, College Station, TX) prior to treatment, and at 18 and 300 days post-treatment, and after a vaccination challenge. Hematology parameters included: erythrocyte counts, hematocrit, hemoglobin, total leukocyte count, and differential leukocyte counts (neutrophils, lymphocytes, monocytes, eosinophils, and basophils), platelet count, mean corpuscular volume, mean corpuscular hemoglobin, mean corpuscular hemoglobin concentration, and fibrinogen.

Immune markers were assayed on all treated cattle and controls at day 0 and 18 and on 20 treated and 10 control cows at 300 days post-treatment and after the Biocor-9 vaccination challenge. Analysis was performed by flow cytometry (FC) using monoclonal antibodies (mAb) developed in Dr. Davis's laboratory (Department of Veterinary Microbiology/ Pathology, CVM, Washington State University, Pullman, Washington). Combinations of mAbs were used in 3-color FC to determine the composition and frequency of different populations of peripheral blood mononuclear cells (PBMC) in peripheral blood and the functional status of CD4 and CD8 alpha beta (αβ) T cells and gamma delta (γδ) T cells. Two mAbs of the same specificity have been used in some combinations to increase the intensity of the fluorescent signal on CD4 and CD8 T cells. Ten mL of blood was obtained at the times indicated and processed for FC (Davis et al., 1995). The blood was lysed in Tris buffered NH₄Cl, washed in phosphate buffered saline containing acid citrate dextrose (PBS/ACD), and then distributed in 96 well conical bottom tissue culture plates containing the different combinations of mAbs. The cells were incubated for 15 min on ice, washed 3X in PBS/ACD and incubated another 15 min with different combinations of isotype specific goat anti-mouse antibodies conjugated with fluorescein, phycoerythrin, or phycoerythrin-Cy5 (Southern Biotechnology Associates, Birmingham, AL, Caltag Laboratories, Burlingame, CA). The cells were then washed 2X and fixed in PBS buffered 2% formaldehyde. The cells were kept in the refrigerator until examined. The cells were examined on a Becton Dickinson FACSort equipped with Cell Quest software. Data were analyzed on Flow Jo (Tree Star, Inc., San Carlos, CA) and FCS Express (De Novo software, Thornton, Ontario, Ca) software. Unless otherwise stated, data are presented as a percentage of the total population assayed with a particular monoclonal antibody or combination thereof (e.g. total CD4⁺ and CD4- cells represent 100% of cells assayed; total CD2-CD3-, CD2-CD3+, CD2+CD3- or CD2+CD3+ represent 100% of cells assayed with both CD2 and CD3 antibody, etc.).

***Biochemistry and insulin measurements.*** Serum samples were collected at 60 and 100 DIM. Serum was aliquoted for RIA and biochemical analysis and stored at -80°C prior to analysis. Biochemical analysis occurred within 48 h after serum collection (Texas Veterinary Medical Diagnostic Laboratory, College Station, TX). Serum biochemical endpoints included alanine aminotransferase, gamma glutamyltransferase, creatine phosphokinase, total bilirubin, total protein, albumin, globulin, blood urea nitrogen, creatinine, phosphorus, calcium, and glucose. Insulin and IGF-I assays were performed within 90 days after serum collection. Samples were analyzed for glucose and insulin levels by an independent laboratory (Texas Veterinary Medical Diagnostic Laboratory, College Station, TX). All samples were analyzed in the same assay. The assay variability was 3.6% for the insulin assay and 4.4% for the glucose assay. Total proteins were measured using a Bio-Rad protein assay kit on the serum samples (Bio-Rad Laboratories, Hercules, CA).

***Radioimmunoassay fo**r **IGF-I.*** Serum IGF-I was measured using a heterologous human immunoradiometric assay kit following the manufacturer's protocol (Diagnostic System Labs, Webster, TX). The kit employs an extraction step to remove binding protein interference. All samples were run in the same assay. The intra-assay variability was 4%. Cross-reactivity of human IGF-I antibody for bovine IGF-I is 100%.

***Surround*^{™} *9 vaccination*:** All animals enrolled in the study (GHRH-treated and controls) were vaccinated at 300 days after the study initiation, using a 9-way vaccine called Surround^{™} 9 (Biocor). Surround^{™} 9 is a multivalent vaccine containing inactivated, adjuvanted, highly antigenic strains of IBR (bovine herpesvirus-1), BVD (bovine virus diarrhea), parainfluenza 3, respiratory syncytial virus (as killed virus), and Leptospira canicola, Leptospira grippotyphosa, Leptospira hardjo, Leptospira icterohaemorrhagiae and Leptospira Pomona bacterins. All fractions are inactivated and adjuvanted to maintain maximum virus antigenicity. The Leptospira serovars are produced in media designed to assure maximum growth and antigenicity. This vaccination addressed a large array of pathogenic conditions in cows, from diarrhea, and pathogens involved in the etiology of mastitis, to respiratory disease. The vaccine is administered subcutaneously or intramuscularly in one injection of a 5 mL solution.

***Statistical analysis*.** Data consisted of repeated measures in different time points with unequal allocation of experimental units to treatment groups (treated n = 32, controls n = 20). Additional comparisons were performed when a significant (P < 0.05) treatment-x-day interaction was detected. A mixed model using SAS (analysis of simple main effects) was used to examine if there were any significant differences among the groups of each variable at different time points. Categorical data, such as morbidity and mortality, culling rate and hoof problems, were analyzed by ANOVA. Data were coded with numerical values such that ANOVA could be performed. The total hoof score for each animal was used in the analysis of this parameter. For mortality rates, an equivalent scoring system: alive = 1, dead = 0 was used. Serum IGF-I was analyzed by ANOVA for repeated measures. Values compared with Students t-test, ANOVA or linear regression are presented in the results, with *P* < 0.05 taken as the level of statistical significance.

### EXAMPLE 4

### CLINICAL RESPONSE OF COWS TREATED WITH GHRH PLASMID THERAPY AND VACCINATED WITH A MULTIVALENT VACCINE

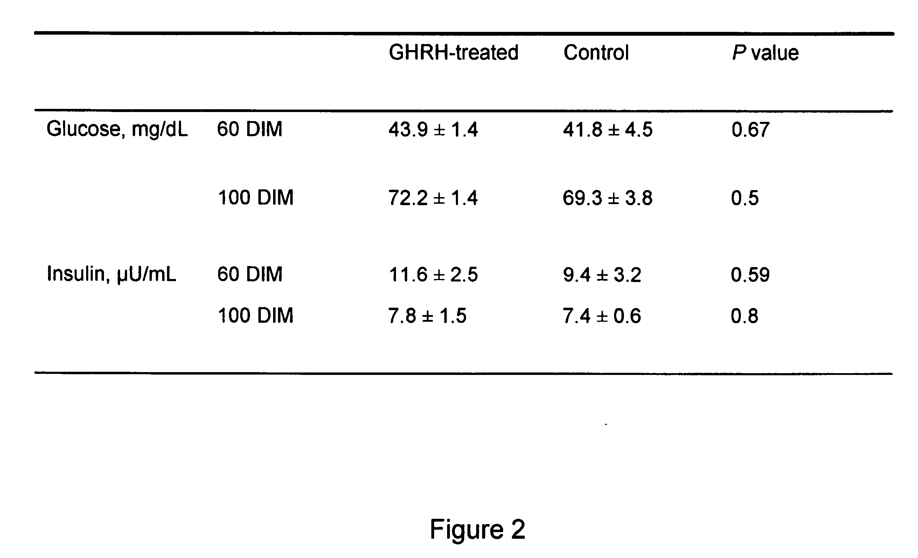

**Biochemistry and CBC values:** The total white blood cell counts were similar between groups. Nevertheless, the percentage of circulating lymphocytes at 300 days was increased in GHRH-treated animals (47.4 ± 3.3% vs. controls 37.8 ± 5.3%, P < 0.06). A physiological increase in hemoglobin (11.55 ± 0.15g/dL in GHRH-treated vs. 10.9 ± 0.15g/dL in controls, P < 0.02) and red blood cells (7.65 ± 0.1 millions/mL vs. 7.3 ± 0.2 millions/mL, P < 0.07) was also observed at this time point. No differences were found between the groups in other CBC or serum biochemistry panels at any time point tested. These were within the normal range of values for cattle. Glucose and insulin levels were not different between groups (Figure 2).

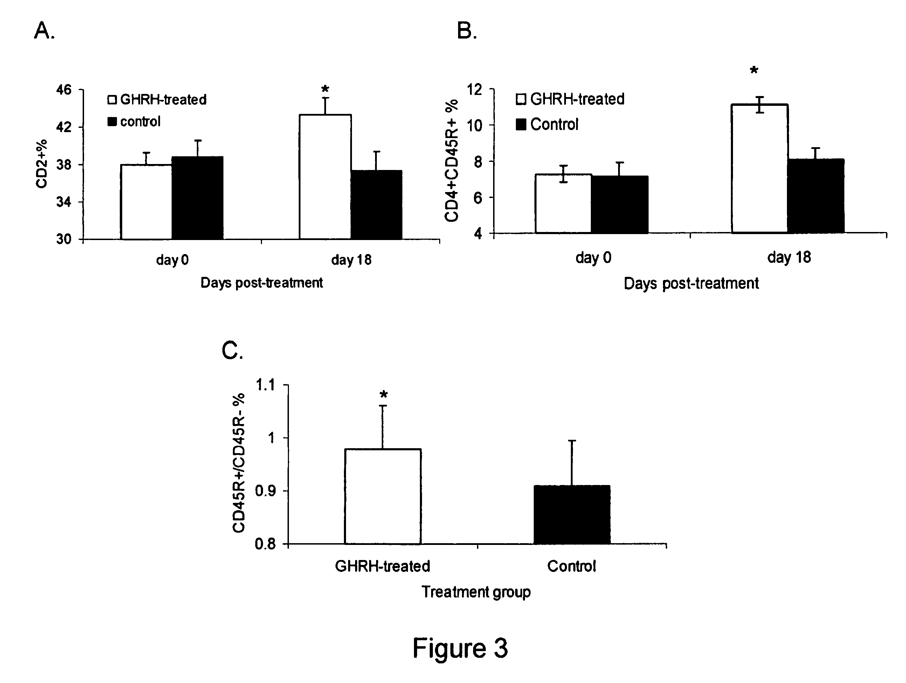

**Immune markers:** The total number of white blood cells, differentials, and flow cytometric (FC) profiles were similar between groups at day 0. At 18 days post-treatment, CD2⁺ values were increased in treated animals by 14%, but there was no change in controls when compared to baseline values: 43.4 ± 1.7% vs. 37.9 ± 1.4%, P < 0.004 in GHRH-treated cattle, and 37.3 ± 2.1% vs. 38.8 ± 1.8% in controls, (Figure 3A). The CD4⁺/CD8⁺ ratio increased in treated animals (day 18 - day 0 = 8 ± 0.6%, P < 0.04), mostly due to increase in CD4⁺ cells (29.1 ± 0.7% at day 18 vs. 24.5 ± 0.8% at day 0). During the same period CD4⁺CD45R⁺ naïve lymphocytes increased by 53% with the GHRH treatment: day 18, 11.1 ± 0.4% vs. day 0, 7.4 ± 0.4% in GHRH-treated animals, P < 0.016, and day 18, 8 ± 0.7% vs. day 0, 7.2 ± 0.8% in control animals (Figure 3B). CD25⁺CD4⁺ cells were also significantly increased with treatment: day 18, 4.3 ± 0.3% vs. day 0, 1 ± 0.1 in GHRH-treated animals P < 0.001 and day 18, 3.8 ± 0.2% vs. day 0,1.7 ± 0.2 in controls.

At 300 days post-treatment, when a more comprehensive panel was performed, the CD45R⁺/CD45R0- naïve lymphocytes were significantly more numerous in treated animals 0.98 ± 0.08 than in controls 0.91 ± 0.08, *P* < 0.05 (Figure 3C). CD2⁺CD3⁺γδ- cells were more numerous with treatment: 68.5 ± 1.4% of all CD2⁺ cells vs. 60 ± 5.6% in controls, *P* < 0.02.

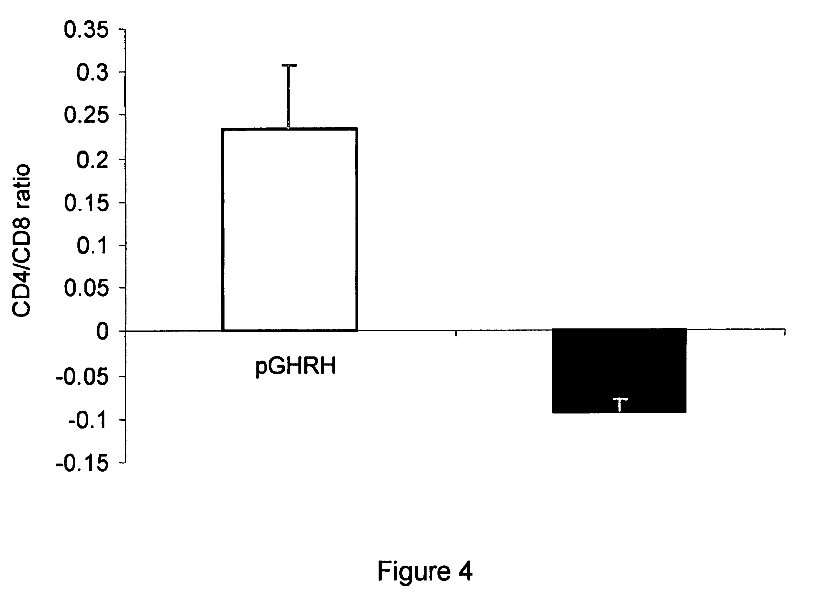

Two weeks after the Surround^{™} 9-way vaccination (Biocor), when a more comprehensive panel was performed, the CD4⁺/CD8⁺ ratio was significantly increased in GHRH-treated animals, while it was decreased in controls: 0.23 ± 0.07 in GHRH-treated animals, versus - 0.09 ± 0.01 in controls, *P* < 0.05 (Figure 4).

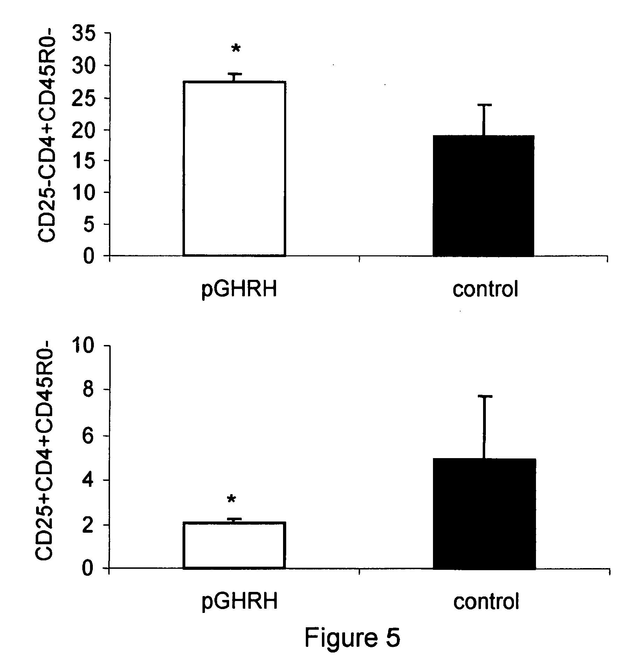

Two weeks after the Surround 9-way vaccination, when the more comprehensive panel was performed, the CD25⁻CD4⁺CD45R0 naïve T-lymphocytes were significantly increased in GHRH-treated animals compared to controls, *P* < 0.05 (Figure 5).

**Mortality in treated animals:** The mortality of the heifers (involuntary cull rate) was different between GHRH-treated animals and controls. During the 360-day study, none of the treated heifers died, while 20% of control heifers had to be culled (P < 0.003). The causes of death were the following: one Johne's disease, one systemic infection from hoof conditions and an infected cut, one animal with severe hoof problems complicated by rear leg paralysis, and one severe mastitis case. One treated animal was culled due to an accident. The overall involuntary cull rate prior to 120 days in milk production (DIM) was decreased by 40% with the treatment. The protection after vaccination against environmental pathogens was increased by the GHRH-plasmid mediated treatment.

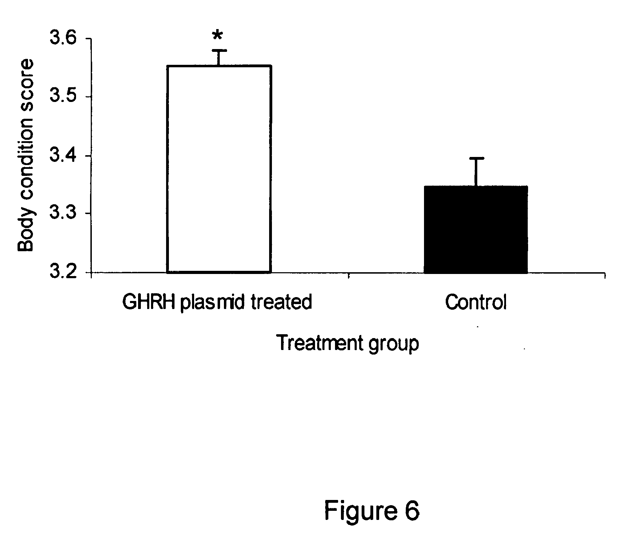

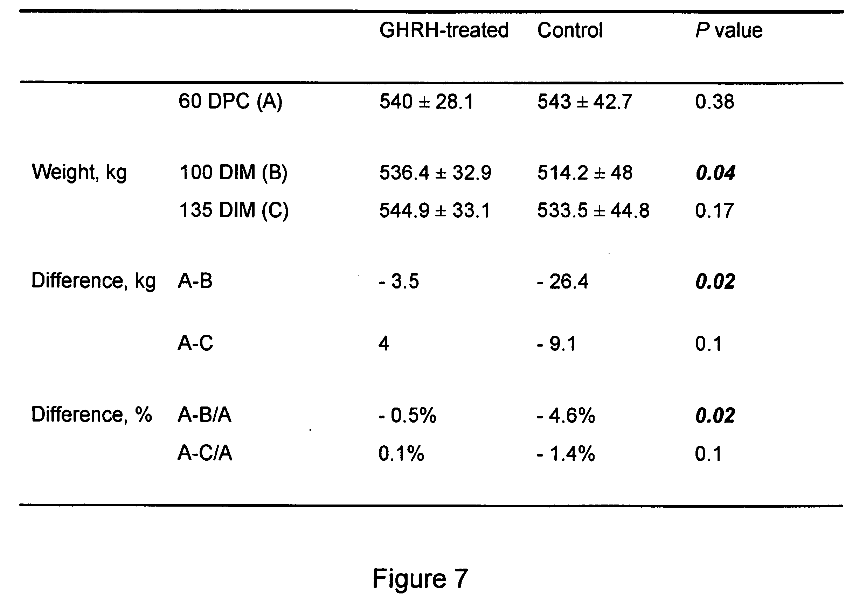

**Body weights and body condition score:** Body condition scores (BCS) of heifers differed between groups at the time of stress and negative energy balance, at 60 to 80 DIM. Heifers treated with pSP-HV-GHRH showed an improvement (*P* < 0.0001, Figure 6) in BCS between 60 and 80 DIM. During the first 100 DIM, treated animals lost an average of 3.5kg (0.06% of total body weight) (P < 0.02, Figure 7) while control cows lost on average 26.4kg (4.6% of body weight at 60 DIM). The better BCS correlated with an increase in the serum IGF-I levels: day 100 - day 60 = 22.4 ± 4ng/mL for GHRH-treated heifers (119.7 ± 6.9 ng/mL at day 100 vs. 97.3 ± 6.6 ng/mL at day 60) vs. 8 ± 7.4ng/mL for controls (99.8 ± 3.9 ng/mL at day 100 vs. 91.8 ± 6.8 ng/mL at day 60) (*P* < 0.04).

**Morbidity:** The herd had significant hoof pathology at the beginning of the study prior to plasmid administration. Foot problems, most probably of bacterial origin (Murray et al., 1996), were also one of the principal causes of morbidity in these animals throughout the study. The digital dermatitis lesions that constituted the major hoof pathology were attenuated by the GHRH treatment. Studies have shown that as much as 29% of dairy cattle and 4% of beef cattle have gross lesions of digital dermatitis and that spirochetes are involved in more than 60% of the cases (Brown et al., 2000). The immune response to the spirochetes is of short duration (Trott et al., 2003), thus, to diminish the infection burden, a stable long-term therapy would be preferable. The combination of vaccination and GHRH treatment proved to be beneficial for the treated animals. The proportion of animals that had worsening foot problems throughout the course of the study was 40% higher for controls when compared to the treated animals: 7 out of 32 GHRH-treated animals versus 7 out of 20 controls. The overall hoof score improvement did not reach statistical significance (*P* < 0.4) due to high inter-animal variability in the control group.

In contrast to injections with porcine recombinant somatotropin (rpST) or bST, which can produce unwanted side effects (e.g. hemorrhagic ulcers, vacuolations of liver and kidney or even death of the animals (Smith et al., 1991)), the plasmid mediated GHRH gene supplementation is well tolerated having no observed side effects in the animals. Regulated tissue/fiber-type-specific hGH-containing plasmids have been used previously for the delivery and stable production of GH in livestock and GH-deficient hosts. The methods used to deliver the hGH-containing plasmids comprise transgenesis, myoblast transfer or liposome-mediated intravenous injection (Barr and Leiden, 1991; Dahler et al., 1994; Pursel et al., 1990). Nevertheless, these techniques have significant disadvantages that preclude them from being used in a large-scale operation and/or on food animals, including: 1) possible toxicity or immune response associated with liposome delivery; 2) need for extensive *ex vivo* manipulation in the transfected myoblast approach; and/or 3) risk of important side effects or inefficiency in transgenesis (Dhawan et al., 1991; Miller et al., 1989). Compared to these techniques, plasmid mediated gene supplementation and DNA injection is simple and effective, with no complication related to the delivery system or to excess expression.

The embodiments provided herein illustrate that enhanced vaccination response and improved clinical outcome results in mammals injected with a GHRH plasmid. Treated subjects display a significant improvement the subject's capability to respond to an infectious challenge. Treated subjects did not experience any side effects from the therapy, including associated pathology or death. Although not wanting to be bound by theory, the enhancement in the vaccination response indicates that ectopic expression of myogenic GHRH vectors will likely stimulate the GH axis in a more physiologically appropriate manner.

One skilled in the art readily appreciates that this invention is well adapted to carry out the objectives and obtain the ends and advantages mentioned as well as those inherent therein. Growth hormone, growth hormone releasing hormone, analogs, plasmids, vectors, pharmaceutical compositions, treatments, methods, procedures and techniques described herein are presently representative of the preferred embodiments and are intended to be exemplary and are not intended as limitations of the scope. Additionally, vaccines comprising bovine herpesvirus-1 ("IBR"), bovine virus diarrhea ("BVD"), parainfluenza 3, respiratory syncytial virus, Leptospira canicola, Leptospira grippotyphosa, Leptospira hardjo, Leptospira icterohaemorrhagiae, Leptospira Pomona bacterins; mycoplasma hyopneumonia, mycoplasma hyopneumonia, or combinations thereof are intended to be exemplary and are not intended as limitations of the scope.

### REFERENCES CITED

### U.S. PATENT DOCUMENTS

U.S. Patent No. 5,847,066 issued on December 8, 1998 with Coy et al*.* listed as inventors
U.S. Patent No. 5,846,936 issued on December 8, 1998 with Felix et al*.* listed as inventors.
U.S. Patent No. 5,792,747 issued on August 11, 1998 with Schally et al*.* listed as inventors.
U.S. Patent No. 5,776,901 issued on July 7, 1998 with Bowers et al*.* listed as inventors.
U.S. Patent No. 5,756,264 issued on May 26, 1998 with Schwartz et al*.* listed as inventors.
U.S. Patent No. 5,696,089 issued on December 9, 1997 with Felix et al*.* listed as inventors.
U.S. Patent No. 5,486,505 issued on January 23, 1996 with Bowers et al*.* listed as inventors.
U.S. Patent No. 5,292,721 issued on March 8, 1994 with Boyd et al*.* listed as inventors.
U.S. Patent No. 5,137,872 issued on August 11, 1992 with Seely et al*.* listed as inventors.
U.S. Patent No. 5,134.210 issued on July 28, 1992 with Boyd et al*.* listed as inventors.
U.S. Patent No. 5,084,442 issued on January 28, 1992 with Felix et al*.* listed as inventors.
U.S. Patent No. 5,061,690 issued on October 29, 1991 with Kann et al*.* listed as inventors.
U.S. Patent No. 5,036,045 issued on July 30, 1991 with Thomer listed as the inventor.
U.S. Patent No. 5,023,322 issued on June 11, 1991 with Kovacs et al*.* listed as inventors.
U.S. Patent No. 4,839,344 issued on June 13, 1989 with Bowers et al*.* listed as inventors.
U.S. Patent No. 4,410,512 issued on October 18, 1983 with Bowers et al*.* listed as inventors.
U.S. Patent No. RE33,699 issued on September 24, 1991 with Drengler listed as the inventor.
U.S. Patent No. 4,833,166 issued on May 23, 1989 with Grosvenor et al*.* listed as inventors.
U.S. Patent No. 4,228,158 issued on October 14, 1980 with Momany et al*.* listed as inventors.
U.S. Patent No. 4,228,156 issued on October 14, 1980 with Momany et al*.* listed as inventors.
U.S. Patent No. 4,226,857 issued on October 7, 1980 with Momany et al*.* listed as inventors.
U.S. Patent No. 4,224,316 issued on September 23, 1980 with Momany et al. listed as inventors.
U.S. Patent No. 4,223,021 issued on September 16, 1980 with Momany et al. listed as inventors.
U.S. Patent No. 4,223,020 issued on September 16, 1980 with Momany et al*.* listed as inventors.
U.S. Patent No. 4,223,019 issued on September 16, 1980 with Momany et al*.* listed as inventors.
U.S. Patent No. 5,702,359 titled "Needle electrodes for mediated delivery of drugs and genes," issued on December 30, 1997, with Hofmann, et al., listed as inventors.
U.S. Patent No. 5,439,440 titled "Electroporation system with voltage control feedback for clinical applications," issued on August 8, 1995 with Hofmann listed as inventor.
PCT application WO/96/12520 titled "Electroporetic Gene and Drug Therapy by Induced Electric Fields," published on May 5, 1996 with Hofmann et al., listed as inventors.
PCT application WO/96/12006 titled "Flow Through Electroporation Apparatus and Method," published on April 25, 1996 with Hofmann et al., listed as inventors.
PCT application WO/95/19805 titled "Electroporation and Iontophoresis Apparatus and Method For insertion of Drugs and genes into Cells," published on July 27, 1995 with Hofmann listed as inventor.
PCT application WO/97/07826 titled "In Vivo Electroporation of Cells," published on March 6, 1997, with Nicolau et al., listed as inventors.

### Reference List

Acsadi, G., G. Dickson, D. R. Love, A. Jani, F. S. Walsh, A. Gurusinghe, Wolff, JA, and K. E. Davies. 1991. Human dystrophin expression in mdx mice after intramuscular injection of DNA constructs. Nature 352:815-818.
Aihara, H. and J. Miyazaki. 1998. Gene transfer into muscle by electroporation in vivo. Nat. Biotechnol. 16:867-870.
Almendro, N., T. Bellon, C. Rius, P. Lastres, C. Langa, A. Corbi, and C. Bernabeu. 1996. Cloning of the human platelet endothelial cell adhesion molecule-1 promoter and its tissue-specific expression. Structural and functional characterization. J. Immunol. 157:5411-5421.
Alpdogan, O., S. J. Muriglan, B. J. Kappel, E. Doubrovina, C. Schmaltz, R. Schiro, J. M. Eng, A. S. Greenberg, L. M. Willis, J. A. Rotolo, R. J. O'Reilly, and M. R. van den Brink. 2003. Insulin-like growth factor-1 enhances lymphoid and myeloid reconstitution after allogeneic bone marrow transplantation. Transplantation 75:1977-1983.
Aratani, Y., R. Okazaki, and H. Koyama. 1992. End extension repair of introduced targeting vectors mediated by homologous recombination in mammalian cells. Nucleic Acids Res. 20:4795-4801.
Babiuk, L. A., R. Pontarollo, S. Babiuk, B. Loehr, and van Drunen Littel-van den Hurk. 2003. Induction of immune responses by DNA vaccines in large animals. Vaccine 21:649-658.
Barr, E. and J. M. Leiden. 1991. Systemic delivery of recombinant proteins by genetically modified myoblasts. Science 254:1507-1509.
Bercu, B. B. and R. F. Walker. 1997. Growth Hormone Secretagogues In Children With Altered Growth. Acta Paediatrica 86:102-106.
Bettan, M., F. Emmanuel, R. Darteil, J. M. Caillaud, F. Soubrier, P. Delaere, D. Branelec, A. Mahfoudi, N. Duverger, and D. Scherman. 2000. High-level protein secretion into blood circulation after electric pulse-mediated gene transfer into skeletal muscle. Mol. Ther. 2:204-210.
Blethen, S. L. and A. C. Rundle. 1996. Slipped capital femoral epiphysis in children treated with growth hormone. A summary of the National Cooperative Growth Study experience. Horm. Res. 46:113-116.
Bohlen, P., F. Esch, P. Brazeau, N. Ling, and R. Guillemin. 1983. Isolation and characterization of the porcine hypothalamic growth hormone releasing factor. Biochem. Biophys. Res. Commun. 116:726-734.
Boshart, M., F. Weber, G. Jahn, K. Dorsch-Hasler, B. Fleckenstein, and W. Schaffner. 1985. A very strong enhancer is located upstream of an immediate early gene of human cytomegalovirus. Cell 41:521-530.
Brown, C. C., P. D. Kilgo, and K. L. Jacobsen. 2000. Prevalence of papillomatous digital dermatitis among culled adult cattle in the southeastern United States. Am. J. Vet. Res. 61:928-930.
Burgess, W., Q. Liu, J. Zhou, Q. Tang, A. Ozawa, R. VanHoy, S. Arkins, R. Dantzer, and K. W. Kelley. 1999. The immune-endocrine loop during aging: role of growth hormone and insulin-like growth factor-I. Neuroimmunomodulation. 6:56-68.
Carbonelli, D. L., E. Corley, M. Seigelchifer, and J. Zorzopulos. 1999. A plasmid vector for isolation of strong promoters in Escherichia coli. FEMS Microbiol. Lett. 177:75-82.
Caroni, P. and C. Schneider. 1994. Signaling by insulin-like growth factors in paralyzed skeletal muscle: rapid induction of IGF1 expression in muscle fibers and prevention of interstitial cell proliferation by IGF-BP5 and IGF-BP4. J. Neurosci. 14:3378-3388.
Chandler, S. D., A. Mayeda, J. M. Yeakley, A. R. Krainer, and X. D. Fu. 1997. RNA splicing specificity determined by the coordinated action of RNA recognition motifs in SR proteins. Proc. Natl. Acad. Sci. U. S. A 94:3596-3601.
Chevalier, R. L., S. Goyal, A. Kim, A. Y. Chang, D. Landau, and D. LeRoith. 2000. Renal tubulointerstitial injury from ureteral obstruction in the neonatal rat is attenuated by IGF-1. Kidney Int. 57:882-890.
Chung, C. S., T. D. Etherton, and J. P. Wiggins. 1985. Stimulation of swine growth by porcine growth hormone. J. Anim Sci. 60:118-130.
Cocea, L. 1997. Duplication of a region in the multiple cloning site of a plasmid vector to enhance cloning-mediated addition of restriction sites to a DNA fragment. Biotechniques 23:814-816.
Corpas, E., S. M. Harman, M. A. Pineyro, R. Roberson, and M. R. Blackman. 1993. Continuous subcutaneous infusions of growth hormone (GH) releasing hormone 1-44 for 14 days increase GH and insulin-like growth factor-I levels in old men. J Clin Endocrinol Metab 76:134-138.
Dahler, A., R. P. Wade, G. E. Muscat, and M. J. Waters. 1994. Expression vectors encoding human growth hormone (hGH) controlled by human muscle-specific promoters: prospects for regulated production of hGH delivered by myoblast transfer or intravenous injection. Gene 145:305-310.
Dai, B., H. Wu, E. Holthuizen, and P. Singh. 2001. Identification of a novel cis element required for cell density-dependent down-regulation of insulin-like growth factor-2 P3 promoter activity in Caco2 cells. J. Biol. Chem. 276:6937-6944.
Danko, I. and J. A. Wolff. 1994. Direct gene transfer into muscle. Vaccine 12:1499-1502.
Darquet, A. M., B. Cameron, P. Wils, D. Scherman, and J. Crouzet. 1997. A new DNA vehicle for nonviral gene delivery: supercoiled minicircle. Gene Ther. 4:1341-1349.
Darquet, A. M., R. Rangara, P. Kreiss, B. Schwartz, S. Naimi, P. Delaere, J. Crouzet, and D. Scherman. 1999. Minicircle: an improved DNA molecule for in vitro and in vivo gene transfer. Gene Ther. 6:209-218.
Davis, H. L., R. G. Whalen, and B. A. Demeneix. 1993. Direct gene transfer into skeletal muscle in vivo: factors affecting efficiency of transfer and stability of expression. Human Gene Therapy 4:151-159.
Davis, W. C., J. E. Davis, and M. J. Hamilton. 1995. Use of monoclonal antibodies and flow cytometry to cluster and analyze leukocyte differentiation molecules. Methods Mol. Biol. 45:149-67.:149-167.
Dhawan, J., L. C. Pan, G. K. Pavlath, M. A. Travis, A. M. Lanctot, and H. M. Blau. 1991. Systemic delivery of human growth hormone by injection of genetically engineered myoblasts. Science 254:1509-1512.
Dialynas, E., H. Brown-Borg, and A. Bartke. 1999. Immune function in transgenic mice overexpressing growth hormone (GH) releasing hormone, GH or GH antagonist. Proc. Soc. Exp. Biol. Med. 221:178-183.
Dolnik, V., M. Novotny, and J. Chmelik. 1993. Electromigration behavior of poly-(L-glutamate) conformers in concentrated polyacrylamide gels. Biopolymers 33:1299-1306.
Dorsch-Hasler, K., G. M. Keil, F. Weber, M. Jasin, W. Schaffner, and U. H. Koszinowski. 1985. A long and complex enhancer activates transcription of the gene coding for the highly abundant immediate early mRNA in murine cytomegalovirus. Proc. Natl. Acad. Sci. U. S. A 82:8325-8329.
Dorshkind, K. and N. D. Horseman. 2001. Anterior pituitary hormones, stress, and immune system homeostasis. Bioessays 23:288-294.
Draghia-Akli, R., K. K. Cummings, A. S. Khan, P. A. Brown, and R. H. Carpenter. 2003a. Effects of plasmid-mediated growth hormone releasing hormone supplementation in young healthy Beagle dogs. Journal of Animal Science 81:2301-2310.
Draghia-Akli, R., K. M. Ellis, L. A. Hill, P. B. Malone, and M. L. Fiorotto. 2003b. High-efficiency growth hormone releasing hormone plasmid vector administration into skeletal muscle mediated by electroporation in pigs. FASEB J 17:526-528.
Draghia-Akli, R., M. L. Fiorotto, L. A. Hill, P. B. Malone, D. R. Deaver, and R. J. Schwartz. 1999. Myogenic expression of an injectable protease-resistant growth hormone-releasing hormone augments long-term growth in pigs. Nat. Biotechnol. 17:1179-1183.
Draghia-Akli, R., K. A. Hahn, G. K. King, K. Cummings, and R. H. Carpenter. 2002a. Effects Of Plasmid Mediated Growth Hormone Releasing Hormone In Severely Debilitated Dogs With Cancer. Molecular Therapy 6:830-836.
Draghia-Akli, R., A. S. Khan, K. K. Cummings, D. Parghi, R. H. Carpenter, and P. A. Brown. 2002b. Electrical Enhancement of Formulated Plasmid Delivery in Animals. Technology in Cancer Research & Treatment 1:365-371.
Draghia-Akli, R., X. G. Li, and R. J. Schwartz. 1997. Enhanced growth by ectopic expression of growth hormone releasing hormone using an injectable myogenic vector. Nat. Biotechnol. 15:1285-1289.
Draghia-Akli, R., P. B. Malone, L. A. Hill, K. M. Ellis, R. J. Schwartz, and J. L. Nordstrom. 2002c. Enhanced animal growth via ligand-regulated GHRH myogenic-injectable vectors. FASEB J. 16:426-428.
Dubreuil, P., D. Petitclerc, G. Pelletier, P. Gaudreau, C. Farmer, Mowles, TF, and P. Brazeau. 1990. Effect of dose and frequency of administration of a potent analog of human growth hormone-releasing factor on hormone secretion and growth in pigs. J Anim Sci 68:1254-1268.
Duck, S. C., H. P. Schwarz, G. Costin, R. Rapaport, S. Arslanian, A. Hayek, M. Connors, and J. Jaramillo. 1992. Subcutaneous growth hormone-releasing hormone therapy in growth hormone-deficient children: first year of therapy. J Clin Endocrinol Metab 75:1115-1120.
Esch, F. S., P. Bohlen, N. C. Ling, P. E. Brazeau, W. B. Wehrenberg, M. O. Thorner, M. J. Cronin, and R. Guillemin. 1982. Characterization of a 40 residue peptide from a human pancreatic tumor with growth hormone releasing activity. BBRC 109:152-158.
Etherton, T. D., J. P. Wiggins, C. S. Chung, C. M. Evock, J. F. Rebhun, and P. E. Walton. 1986. Stimulation of pig growth performance by porcine growth hormone and growth hormone-releasing factor. Journal of Animal Science 63:1389-1399.
Evans, W. S., M. L. Vance, D. L. Kaiser, R. P. Sellers, J. L. Borges, T. R. Downs, L. A. Frohman, J. Rivier, W. Vale, and M. O. Thorner. 1985. Effects of intravenous, subcutaneous, and intranasal administration of growth hormone (GH)-releasing hormone-40 on serum GH concentrations in normal men. Journal of Clinical Endocrinology & Metabolism 61:846-850.
Farmer, C., D. Petitclerc, G. Pelletier, and P. Brazeau. 1992. Lactation performance of sows injected with growth hormone- releasing factor during gestation and(or) lactation. Journal of Animal Science 70:2636-2642.
Farmer, C., S. Robert, and J. J. Matte. 1996. Lactation performance of sows fed a bulky diet during gestation and receiving growth hormone-releasing factor during lactation. J. Anim Sci. 74:1298-1306.
Fewell, J. G., F. MacLaughlin, V. Mehta, M. Gondo, F. Nicol, E. Wilson, and L. C. Smith. 2001. Gene therapy for the treatment of hemophilia B using PINC-formulated plasmid delivered to muscle with electroporation. Mol. Ther. 3:574-583.
Frederickson, R. M., B. J. Carter, and A. M. Pilaro. 2003. Nonclinical Toxicology in Support of Licensure of Gene Therapies. Mol. Ther. 8:8-10.
Frohman, L. A., T. R. Downs, E. P. Heimer, and A. M. Felix. 1989. Dipeptidylpeptidase IV and trypsin-like enzymatic degradation of human growth hormone-releasing hormone in plasma. J. Clin. Invest. 83:1533-1540.
Frohman, L. A., J. L. Thominet, C. B. Webb, M. L. Vance, H. Uderman, J. Rivier, W. Vale, and M. O. Thorner. 1984. Metabolic clearance and plasma disappearance rates of human pancreatic tumor growth hormone releasing factor in man. J. Clin. Invest. 73:1304-1311.
Fryer, A. D. and D. B. Jacoby. 1993. Effect of inflammatory cell mediators on M2 muscarinic receptors in the lungs. Life Sci. 52:529-536.
Gehl, J., T. Skovsgaard, and L. M. Mir. 1998. Enhancement of cytotoxicity by electropermeabilization: an improved method for screening drugs. Anticancer Drugs 9:319-325.
Gehl, J., T. H. Sorensen, K. Nielsen, P. Raskmark, S. L. Nielsen, T. Skovsgaard, and L. M. Mir. 1999. In vivo electroporation of skeletal muscle: threshold, efficacy and relation to electric field distribution. Biochim. Biophys. Acta 1428:233-240.
Gelato, M. C. 1996. Aging and immune function: a possible role for growth hormone. Horm. Res. 45:46-49.
German, M., S. Ashcroft, K. Docherty, H. Edlund, T. Edlund, S. Goodison, H. Imura, G. Kennedy, O. Madsen, D. Melloul, and. 1995. The insulin gene promoter. A simplified nomenclature. Diabetes 44:1002-1004.
Ghigo, E., E. Arvat, F. Broglio, R. Giordano, L. Gianotti, G. Muccioli, M. Papotti, A. Graziani, G. Bisi, R. Deghenghi, and F. Camanni. 1999. Endocrine and non-endocrine activities of growth hormone secretagogues in humans. Horm. Res. 51 Suppl 3:9-15.
Gopinath, R. and T. D. Etherton. 1989a. Effects of porcine growth hormone on glucose metabolism of pigs: I. Acute and chronic effects on plasma glucose and insulin status. J. Anim Sci. 67:682-688.
Gopinath, R. and T. D. Etherton. 1989b. Effects of porcine growth hormone on glucose metabolism of pigs: II. Glucose tolerance, peripheral tissue insulin sensitivity and glucose kinetics. J. Anim Sci. 67:689-697.
Guillemin, R., P. Brazeau, P. Bohlen, F. Esch, N. Ling, and W. B. Wehrenberg. 1982. Growth hormone-releasing factor from a human pancreatic tumor that caused acromegaly. Science 218:585-587.
Heller, R., M. J. Jaroszeski, L. F. Glass, J. L. Messina, D. P. Rapaport, R. C. DeConti, N. A. Fenske, R. A. Gilbert, L. M. Mir, and D. S. Reintgen. 1996. Phase I/II trial for the treatment of cutaneous and subcutaneous tumors using electrochemotherapy. Cancer 77:964-971.
Horlick, R. A. and P. A. Benfield. 1989. The upstream muscle-specific enhancer of the rat muscle creatine kinase gene is composed of multiple elements. Mol. Cell Biol. 9:2396-2413.
Inouye, C., P. Remondelli, M. Karin, and S. Elledge. 1994. Isolation of a cDNA encoding a metal response element binding protein using a novel expression cloning procedure: the one hybrid system. DNA Cell Biol. 13:731-742.
Inouye, S., A. Nakazawa, and T. Nakazawa. 1985. Determination of the transcription initiation site and identification of the protein product of the regulatory gene xylR for xyl operons on the TOL plasmid. J. Bacteriol. 163:863-869.
Jardieu, P., R. Clark, D. Mortensen, and K. Dorshkind. 1994. In vivo administration of insulin-like growth factor-I stimulates primary B lymphopoiesis and enhances lymphocyte recovery after bone marrow transplantation. J Immunol. 152:4320-4327.
Jaynes, J. B., J. E. Johnson, J. N. Buskin, C. L. Gartside, and S. D. Hauschka. 1988. The muscle creatine kinase gene is regulated by multiple upstream elements, including a muscle-specific enhancer. Mol. Cell Biol. 8:62-70.
Kawamoto, T., K. Makino, H. Niwa, H. Sugiyama, S. Kimura, M. Amemura, A. Nakata, and T. Kakunaga. 1988. Identification of the human beta-actin enhancer and its binding factor. Mol. Cell Biol. 8:267-272.
Kawamoto, T., K. Makino, S. Orita, A. Nakata, and T. Kakunaga. 1989. DNA bending and binding factors of the human beta-actin promoter. Nucleic Acids Res. 17:523-537.
Khorram, O., M. Garthwaite, and T. Golos. 2001. The influence of aging and sex hormones on expression of growth hormone-releasing hormone in the human immune system. J Clin. Endocrinol. Metab 86:3157-3161.
Khorram, O., M. Yeung, L. Vu, and S. S. Yen. 1997. Effects of [norleucine27]growth hormone-releasing hormone (GHRH) (1-29)-NH2 administration on the immune system of aging men and women. J Clin. Endocrinol. Metab 82:3590-3596.
Klamut, H. J., L. O. Bosnoyan-Collins, R. G. Worton, P. N. Ray, and H. L. Davis. 1996. Identification of a transcriptional enhancer within muscle intron 1 of the human dystrophin gene. Hum. Mol. Genet. 5:1599-1606.
Klamut, H. J., S. B. Gangopadhyay, R. G. Worton, and P. N. Ray. 1990. Molecular and functional analysis of the muscle-specific promoter region of the Duchenne muscular dystrophy gene. Mol. Cell Biol. 10:193-205.
Klindt, J., J. T. Yen, F. C. Buonomo, A. J. Roberts, and T. Wise. 1998. Growth, body composition, and endocrine responses to chronic administration of insulin-like growth factor I and(or) porcine growth hormone in pigs. J. Anim Sci. 76:2368-2381.
Koo, G. C., C. Huang, R. Camacho, C. Trainor, J. T. Blake, A. Sirotina-Meisher, K. D. Schleim, T. J. Wu, K. Cheng, R. Nargund, and G. McKissick. 2001. Immune enhancing effect of a growth hormone secretagogue. J Immunol. 166:4195-4201.
Kraus, J., M. Woltje, N. Schonwetter, and V. Hollt. 1998. Alternative promoter usage and tissue specific expression of the mouse somatostatin receptor 2 gene. FEBS Lett. 428:165-170.
Krishnaraj, R., A. Zaks, and T. Unterman. 1998. Relationship between plasma IGF-I levels, in vitro correlates of immunity, and human senescence. Clin. Immunol. Immunopathol. 88:264-270.
Lapierre, H., G. Pelletier, D. Petitclerc, P. Dubreuil, J. Morisset, P. Gaudreau, Y. Couture, and P. Brazeau. 1991. Effect of human growth hormone-releasing factor and(or) thyrotropin-releasing factor on growth, carcass composition, diet digestibility, nutrient balance, and plasma constituents in dairy calves. Journal of Animal Science 69:587-598.
Lareyre, J. J., T. Z. Thomas, W. L. Zheng, S. Kasper, D. E. Ong, M. C. Orgebin-Crist, and R. J. Matusik. 1999. A 5-kilobase pair promoter fragment of the murine epididymal retinoic acid-binding protein gene drives the tissue-specific, cell-specific, and androgen-regulated expression of a foreign gene in the epididymis of transgenic mice. J. Biol. Chem. 274:8282-8290.
Larsen, P. R., J. W. Harney, and D. D. Moore. 1986. Sequences required for cell-type specific thyroid hormone regulation of rat growth hormone promoter activity. J. Biol. Chem. 261:14373-14376.
Lee, S. H., W. Wang, S. Yajima, P. A. Jose, and M. M. Mouradian. 1997. Tissue-specific promoter usage in the D1A dopamine receptor gene in brain and kidney. DNA Cell Biol. 16:1267-1275.
Lesbordes, J. C., T. Bordet, G. Haase, L. Castelnau-Ptakhine, S. Rouhani, H. Gilgenkrantz, and A. Kahn. 2002. In vivo electrotransfer of the cardiotrophin-1 gene into skeletal muscle slows down progression of motor neuron degeneration in pmn mice. Hum. Mol. Genet. 11:1615-1625.
Levenson, V. V., E. D. Transue, and I. B. Roninson. 1998. Internal ribosomal entry site-containing retroviral vectors with green fluorescent protein and drug resistance markers. Hum. Gene Ther. 9:1233-1236.
Li, C., S. Ke, Q. P. Wu, W. Tansey, N. Hunter, L. M. Buchmiller, L. Milas, C. Charnsangavej, and S. Wallace. 2000. Tumor irradiation enhances the tumor-specific distribution of poly(L-glutamic acid)-conjugated paclitaxel and its antitumor efficacy. Clin. Cancer Res. 6:2829-2834.
Li, X., E. M. Eastman, R. J. Schwartz, and R. Draghia-Akli. 1999. Synthetic muscle promoters: activities exceeding naturally occurring regulatory sequences. Nat. Biotechnol. 17:241-245.
Lin, H., K. E. Yutzey, and S. F. Konieczny. 1991. Muscle-specific expression of the troponin I gene requires interactions between helix-loop-helix muscle regulatory factors and ubiquitous transcription factors. Mol. Cell Biol. 11:267-280.
Liu, J. L. and D. LeRoith. 1999. Insulin-like growth factor I is essential for postnatal growth in response to growth hormone. Endocrinology 140:5178-5184.
Liu, Y., H. Li, K. Tanaka, N. Tsumaki, and Y. Yamada. 2000. Identification of an enhancer sequence within the first intron required for cartilage-specific transcription of the alpha2(XI) collagen gene. J. Biol. Chem. 275:12712-12718.
Lucas, M. L., L. Heller, D. Coppola, and R. Heller. 2002. IL-12 plasmid delivery by in vivo electroporation for the successful treatment of established subcutaneous B16.F10 melanoma. Mol. Ther. 5:668-675.
Lucas, M. L., M. J. Jaroszeski, R. Gilbert, and R. Heller. 2001. In vivo electroporation using an exponentially enhanced pulse: a new waveform. DNA Cell Biol. 20:183-188.
Macejak, D. G. and P. Sarnow. 1991. Internal initiation of translation mediated by the 5' leader of a cellular mRNA. Nature 353:90-94.
Marshall, L., B. Perras, H. L. Fehm, and J. Born. 2001. Changes in immune cell counts and interleukin (IL)-1beta production in humans after a somnogenically active growth hormone-releasing hormone (GHRH) administration. Brain Behav. Immun. 15:227-234.
Matsubara, H., Y. Gunji, T. Maeda, K. Tasaki, Y. Koide, T. Asano, T. Ochiai, S. Sakiyama, and M. Tagawa. 2001. Electroporation-mediated transfer of cytokine genes into human esophageal tumors produces anti-tumor effects in mice. Anticancer Res. 21:2501-2503.
Matsuo, A., I. Tooyama, S. Isobe, Y. Oomura, I. Akiguchi, K. Hanai, J. Kimura, and H. Kimura. 1994. Immunohistochemical localization in the rat brain of an epitope corresponding to the fibroblast growth factor receptor-1. Neuroscience 60:49-66.
McNally, M. A., J. S. Lebkowski, T. B. Okarma, and L. B. Lerch. 1988. Optimizing electroporation parameters for a variety of human hematopoietic cell lines. Biotechniques 6:882-886.
Miklavcic, D., K. Beravs, D. Semrov, M. Cemazar, F. Demsar, and G. Sersa. 1998. The importance of electric field distribution for effective in vivo electroporation of tissues. Biophys. J 74:2152-2158.
Miller, K. F., D. J. Bolt, V. G. Pursel, R. E. Hammer, C. A. Pinkert, R. D. Palmiter, and R. L. Brinster. 1989. Expression of human or bovine growth hormone gene with a mouse metallothionein-1 promoter in transgenic swine alters the secretion of porcine growth hormone and insulin-like growth factor-I. J. Endocrinol. 120:481-488.
Mumper, R. J., J. Wang, S. L. Klakamp, H. Nitta, K. Anwer, F. Tagliaferri, and A. P. Rolland. 1998. Protective interactive noncondensing (PINC) polymers for enhanced plasmid distribution and expression in rat skeletal muscle. J. Control Release 52:191-203.
Muramatsu, T., S. Arakawa, K. Fukazawa, Y. Fujiwara, T. Yoshida, R. Sasaki, S. Masuda, and H. M. Park. 2001. In vivo gene electroporation in skeletal muscle with special reference to the duration of gene expression. Int. J Mol. Med. 7:37-42.
Murray, R. D., D. Y. Downham, M. J. Clarkson, W. B. Faull, J. W. Hughes, F. J. Manson, J. B. Merritt, W. B. Russell, J. E. Sutherst, and W. R. Ward. 1996. Epidemiology of lameness in dairy cattle: description and analysis of foot lesions. Vet. Rec. 138:586-591.
Nairn, R. S., G. M. Adair, T. Porter, S. L. Pennington, D. G. Smith, J. H. Wilson, and M. M. Seidman. 1993. Targeting vector configuration and method of gene transfer influence targeted correction of the APRT gene in Chinese hamster ovary cells. Somat. Cell Mol. Genet. 19:363-375.
Narum, D. L., S. Kumar, W. O. Rogers, S. R. Fuhrmann, H. Liang, M. Oakley, A. Taye, B. K. Sim, and S. L. Hoffman. 2001. Codon optimization of gene fragments encoding Plasmodium falciparum merzoite proteins enhances DNA vaccine protein expression and immunogenicity in mice. Infect. Immun. 69:7250-7253.
Neumann, E., M. Schaefer-Ridder, Y. Wang, and P. H. Hofschneider. 1982. Gene transfer into mouse lyoma cells by electroporation in high electric fields. EMBO J. 1:841-845.
Nomoto, S., Y. Tatematsu, T. Takahashi, and H. Osada. 1999. Cloning and characterization of the alternative promoter regions of the human LIMK2 gene responsible for alternative transcripts with tissue-specific expression. Gene 236:259-271.
Ohlsson, H., S. Thor, and T. Edlund. 1991. Novel insulin promoter- and enhancer-binding proteins that discriminate between pancreatic alpha- and beta-cells. Mol. Endocrinol. 5:897-904.
Otani, Y., Y. Tabata, and Y. Ikada. 1996. Rapidly curable biological glue composed of gelatin and poly(L-glutamic acid). Biomaterials 17:1387-1391.
Otani, Y., Y. Tabata, and Y. Ikada. 1998. Hemostatic capability of rapidly curable glues from gelatin, poly(L-glutamic acid), and carbodiimide. Biomaterials 19:2091-2098.
Pech, M., C. D. Rao, K. C. Robbins, and S. A. Aaronson. 1989. Functional identification of regulatory elements within the promoter region of platelet-derived growth factor 2. Mol. Cell Biol. 9:396-405.
Pelletier, J. and N. Sonenberg. 1988. Internal initiation of translation of eukaryotic mRNA directed by a sequence derived from poliovirus RNA. Nature 334:320-325.
Pinkert, C. A., D. M. Ornitz, R. L. Brinster, and R. D. Palmiter. 1987. An albumin enhancer located 10 kb upstream functions along with its promoter to direct efficient, liver-specific expression in transgenic mice. Genes Dev. 1:268-276.
Potter, H., L. Weir, and P. Leder. 1984. Enhancer-dependent expression of human kappa immunoglobulin genes introduced into mouse pre-B lymphocytes by electroporation. Proc. Natl. Acad. Sci. U. S. A 81:7161-7165. Prentice, H., R. A. Kloner, T. Prigozy, T. Christensen, L. Newman, Y. Li, and L. Kedes. 1994. Tissue restricted gene expression assayed by direct DNA injection into cardiac and skeletal muscle. Journal of Molecular & Cellular Cardiology 26:1393-1401.
Pursel, V. G., D. J. Bolt, K. F. Miller, C. A. Pinkert, R. E. Hammer, R. D. Palmiter, and R. L. Brinster. 1990. Expression and performance in transgenic pigs. J. Reprod. Fertil. Suppl 40:235-45:235-245.
Rabinovsky, E. D., G. M. Smith, D. P. Browder, H. D. Shine, and J. L. McManaman. 1992. Peripheral nerve injury down-regulates CNTF expression in adult rat sciatic nerves. J. Neurosci. Res. 31:188-192.
Rahim, A., P. A. O'Neill, and S. M. Shalet. 1999. The effect of chronic hexarelin administration on the pituitary-adrenal axis and prolactin. Clin. Endocrinol. (Oxf) 50:77-84.
Rahim, A. and S. M. Shalet. 1998. Does desensitization to hexarelin occur? Growth Horm. IGF. Res. 8 Suppl B:141-143. Robbins, K., S. McCabe, T. Scheiner, J. Strasser, R. Clark, and P. Jardieu. 1994. Immunological effects of insulin-like growth factor-I--enhancement of immunoglobulin synthesis. Clin. Exp. Immunol. 95:337-342.
Rodenburg, J. Body Condition Scoring of Cattle. Ontario Ministry of Agriculture and Food Fact Sheet # 92-122. 1996.
   Ref Type: Bill/Resolution
Sakai, M., Y. Kajita, M. Kobayashi, and H. Kawauchi. 1997. Immunostimulating effect of growth hormone: in-vivo administration of growth hormone in rainbow trout enhances resistance to Vibrio anguillarum infection. Vet. Immunol. Immunopathol. 57:147-152.
Satozawa, N., K. Takezawa, T. Miwa, S. Takahashi, M. Hayakawa, and H. Ooka. 2000. Differences in the effects of 20 K- and 22 K-hGH on water retention in rats. Growth Horm. IGF. Res. 10:187-192.
Schleim, K. D., T. Jacks, P. Cunningham, W. Feeney, E. G. Frazier, G. W. Niebauer, D. Zhang, H. Chen, R. G. Smith, and G. Hickey. 1999. Increases in circulating insulin-like growth factor I levels by the oral growth hormone secretagogue MK-0677 in the beagle are dependent upon pituitary mediation. Endocrinology 140:1552-1558.
Siejka, A., H. Lawnicka, J. Komorowski, T. Stepien, R. Krupinski, and H. Stepien. 2004. Effect of growth hormone-releasing hormone (GHRH) and GHRH antagonist (MZ-4-71) on interferon-gamma secretion from human peripheral blood mononuclear cells in vitro. Neuropeptides 38:35-39.
Skroch, P., C. Buchman, and M. Karin. 1993. Regulation of human and yeast metallothionein gene transcription by heavy metal ions. Prog. Clin. Biol. Res. 380:113-28.:113-128.
Smith, L. C. and J. L. Nordstrom. 2000. Advances in plasmid gene delivery and expression in skeletal muscle. Curr. Opin. Mol. Ther. 2:150-154.
Smith, V. G., A. D. Leman, W. J. Seaman, and F. VanRavenswaay. 1991. Pig weaning weight and changes in hematology and blood chemistry of sows injected with recombinant porcine somatotropin during lactation. J. Anim Sci. 69:3501-3510.
Soubrier, F., B. Cameron, B. Manse, S. Somarriba, C. Dubertret, G. Jaslin, G. Jung, C. L. Caer, D. Dang, J. M. Mouvault, D. Scherman, J. F. Mayaux, and J. Crouzet. 1999. pCOR: a new design of plasmid vectors for nonviral gene therapy. Gene Ther. 6:1482-1488.
Svensson, J. A. and B. Bengtsson. 1999. Clinical and experimental effects of growth hormone secretagogues on various organ systems. Horm. Res. 51 Suppl 3:16-20.
Terada, Y., H. Tanaka, T. Okado, S. Inoshita, M. Kuwahara, T. Akiba, S. Sasaki, and F. Marumo. 2001. Efficient and ligand-dependent regulated erythropoietin production by naked dna injection and in vivo electroporation. Am. J Kidney Dis. 38:S50-S53.
Thorner, M. O., L. A. Frohman, D. A. Leong, J. Thominet, T. Downs, P. Hellmann, J. Chitwood, J. M. Vaughan, and W. Vale. 1984. Extrahypothalamic growth-hormone-releasing factor (GRF) secretion is a rare cause of acromegaly: plasma GRF levels in 177 acromegalic patients. Journal of Clinical Endocrinology & Metabolism 59:846-849.
Thorner, M. O., M. L. Vance, W. S. Evans, R. M. Blizzard, A. D. Rogol, K. Ho, Leong, DA, J. L. Borges, M. J. Cronin, R. M. MacLeod, and et al. 1986a. Physiological and clinical studies of GRF and GH. Recent Progress in Hormone Research 42:589-640.
Thorner, M. O., M. L. Vance, W. S. Evans, A. D. Rogol, J. Rivier, W. Vale, Blizzard, and RM. 1986b. Clinical studies with GHRH in man. Hormone Research 24:91-98.
Tollefsen, S., M. Vordermeier, I. Olsen, A. K. Storset, L. J. Reitan, D. Clifford, D. B. Lowrie, H. G. Wiker, K. Huygen, G. Hewinson, I. Mathiesen, and T. E. Tjelle. 2003. DNA injection in combination with electroporation: a novel method for vaccination of farmed ruminants. Scand. J Immunol. 57:229-238.
Toneguzzo, F., A. Keating, S. Glynn, and K. McDonald. 1988. Electric field-mediated gene transfer: characterization of DNA transfer and patterns of integration in lymphoid cells. Nucleic Acids Res. 16:5515-5532. Tripathy, S. K., E. C. Svensson, H. B. Black, E. Goldwasser, M. Margalith, Hobart, PM, and J. M. Leiden. 1996. Long-term expression of erythropoietin in the systemic circulation of mice after intramuscular injection of a plasmid DNA vector. Proc. Natl. Acad. Sci. USA 93:10876-10880.
Tronche, F., A. Rollier, I. Bach, M. C. Weiss, and M. Yaniv. 1989. The rat albumin promoter: cooperation with upstream elements is required when binding of APF/HNF1 to the proximal element is partially impaired by mutation or bacterial methylation. Mol. Cell Biol. 9:4759-4766.
Tronche, F., A. Rollier, P. Herbomel, I. Bach, S. Cereghini, M. Weiss, and M. Yaniv. 1990. Anatomy of the rat albumin promoter. Mol. Biol. Med. 7:173-185.
Trott, D. J., M. R. Moeller, R. L. Zuerner, J. P. Goff, W. R. Waters, D. P. Alt, R. L. Walker, and M. J. Wannemuehler. 2003. Characterization of Treponema phagedenis-like spirochetes isolated from papillomatous digital dermatitis lesions in dairy cattle. J. Clin. Microbiol. 41:2522-2529.
Trudel, M. and F. Costantini. 1987. A 3' enhancer contributes to the stage-specific expression of the human beta-globin gene. Genes Dev. 1:954-961.
Tsumaki, N., T. Kimura, K. Tanaka, J. H. Kimura, T. Ochi, and Y. Yamada. 1998. Modular arrangement of cartilage- and neural tissue-specific cis-elements in the mouse alpha2(XI) collagen promoter. J. Biol. Chem. 273:22861-22864.
Tsunekawa, B., M. Wada, M. Ikeda, H. Uchida, N. Naito, and M. Honjo. 1999. The 20-kilodalton (kDa) human growth hormone (hGH) differs from the 22-kDa hGH in the effect on the human prolactin receptor. Endocrinology 140:3909-3918.
Tsurumi, Y., S. Takeshita, D. Chen, M. Kearney, S. T. Rossow, J. Passeri, J. R. Horowitz, J. F. Symes, and J. M. Isner. 1996. Direct intramuscular gene transfer of naked DNA encoding vascular endothelial growth factor augments collateral development and tissue perfusion [see comments]. Circulation 94:3281-3290.
Tur-Kaspa, R., L. Teicher, B. J. Levine, A. I. Skoultchi, and D. A. Shafritz. 1986. Use of electroporation to introduce biologically active foreign genes into primary rat hepatocytes. Mol. Cell Biol. 6:716-718.
van Rooij, E. M., B. L. Haagmans, H. L. Glansbeek, Y. E. de Visser, M. G. de Bruin, W. Boersma, and A. T. Bianchi. 2000. A DNA vaccine coding for glycoprotein B of pseudorabies virus induces cell-mediated immunity in pigs and reduces virus excretion early after infection. Vet. Immunol. Immunopathol. 74:121-136.
Vance, M. L., D. L. Kaiser, W. S. Evans, R. Furlanetto, W. Vale, J. Rivier, and M. O. Thorner. 1985. Pulsatile growth hormone secretion in normal man during a continuous 24-hour infusion of human growth hormone releasing factor (1-40). Evidence for intermittent somatostatin secretion. J. Clin. Invest. 75:1584-1590.
Vance, M. L., D. L. Kaiser, P. M. Martha, Jr., R. Furlanetto, J. Rivier, W. Vale, and M. O. Thorner. 1989. Lack of in vivo somatotroph desensitization or depletion after 14 days of continuous growth hormone (GH)-releasing hormone administration in normal men and a GH-deficient boy. Journal of Clinical Endocrinology & Metabolism 68:22-28.
Veldhuis, J. D., A. Iranmanesh, and A. Weltman. 1997. Elements in the pathophysiology of diminished growth hormone (GH) secretion in aging humans. Endocrine 7:41-48.
Verhelst, J., R. Abs, M. Vandeweghe, J. Mockel, J. J. Legros, G. Copinschi, C. Mahler, B. Velkeniers, L. Vanhaelst, A. Van Aelst, D. De Rijdt, A. Stevenaert, and A. Beckers. 1997. Two years of replacement therapy in adults with growth hormone deficiency. Clin. Endocrinol. (Oxf) 47:485-494.
Vilquin, J. T., P. F. Kennel, M. Paturneau-Jouas, P. Chapdelaine, N. Boissel, P. Delaere, J. P. Tremblay, D. Scherman, M. Y. Fiszman, and K. Schwartz. 2001. Electrotransfer of naked DNA in the skeletal muscles of animal models of muscular dystrophies. Gene Ther. 8:1097-1107.
Vittone, J., M. R. Blackman, J. Busby-Whitehead, C. Tsiao, K. J. Stewart, J. Tobin, T. Stevens, M. F. Bellantoni, M. A. Rogers, G. Baumann, J. Roth, S. M. Harman, and R. G. S. Spencer. 1997. Effects of single nightly injections of growth hormone-releasing hormone (GHRH 1-29) in healthy elderly men. Metabolism: Clinical and Experimental 46:89-96.
Wada, M., H. Uchida, M. Ikeda, B. Tsunekawa, N. Naito, S. Banba, E. Tanaka, Y. Hashimoto, and M. Honjo. 1998. The 20-kilodalton (kDa) human growth hormone (hGH) differs from the 22-kDa hGH in the complex formation with cell surface hGH receptor and hGH-binding protein circulating in human plasma. Mol. Endocrinol. 12:146-156.
Wells, K. E., J. Maule, R. Kingston, K. Foster, J. McMahon, E. Damien, A. Poole, and D. J. Wells. 1997. Immune responses, not promoter inactivation, are responsible for decreased long-term expression following plasmid gene transfer into skeletal muscle. FEBS Lett. 407:164-168.
Wolff, J. A., R. W. Malone, P. Williams, W. Chong, G. Acsadi, A. Jani, Felgner, and PL. 1990. Direct gene transfer into mouse muscle in vivo. Science 247:1465-1468.
Wu, H. K., J. A. Squire, Q. Song, and R. Weksberg. 1997. Promoter-dependent tissue-specific expressive nature of imprinting gene, insulin-like growth factor II, in human tissues. Biochem. Biophys. Res. Commun. 233:221-226.
Xie, T. D. and T. Y. Tsong. 1993. Study of mechanisms of electric field-induced DNA transfection. V. Effects of DNA topology on surface binding, cell uptake, expression, and integration into host chromosomes of DNA in the mammalian cell. Biophys. J. 65:1684-1689.
Yasui, A., K. Oda, H. Usunomiya, K. Kakudo, T. Suzuki, T. Yoshida, H. M. Park, K. Fukazawa, and T. Muramatsu. 2001. Elevated gastrin secretion by in vivo gene electroporation in skeletal muscle. Int. J Mol. Med. 8:489-494.
Yin, D. and J. G. Tang. 2001. Gene therapy for streptozotocin-induced diabetic mice by electroporational transfer of naked human insulin precursor DNA into skeletal muscle in vivo. FEBS Lett. 495:16-20.
Yorifuji, T. and H. Mikawa. 1990. Co-transfer of restriction endonucleases and plasmid DNA into mammalian cells by electroporation: effects on stable transformation. Mutat. Res. 243:121-126.
Yutzey, K. E. and S. F. Konieczny. 1992. Different E-box regulatory sequences are functionally distinct when placed within the context of the troponin I enhancer. Nucleic Acids Res. 20:5105-5113.
Zhao-Emonet, J. C., O. Boyer, J. L. Cohen, and D. Klatzmann. 1998. Deletional and mutational analyses of the human CD4 gene promoter: characterization of a minimal tissue-specific promoter. Biochim. Biophys. Acta 1442:109-119.

## Claims

1. Use of an isolated nucleic acid expression construct composition for a preparation of a medicament for a vaccination treatment in a subject, wherein the isolated nucleic acid expression construct composition comprises: a nucleic acid expression construct that encodes a growth-hormone-releasing-hormone ("GHRH"); and the vaccination treatment comprises:
(a) delivering into a tissue of the subject a composition having the nucleic acid expression that encodes a growth-hormone-releasing-hormone ("GHRH"); and
(b) providing a vaccine to the subject in an amount effective to induce anti-vaccine antibodies in the subject;
wherein, GHRH is expressed *in vivo* in the subject and a vaccination response is enhanced when compared to a control subject not having a GHRH expression construct delivered.

2. The use of claim 1, wherein delivering the composition having the nucleic acid expression construct occurs concomitantly with providing the vaccine to the subject.

3. The use of claim 1, further comprising: waiting a period of time after delivering the composition having the nucleic acid expression construct encoding GHRH into the subject, but before providing the vaccine.

4. The use of claim 3 wherein the period of time is up to about 1 year.

5. The use of claim 4 wherein the period of time is in the range of about 7 days to about 14 days.

6. The use of claim 1, wherein delivering into the tissue of the subject the composition having the nucleic acid expression construct comprises tissue electroporation.

7. The use of claim 6, wherein tissue electroporation comprises:
penetrating the tissue in the subject with a plurality of needle electrodes, wherein the plurality of needle electrodes are arranged in a spaced relationship and the tissue of the subject comprise muscle cells;
introducing the composition having the nucleic acid expression construct into the tissue between the plurality of needle electrodes in an amount in a range of about 0.01 - 5 mg; and
applying an electrical pulse to the plurality of needle electrodes, wherein the electrical pulse allows the nucleic acid expression construct to traverse a muscle cell membrane.

8. The use of claim 7, wherein the composition having the nucleic acid expression construct further comprises a transfection-facilitating polypeptide or a charged polypeptide.

9. The use of claim 8, wherein the transfection-facilitating polypeptide, or charged polypeptide comprises poly-L-glutamate.

10. The use of claim 1, wherein the nucleic acid expression construct comprises a sequence that encodes a polypeptide having an amino acid sequence of which is at leas 90% identical to the encoded GHRH of SEQID#14.

11. The use of claim 1, wherein the nucleic acid expression construct comprising a sequence that is at least 97% identical to mouse pAV0202 (SEQID#23); rat pAV0203 (SEQID#24); HV-GHRH pAV0224 (SEQID#25); pig-wt-GHRH pAV0225 (SEQID#26); dog pAV0235 (SEQID#27); bovine pAV0236 (SEQID#28); cat pAV0238 (SEQID#29); TI-GHRH pAV0239 (SEQID#30); ovine pAV0240 (SEQID#31); chicken pAV0241 (SEQID#32); horse pAV0249 (SEQID#33), or human pAV0226 (SEQID#34).

12. The use of claim 1, wherein the vaccine comprises at least part of a microorganism, an infectious agent, or a toxoid.

13. The use of claim 12, wherein the microorganism comprises: a virus, a bacteria, a mycoplasma, or a parasite.

14. The use of claim 12, wherein the microorganism comprises a bovine herpesvirus-1 ("IBR"), bovine virus diarrhea ("BVD"), parainfluenza 3, respiratory syncytial virus, Leptospira canicola, Leptospira grippotyphosa, Leptospira hardjo, Leptospira icterohaemorrhagiae, Leptospira Pomona bacterins, Mycoplasma hyopneumonia, or combination thereof.

15. The use of claim 1, wherein the subject comprises a human, a ruminant animal, a food animal, or a work animal.

16. A composition comprising:
(a) a nucleic acid expression construct that encodes a growth-hormone-releasing-hormone ("GHRH"); and
(b) a vaccine;
wherein, GHRH is expressed *in vivo* in the subject.

17. The composition of claim 16, wherein the vaccine comprises at least part of a microorganism, an infectious agent, or a toxoid.

18. The composition of claim 17, wherein the microorganism comprises: a virus, a bacteria, a mycoplasma, or a parasite.

19. The composition of claim 17, wherein the microorganism comprises a bovine herpesvirus-1 ("IBR"), bovine virus diarrhea ("BVD"), parainfluenza 3, respiratory syncytial virus, Leptospira canicola, Leptospira grippotyphosa, Leptospira hardjo, Leptospira icterohaemorrhagiae, Leptospira Pomona bacterins, Mycoplasma hyopneumonia, or combination thereof.

## Patentansprüche

1. Verwendung einer Zusammensetzung eines isolierten Nukleinsäureexpressionskonstrukts zur Herstellung eines Medikaments für die Impfstoffbehandlung in einem Subjekt, wobei die Zusammensetzung eines isolierten Nukleinsäureexpressionskonstrukts ein Nukleinsäureexpressionskonstrukt umfaßt, das ein Wachstumshormon-freisetzendes-Hormon (growth-hormone-releasing-hormone, "GHRH") kodiert, und die Impfstoffbehandlung
(a) das Verabreichen einer Zusammensetzung, welche die Nukleinsäureexpression, die ein Wachstumshormon-freisetzendes-Hormon ("GHRH") kodiert, aufweist, in ein Gewebe des Subjekts, und
(b) das Bereitstellen eines Impfstoffs an das Subjekt, in einer Menge die wirksam ist, anti-Impfstoff Antikörper in dem Subjekt zu induzieren, umfaßt,
wobei GHRH *in vivo* in dem Subjekt exprimiert wird und eine Antwort auf die Impfung verstärkt wird, verglichen mit einem Kontrollsubjekt, das kein GHRH-Expressionskonstrukt verabreicht bekommen hat.

2. Verwendung nach Anspruch 1, wobei das Verabreichen der Zusammensetzung, die das Nukleinsäureexpressionskonstrukt aufweist, gleichzeitig mit dem Bereitstellen des Impfstoffs an das Subjekt geschieht.

3. Verwendung nach Anspruch 1, weiter umfassend das Abwarten einer Zeitspanne nach dem Verabreichen der Zusammensetzung, die das Nukleinsäureexpressionskonstrukt, das GHRH kodiert, aufweist, in das Subjekt, aber vor dem Bereitstellen des Impfstoffs.

4. Verwendung nach Anspruch 3, wobei die Zeitspanne bis zu etwa ein Jahr beträgt.

5. Verwendung nach Anspruch 4, wobei die Zeitspanne im Bereich von etwa sieben Tagen bis etwa 14 Tagen liegt.

6. Verwendung nach Anspruch 1, wobei das Verabreichen der Zusammensetzung, die das Nukleinsäureexpressionskonstrukt aufweist, in das Gewebe des Subjekts Gewebeelektroporation umfaßt.

7. Verwendung nach Anspruch 6, wobei die Gewebeelektroporation umfaßt:
Durchdringen des Gewebes in dem Subjekt mit einer Vielzahl von Nadelelektroden, wobei die Vielzahl der Nadelelektroden in regelmäßigen Abständen angeordnet sind, und das Gewebe des Subjekts Muskelzellen umfaßt,
Einbringen der Zusammensetzung, die das Nukleinsäureexpressionskonstrukt aufweist, in das Gewebe zwischen der Vielzahl an Nadelelektroden, in einer Menge im Bereich von etwa 0,01 bis 5 mg, und
Anlegen eines elektrischen Impulses an die Vielzahl von Nadelelektroden, wobei der elektrische Impuls dem Nukleinsäureexpressionskonstrukt erlaubt, eine Muskelzellmembran zu durchqueren.

8. Verwendung nach Anspruch 7, wobei die Zusammensetzung, die das Nukleinsäureexpressionskonstrukt aufweist, weiter ein die Transfektion erleichterndes Polypeptid oder ein geladenes Polypeptid umfaßt.

9. Verwendung nach Anspruch 8, wobei das die Transfektion erleichternde Polypeptid oder geladene Polypeptid poly-L-Glutamat umfaßt.

10. Verwendung nach Anspruch 1, wobei das Nukleinsäureexpressionskonstrukt eine Sequenz umfaßt, die ein Polypeptid kodiert, das eine Aminosäuresequenz aufweist, von der zumindest 90% identisch zu dem kodierten GHRH von SEQ ID Nr. 14 sind.

11. Verwendung nach Anspruch 1, wobei das Nukleinsäureexpressionskonstrukt eine Sequenz umfaßt, die zumindest 97% identisch ist zu Maus pAV0202 (SEQ ID Nr. 23), Ratte pAV0203 (SEQ ID Nr. 24), HV-GHRH pAV0224 (SEQ ID Nr. 25), Schwein wt-GHRH pAV0225 (SEQ ID Nr. 26), Hund pAV0235 (SEQ ID Nr. 27), bovinem pAV0236 (SEQ ID Nr. 28), Katze pAV0238 (SEQ ID Nr. 29), TI-GHRH pAV0239 (SEQ ID Nr. 30), ovinem pAV0240 (SEQ ID Nr. 31), Huhn pAV0241 (SEQ ID Nr. 32), Pferd pAV0249 (SEQ ID Nr. 33), oder humanem pAV0226 (SEQ ID Nr. 34).

12. Verwendung nach Anspruch 1, wobei der Impfstoff zumindest einen Teil eines Mikroorganismus, ein infektiöses Mittel oder ein Toxoid umfaßt.

13. Verwendung nach Anspruch 12, wobei der Mikroorganismus ein Virus, ein Bakterium, ein Mycoplasma oder einen Parasiten umfaßt.

14. Verwendung nach Anspruch 12, wobei der Mikroorganismus ein bovines Herpesvirus-1 ("IBR"), bovine Virusdiarrhö ("BVD"), Parainfluenza-3, RS-Virus (respiratory syncytial virus), *Leptospira canicola, Leptospira grippotyphosa, Leptospira hardjo*, *Leptospira icterohaemorrhagiae*, *Leptospira pomona* Bakterine, *Mycoplasma hyopneumoniae* und Mischungen davon, umfaßt.

15. Verwendung nach Anspruch 1, wobei das Subjekt einen Menschen, ein wiederkäuendes Tier, ein Nutztier oder ein Arbeitstier umfaßt.

16. Zusammensetzung, umfassend:
(a) ein Nukleinsäureexpressionskonstrukt, das ein Wachstumshormon-freisetzendes-Hormon ("GHRH") kodiert, und
(b) einen Impfstoff,
wobei GHRH *in vivo* in dem Subjekt exprimiert wird.

17. Zusammensetzung nach Anspruch 16, wobei der Impfstoff zumindest einen Teil eines Mikroorganismus, ein infektiöses Mittel oder ein Toxoid umfaßt.

18. Zusammensetzung nach Anspruch 17, wobei der Mikroorganismus ein Virus, ein Bakterium, ein Mycoplasma oder einen Parasiten umfaßt.

19. Zusammensetzung nach Anspruch 17, wobei der Mikroorganismus ein bovines Herpesvirus-1 ("IBR"), bovine Virusdiarrhö ("BVD"), Parainfluenza-3, RS-Virus (respiratory syncytial virus), *Leptospira canicola, Leptospira grippotyphosa, Leptospira hardjo*, *Leptospira icterohaemorrhagiae*, *Leptospira pomona* Bakterine, *Mycoplasma hyopneumoniae* und Mischungen davon, umfaßt.

## Revendications

1. Utilisation d'une composition d'une construction d'expression d'un acide nucléique isolé pour la préparation d'un médicament destiné à un traitement vaccinal chez un sujet, dans laquelle la composition d'une construction d'expression d'un acide nucléique isolé comprend : une construction d'expression d'un acide nucléique codant pour une hormone de libération de l'hormone de croissance (« GHRH ») ; et le traitement vaccinal comprenant :
(a) l'administration dans un tissu du sujet d'une composition comportant l'expression d'un acide nucléique codant pour une hormone de libération de l'hormone de croissance (« GHRH ») ; et
(b) l'administration d'un vaccin au sujet en quantité suffisante pour induire des anticorps anti-vaccinaux chez le sujet ;
dans laquelle la GHRH est exprimée *in vivo* chez le sujet et la réponse vaccinale est augmentée par comparaison à un sujet témoin n'ayant pas reçu de construction d'expression de la GHRH.

2. Utilisation selon la revendication 1, dans laquelle l'administration de la composition comportant la construction d'expression d'un acide nucléique intervient de façon concomitante à l'administration du vaccin au sujet.

3. Utilisation selon la revendication 1, comprenant, en outre, l'attente pendant une durée après l'administration de la composition comportant la construction d'expression d'un acide nucléique codant pour la GHRH au sujet, mais avant l'administration du vaccin.

4. Utilisation selon la revendication 3, dans laquelle ladite durée peut aller jusqu'à environ 1 an.

5. Utilisation selon la revendication 4, dans laquelle ladite durée se situe dans une fourchette d'environ 7 à environ 14 jours.

6. Utilisation selon la revendication 1, dans laquelle l'administration, dans un tissu du sujet, de la composition comportant la construction d'expression d'un acide nucléique, comprend l'électroporation dans le tissu.

7. Utilisation selon la revendication 6, l'électroporation dans le tissu comprenant :
l'introduction dans le tissu du sujet d'une pluralité d'électrodes aiguilles, dans laquelle la pluralité d'électrodes aiguilles sont arrangées de manière espacée les unes des autres et le tissu du sujet comprend des cellules musculaires ;
l'introduction de la composition comportant la construction d'expression d'un acide nucléique dans le tissu entre la pluralité d'électrodes aiguilles dans une quantité dans une gamme d'environ 0,01 à 5 mg ; et
l'application d'une impulsion électrique à la pluralité d'électrodes aiguilles, dans laquelle l'impulsion électrique permet à la construction d'expression d'un acide nucléique de traverser une membrane de cellule musculaire.

8. Utilisation selon la revendication 7, dans laquelle la composition comporte la construction d'expression d'un acide nucléique comprend, en outre, un polypeptide facilitant la transfection ou un polypeptide chargé.

9. Utilisation selon la revendication 8, dans laquelle le polypeptide facilitant la transfection ou le polypeptide chargé comprend du poly-L-glutamate.

10. Utilisation selon la revendication 1, dans laquelle la construction d'expression d'un acide nucléique comprend une séquence codant pour un polypeptide comportant une séquence d'acides aminés identique à hauteur d'au moins 90 % avec la GHRH encodée par SEQ ID NO : 14.

11. Utilisation selon la revendication 1, dans laquelle la construction d'expression d'un acide nucléique comprend une séquence identique à hauteur d'au moins 97 % avec le pAV0202 de souris (SEQ ID NO : 23) ; le pAV0203 de rat (SEQ ID NO : 24) ; le pAV0224 de la HV-GHRH (SEQ ID NO : 25) ; le pAV0225 de la wt-GHRH de porc (SEQ ID NO : 26) ; le pAV0235 de chien (SEQ ID NO : 27) ; le pAV0236 bovin (SEQ ID NO : 28) ; le pAV0238 de chat (SEQ ID NO : 29) ; le pAV0239 de la TI-GHRH (SEQ ID NO : 30) ; le pAV0240 ovin (SEQ ID NO : 31) ; le pAV0241 de poulet (SEQ ID NO : 32) ; le pAV0249 de cheval (SEQ ID NO : 33) ; ou le pAV0226 humain (SEQ ID NO : 34).

12. Utilisation selon la revendication 1, dans laquelle le vaccin comprend au moins une partie d'un microorganisme, d'un agent infectieux ou d'une anatoxine.

13. Utilisation selon la revendication 12, dans laquelle le microorganisme comprend un virus, une bactérie, un mycoplasme ou un parasite.

14. Utilisation selon la revendication 12, dans laquelle le microorganisme comprend un herpèsvirus bovin de type 1 (« IBR »), un virus de la diarrhée virale bovine (« BVD »), un parainfluenza de type 3, un virus respiratoire syncytial, *Leptospira canicola, Leptospira grippotyphosa*, *Leptospira hardjo, Leptospira icterohaemorrhagiae,* des bactérines *Leptospira pomona, Mycoplasma hyopneumonia* ou une combinaison de ceux-ci.

15. Utilisation selon la revendication 1, dans laquelle le sujet comprend un être humain, un ruminant, un animal élevé pour sa viande ou un animal de trait.

16. Composition comprenant :
(a) une construction d'expression d'un acide nucléique codant pour une hormone de libération de l'hormone de croissance (« GHRH ») ; et
(b) un vaccin ;
dans laquelle la GHRH est exprimée *in vivo* chez le sujet.

17. Composition selon la revendication 16, dans laquelle le vaccin comprend au moins une partie d'un microorganisme, d'un agent infectieux ou d'une anatoxine.

18. Composition selon la revendication 17, dans laquelle le microorganisme comprend : un virus, une bactérie, un mycoplasme ou un parasite.

19. Composition selon la revendication 17, dans laquelle le microorganisme comprend un herpèsvirus bovin de type 1 (« IBR »), un virus de la diarrhée virale bovine (« BVD »), un parainfluenza de type 3, un virus respiratoire syncytial, *Leptospira canicola, Leptospira grippotyphosa, Leptospira hardjo*, *Leptospira icterohaemorrhagiae,* des bactérines *Leptospira pomona*, *Mycoplasma hyopneumonia* ou une combinaison de ceux-ci.
